# FASCICULE DE BREVET EUROPEEN

(11) **EP 0 477 049 B1**
(45) Date de publication et mention de la délivrance du brevet: **01.12.1999**
(21) Numéro de dépôt: 91402269.4
(22) Date de dépôt: 20.08.1991
(51) Int. Cl.: C07D 231/14, C07D 231/54, C07D 401/04, C07D 403/06, C07D 403/12, C07D 401/12, C07D 409/12, A61K 31/415, C07D 453/02, C07D 403/04, C07D 417/04

(54) **Dérivés d'amido-3 pyrazole, procédé pour leur préparation et compositions pharmaceutiques les contenant**
Amido-3-Pyrazol-Derivate, Verfahren zu deren Herstellung und diese enthaltende pharmazeutische Zusammenstellungen
Amido-3-pyrazole derivatives, process for their preparation and pharmaceutical compositions containing them

(30) Priorité: 20.08.1990 FR 9010486
(43) Date de publication de la demande: 25.03.1992
(73) Titulaire: Sanofi-Synthélabo, 75013 Paris (FR)
(72) Inventeur: Boigegrain, Robert, F-34820 Assas (FR); Gully, Danielle, F-31600 Saubens (FR); Jeanjean, Francis, F-34270 Valflaunes (FR); Molimard, Jean-Charles, F-34980 Saint-Gely-du-Fesc (FR)
(74) Mandataire: Gillard, Marie-Louise

(56) Documents cités:
- EP-A- 0 068 806
- EP-A- 0 277 794
- EP-A- 0 289 879
- EP-A- 0 322 126
- WO-A-90/14347
- DE-A- 3 332 633
- FR-A- 2 337 997
- FR-A- 2 597 866
- GB-A- 3 130 205
- US-A- 4 610 983

## Description

La présente invention concerne de nouveaux dérivés du pyrazole possédant un groupement amide substitué par un aminoacide ou un de ses dérivés en position 3 et diversement substitués dans les positions 1, 2, 4 ou 5 du noyau pyrazole, un procédé pour leur préparation et des compositions pharmaceutiques contenant lesdits dérivés du pyrazole comme ingrédient actif.

Les composés selon l'invention possèdent une activité sur le système nerveux central, sur le système cardiovasculaire ou sur le système gastrointestinal.

De nombreux dérivés du pyrazole sont décrits dans la littérature.

Des diaryl-1,5 pyrazoles substitués en 3 par une chaîne alkyle contenant de 2 à 16 atomes de carbone et diversement substitués, notamment par un amide et répondant à la formule: 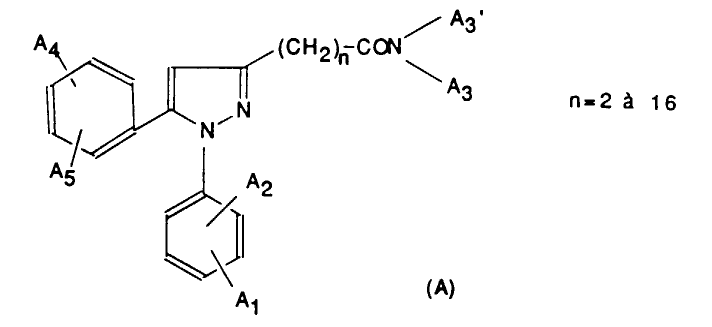 sont décrits dans EP-A-0 248 594 comme possédant une activité anti-inflammatoire et une activité sur le système cardio-vasculaire.

Des dérivés du pyrazole de formule : 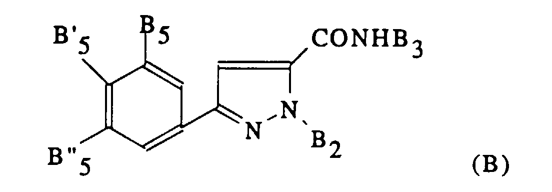 où B₂ représente soit un atome d'hydrogène, soit un groupement méthyle, B₃ représente par exemple un alkyle et B₅, B'₅ et B"₅ représentent indépendamment par exemple l'hydrogène, un halogène, un alcoxy en C₁-C₃, sont décrits dans GB-A-2 130 205 comme pouvant être utilisés afin de diminuer le taux sanguin d'acide urique chez les mammifères.

II est par ailleurs décrit dans Journal of the Chemical Society, 1973, 2532-2534 que des sels de morpholino-2 phényl-5 phénylazofurane-5 se réarrangent en diphényl-1,5 pyrazoles substitués en position 3 de formule :

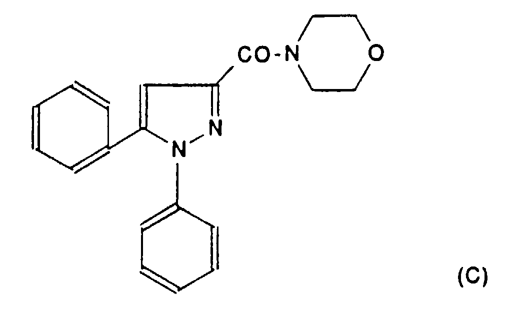

La demande de brevet WO 89/02431 décrit des nouveaux composés N-hétérocycliques, notamment pyrazolyle, de formule : 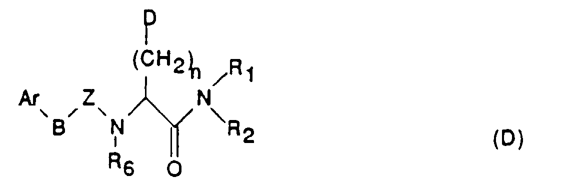 dans laquelle par exemple :
- Ar représente un pyrazolyle,
- B représente (CH₂)ₘ avec m = 0 à 4,
- Z représente -C = O, n = 1 à 3,
- D représente COR₃,
- R₁ et R₂ représentent un hydrogène, un alkyle en C₁-C₈ ou ensemble complètent une amine cyclique.

Ces dérivés amidopyrazole amide d'acide acylglutamique ou aspartique sont décrits comme possédant des propriétés inhibitrices de la cholécystokinine.

Des dérivés d'acides carboxyliques substitués de formule : 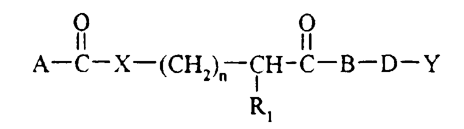 dans laquelle :
. A est notamment un cycle pyrazole di-substitué par exemple par un groupe aryle et substitué par un groupe -COZ ;
. B et D peuvent représenter ensemble le groupe 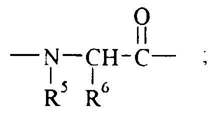
. Z et Y sont notamment un groupe -OR₈,
sont décrits dans DE-A1-3332633 comme composés ayant des propriétés pharmacologiques intéressantes.

Des dérivés du pyrazole de formule : 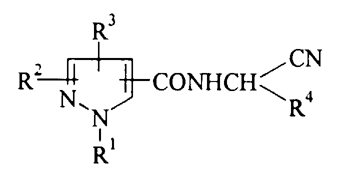 sont décrits dans FR-A-2 597 866 comme composés fongicides.

Des dérivés du pyrazole de formule : 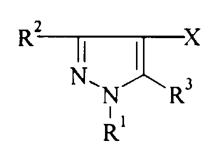 dans laquelle X représente notamment un groupe acylamino sont décrits dans EP-A1-0 289 879 comme insecticides et miticides.

La demande FR-A-2 337 997 décrit une nouvelle composition fongicide à base de dérivés pyrazoliques.

Des dérivés du pyrazole de formule : 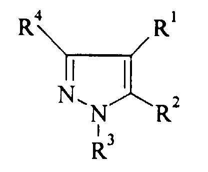 sont décrits dans EP-A3-0 068 806 comme composés utiles pour traiter les psychoses.

Le brevet US 4 610 983 décrit des composés de formule : 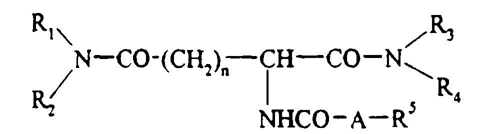 dans laquelle A-R₅ peut être un groupe pyrazole substitué par plusieurs groupes phényle. Ces composés ont une activité anti-ulcère.

D'autres composés hétérocycliques sont également décrits dans la littérature.

Le document EP-A1-0 322 126 décrit des dérivés de pyrazoline comme insecticides.

Le document EP-A3-0 277 794 décrit des N-(4-pipéridyl)amides N-hétérocycliques ayant des propriétés analgésiques et antagonistes.

Le document WO90/14347 décrit des dérivés d'indole comme antagonistes de la sérotonine.

On a maintenant trouvé que des dérivés diversement substitués d'amido-3 pyrazole possèdent une activité sur le système nerveux central et en particulier sur les systèmes de régulation des neuropeptides en déplaçant, par exemple, la neurotensine tritiée ou iodée de son récepteur.

Ainsi, la présente invention a pour objet, selon un de ses aspects, un amido-3 pyrazole de formule (I) ou (I') : 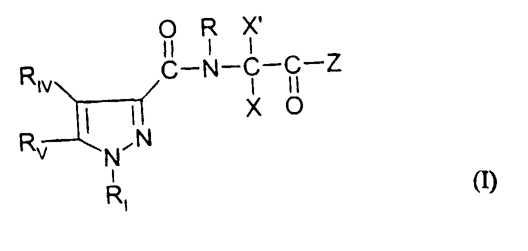 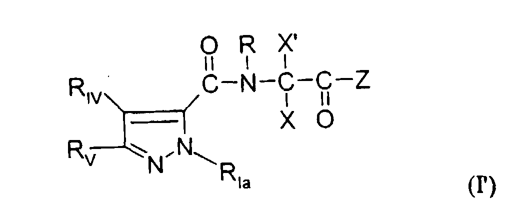 dans laquelle
- R_{I} représente
   . un groupe 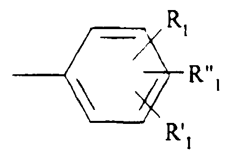 où R₁, R'₁ et R"₁ représentent chacun indépendamment un atome d'hydrogène, un atome d'halogène, un hydroxyle, un groupe alkyle droit ou ramifié en C₁-C₄, un groupe alcoxy en C₁-C₄, un groupe trifluorométhyle, un groupe trifluorométhoxy, un groupe nitro, un groupe carboxy, un groupe amino ;
   . un groupe carboxyalkyle ou alcoxycarbonylalkyle dans lequel les alkyles sont en C₁-C₄ ;
   . un groupe cycloalkyle dans lequel les alkyles sont en C₃-C₆ ;
   . un groupe tétrahydronaphtyle ;
   . un groupe pyridyle ;
   . un naphtyle substitué par R₁, R'₁ et R"₁ tels que définis précédemment ;
   . un groupe benzyle substitué par R₁, R'₁ et R"₁ tels que définis précédemment ;
   . un groupe cinnamyle éventuellement substitué sur le noyau aromatique par un halogène, un hydroxyle, un alcoxy en C₁-C₄ ;
   . un groupe quinolyle ou isoquinolyle, éventuellement substitué par R₁, R_{1'} et R_{1"} tels que définis ci-dessus ;
   . un groupe benzothiazolyle-2 ;
   . un groupe quinoxalinyldione ;
   . un groupe phtalazinyl-1 ;
   . un groupe benzothiadiazolyle ;
   . un groupe méthylène substitué par un groupement hétérocyclique choisi parmi les groupes pyridyle et thiényle ;
- R_{Ia} représente un groupe bcnzyle substitué par R₁, R'₁ et R"₁ tels que définis ci-dessus ;
- R_{IV} représente un atome d'hydrogène, un atome d'halogène, un alkyle en C₁-C₆ ;
- R_{V} représente
   . un groupe 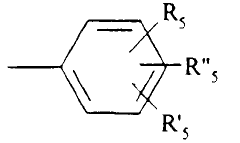
- où R₅, R'₅ et R"₅ représentent chacun indépendamment un atome d'hydrogène, un atome d'halogène, un alkyle droit ou ramifié en C₁-C₄, un hydroxyle, un alcoxy en C₁-C₄, un nitro, un trifluorométhyle, un trifluorométhoxy, un cyano, un amino, un carboxy, un carboxyalkyle en C₁-C₄, un phényle ;
   . un groupe naphtyle non substitué ou substitué par un alkyle en C₁-C₄ ;
   . un groupe pyridyle ;
   . un groupe styrylc non substitué ou substitué par un alkyle en C₁-C₄ ;
- ou bien R_{IV} et R_{V} considérés ensemble représentent :
   un groupe 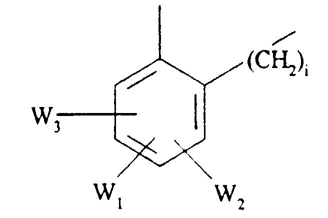 dans lequel le groupe phényle substitue le pyrazole en position 5 et le groupe -(CH₂)ᵢ- dans lequel i = 1 à 3 substitue le pyrazole en position 4, W₁, W₂ et W₃ substituent le cycle benzénique et représentent indépendamment l'hydrogène, un halogène ou un groupe hydroxyle ;
- R représente l'hydrogène, un alkylc en C₁-C₄ droit ou ramifié ;
- Z représente un groupe hydroxyle, un groupe alcoxy en C₁-C₆ ; un atome d'oxygène substitué par un groupe choisi parmi les groupes tertio-butyle, benzyle, benzyle substitué par un atome d'halogène, alkyle en C₁-C₆, trifluorométhyle, trifluorométhoxy et carboxy ; un groupe amino ;
- soit X représente l'hydrogène et X' représente l'hydrogène, un alkyle droit ou ramifié en C₁-C₆ ; un phényle ; un aminoalkyle en C₁-C₄ ; un hydroxyalkyle en C₁-C₄ ; un carboxyalkyle dans lequel le groupe alkyle est en C₁-C₄ ; un guanidinoalkylc dont le groupe alkyle est en C₁-C₄ ; un nitroguanidinoalkyle dont le groupe alkyle est en C₁-C₄ ; un cycloalkyle en C₃-C₇ ; un phénylalkyle dans lequel l'alkyle est en C₁-C₄ et dans lequel le phényle est éventuellement substitué par un halogène, un hydroxyle ou par un alkyle en C₁-C₃ ; un hétéroarylalkyle dans lequel l'hétéroaryle représente un imidazolyle, un indolyle non substitué ou substitué par un alkyle en C₁-C₄ ; par un hydroxy ou par un alcoxy en C₁-C₄ et dans lequel l'alkyle est en C₁-C₄ ; un groupe adamantyle ; un groupe adamantyle substitué par un ou deux méthyles, par un hydroxyle, un alcoxy en C₁-C₃ ; un atome d'halogène ; un groupe aza-1 adamantyle ;
- soit X représente l'hydrogène, X' et R considérés ensemble forment avec l'atome d'azote auquel R est lié et avec l'atome de carbone auquel X est lié un cycle . non substitué ou substitué par un hydroxyle, de formule : 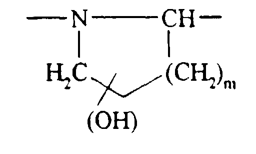 avec m = 0, 1 ou 2
   ou un cycle de formule : 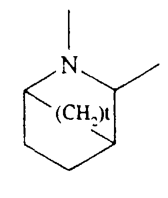 avec t = 1 ou 2
   ou un cycle de formule : 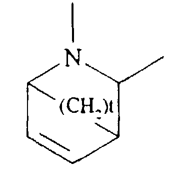 avec t = 1 ou 2
   ou un cycle indolinyle ; perhydroindole, tétrahydro-4,5,6,7 thiéno [2,3-c]pyridyl-6;
- soit X et X' représentent chacun indépendamment un alkyle en C₁-C₄ ; un cycloalkyle en C₃-C₆ ; un phényle ;
- ou X, X' et l'atome de carbone auquel ils sont liés forment un groupe cycloalkylidène ayant au maximum 12 atomes de carbone, éventuellement substitué par un alkyle en C₁-C₃ ; un groupe adamantylidène ; un groupe adamantylidène substitué par un ou deux groupes méthyle ou par un hydroxyle, un alcoxy en C₁-C₃, un atome d'halogène ; un groupe aza-1 adamantylidène ; un groupe quinuclidinylidène ; un groupe pipéridinylidène-4 éventuellement N- substitué par un groupe benzyle ; un groupe tétraméthyl-2,2,6,6 pipéridinylidène ; un groupe tétrahydronaphtylidène ; un groupe tétrahydropyranilidène-4 , ou tétrahydrothiopyranylidène-4 ; un groupe dihydro-2,3 (4H) benzopyranylidène-4; un groupe dihydro-2,3(4H)benzothiopyranylidène-4 ; un groupe bicyclo [2,2,1] heptène-5 ylidène-2 ; un groupe oxa-8 bicyclo [3,2,1] octèn-6-ylidène-3 ; un groupe thia-8 bicyclo [3,2,1]octanylidène-3 ;
- ou un de ses sels éventuels avec des acides organiques ou minéraux ou avec des bases minérales ou organiques.

Lorsque les composés (I) ou (I') incluent un carbone asymétrique, les énantiomères font partie de l'invention.

Les sels éventuels des produits de formule (I) ou (I') selon la présente invention comprennent aussi bien ceux avec des acides minéraux ou organiques qui permettent une séparation ou une cristallisation convenable des composés de formule (I) ou (I') tels que l'acide picnque ou l'acide oxalique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le naphtalène-2 sulfonate.

Les sels éventuels des produits de formule (I) ou (I') comprennent également les sels avec des cations, par exemple les sels des métaux alcalins ou alcalino-terreux comme les sels de sodium, de potassium, de calcium, le sel de sodium étant préféré, lorsque ledit produit de formule (I) ou (I') contient un groupe acide carboxylique.

Une classe particulière des composés de l'invention est constituée par les composés de formule (I) dans laquelle R₁ est soit un groupe naphtyle, soit une groupe phényle, substitué par R₁, R'₁ et R"₁ tels que définis ci-dessus, les autres substituants étant tels que définis ci-dessus.

Un autre groupe préféré des composés de l'invention est constitué des composés de formule (I) ou (I') dans laquelle R_{V} représente un groupe naphtyle ou phényle, substitué par R₅, R'₅ et R"₅ tels que définis précédemment, les autres substituants étant tels que définis précédemment. De préférence R₅, R'₅ ou R"₅ est l'hydrogène ou un alcoxy en C₁-C₄.

Un autre groupe préféré des composés de l'invention est constitué des composés de formule (I) ou (I') dans laquelle X, X' et l'atome de carbone auquel ils sont liés forment un groupe adamantyle.

Selon un autre de ses aspects, la présente invention concerne un procédé pour la préparation des composés de formule (I) et (I') caractérisé en ce que l'on traite un dérivé fonctionnel d'un acide pyrazolecarboxylique de formule (II) ou (II') : 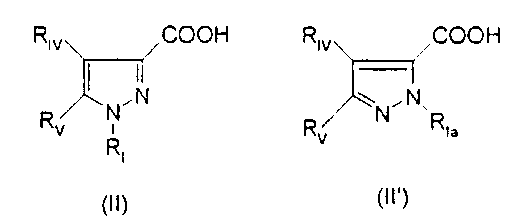 dans laquelle R_{I}, R_{IV}, R_{V} et Rᵢₐ sont tels que définis ci-dessus, avec un aminoacide, éventuellement protégé par les groupements protecteurs habituels en synthèse peptidique, de formule : 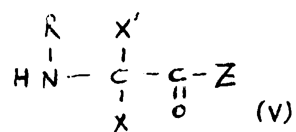 dans laquelle R, X, X' et Z sont tels que définis ci-dessus ou éventuellement protégés.

Comme dérivé fonctionnel de l'acide pyrazolecarboxylique de formule (II) ou (II'), on peut utiliser le chlorure d'acide, l'anhydride, un anhydride mixte, un ester, un ester activé, par exemple l'ester de p-nitrophényle, ou l'acide libre opportunément activé, par exemple, avec le N,N-dicyclohexylcarbodiimide ou avec l'hexafluorophosphate de benzotriazolyl-N-oxytris-(diméthylamino)-phosphonium. (BOP).

Les composés (I) et (I') ainsi préparés peuvent alors éventuellement être déprotégés pour conduire aux acides libres correspondants.

Les esters (IIa) et (II'a), précurseurs des acides carboxyliques (II) et (II'), définis ci-dessus, sont synthétisés en appliquant la méthode décrite dans Chem. Pharm. Bull, 1984, 32, 4, 1577.

Le procédé de préparation des composés (I) ou (I') via les esters (IIa) et (II'a) est représenté par le schéma suivant:

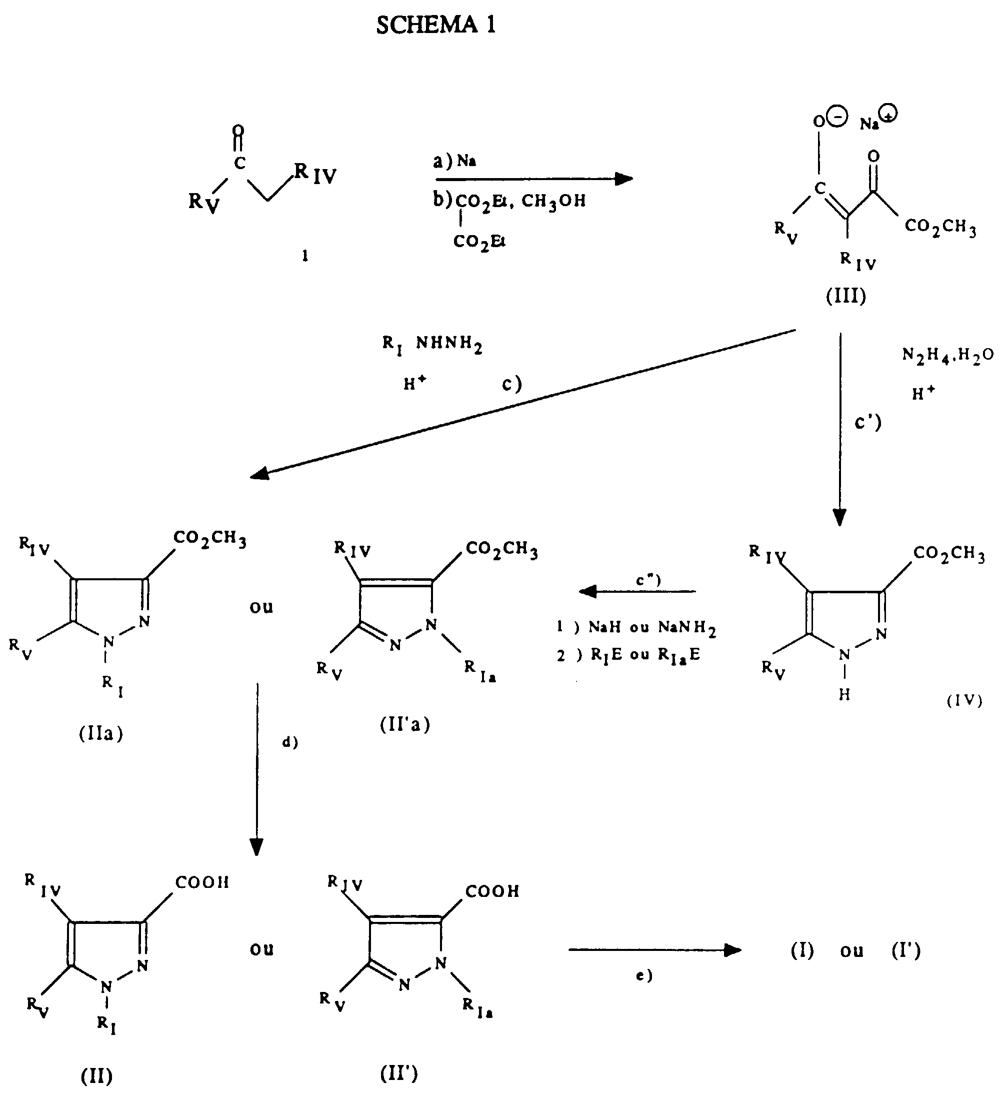

La première étape a) consiste en la préparation des énolates de sodium d'une cétone de formule 1 dans laquelle R_{V} et R_{IV} sont tels que définis précédemment, sur lesquels on fait agir une quantité équimolaire d'oxalate d'éthyle (étape b)) dans un alcanol comme par exemple le méthanol, selon L. CLAISEN, Ber., 1909, 42, 59. Après précipitation dans l'éther éthylique, les énolates de sodium (III) sont séparés par filtration.

Les énolates de sodium (III) ainsi préparés et un excès d'hydrazine ou d'un dérivé de l'hydrazine R_{I}-NHNH₂ sont alors chauffés au reflux de l'acide acétique (étape c)).

Dans le cas où R_{I} représente un groupement benzyle substitué ou non substitué R_{Ia}, on obtient, lors de la condensation de la benzylhydrazine sur les composés (III), un mélange en proportions variables selon la nature et la position des substituants de R_{V}, des composés (IIa) et de son isomère (II'a) de formule : 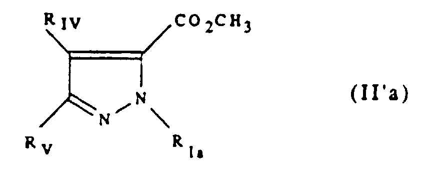 dans laquelle R_{Ia}, R_{IV} et R_{V} sont tels que définis précédemment.

Les deux isomères (IIa) et (II'a) peuvent alors être séparés par chromatographie sur colonne. Par saponification des esters, on obtient les acides isomères purs que l'on fait réagir par exemple avec le chlorure de sulfinyle. Les chlorures d'acides sont alors condensés sur les aminoacides de formule (V) pour conduire aux composés (I) et (I') selon l'invention (étape e)).

Une variante au procédé dans le cas où R_{I} est un groupe benzyle ou cinnamyle consiste en la condensation de l'hydrazine non substituée sur le composé (III) (étape c')) pour conduire au dérivé (1H) pyrazole (IV) qui est ensuite substitué, en présence de NaH ou de NaNH₂ par un groupement R_{I}E ou R_{Ia}E (étape c")) où E représente un groupe éliminable tel qu'un halogène, un p-toluènesulfonyloxy (tosyloxy) ou un méthanesulfonyloxy (mésyloxy).

Les dérivés d'amido-3 pyrazole (I) et (I'), objets de l'invention, sont alors préparés à partir des pyrazoles acides en transformant les dérivés esters (IIa) et (II'a) en leurs acides correspondants (II) ou (II') par action d'un agent alcalin comme par exemple l'hydroxyde de potassium puis acidification (étape d), puis les composés de formule (I) et (I') correspondants sont alors préparés comme décrit précédemment.

Si l'aminoacide comporte un groupe hydroxyle comme substituant, celui-ci peut être protégé par un groupe O-protecteur habituellement utilisé puis déprotégé selon les méthodes habituelles.

Lorsque le produit de formule (I) ou (I') présente une fonction basique et est obtenu sous forme de base libre, la salification est effectuée par traitement avec l'acide choisi dans un solvant organique. Par traitement de la base libre dissoute par exemple dans un alcool tel que l'isopropanol, avec une solution de l'acide choisi dans le même solvant, on obtient le sel correspondant qui est isolé selon les techniques conventionnelles. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate le fumarate, le naphtalène-2 sulfonate.

Lorsque le composé de formule (I) ou (I') présente une fonction basique et est isolé sous forme d'un de ses sels, par exemple le chlorhydrate ou l'oxalate, la base libre peut être préparée par neutralisation dudit sel avec une base minérale ou organique telle que l'hydroxyde de sodium ou la triéthylamine ou avec un carbonate ou bicarbonate alcalin, tel que le carbonate ou bicarbonate de sodium ou de potassium.

Lorsque que le produit de formule (I) ou (I') contient un groupe acide, le composé ainsi obtenu peut être transformé en un sel métallique, notamment alcalin, tel que le sel de sodium, ou alcalino-terreux, tel que le sel de calcium, selon les procédés classiques.

Les composés (I) ou (I') selon l'invention ont fait l'objet d'essais biochimiques.

Les mêmes composés (I) ou (I') et leurs sels déplacent, à des concentrations inférieures à la micromole, la neurotensine [iodée Tyr³] de son récepteur, sur des membranes de cerveau de cobaye, selon la méthode décrite par SADOUL J.L. et al., Biochemical and Biophysical Research Communications, 1984, 120, 3, 812-819.

Les composés de la présente invention sont peu toxiques ; notamment, leur toxicité aiguë est compatible avec leur utilisation comme médicament. Pour une telle utilisation, on administre aux mammifères une quantité efficace d'un composé de formule (I) ou (I') ou d'un de leurs sels pharmaceutiquement acceptables.

Les composés (I) ou (I') selon l'invention sont les premiers médicaments potentiels de synthèse, non peptidiques, capables de se lier au récepteur de la neurotensine et pouvant être utiles dans les états pathologiques associés à un dysfonctionnement des systèmes dopaminergiques, par exemple comme antipsychotiques (D.R. HANDRICH et al., Brain Research, 1982, 231, 216-221 et C.B. NEMEROFF, Biological Psychiatry, 1980, 15-2, 283-302), dans les désordres des systèmes cardiovasculaire ou gastrointestinal.

Ainsi, la présente invention a pour objet, selon un autre de ses aspects, des compositions pharmaceutiques contenant, comme principes actifs, les composés de formule (I) ou (r) ou leurs sels éventuels pharmaceutiquement acceptables.

Dans les compositions pharmaceutiques de la présente invention pour l'administration orale, sublinguale, sous cutanée, intramusculaire, intraveineuse, transdermique ou rectale, les principes actifs peuvent être administrés, sous formes unitaires d'administration, en mélange ou avec des supports pharmaceutiques classiques, aux animaux et aux êtres humains. Les formes unitaires d'administration appropriées comprennent les formes pour voie orale telles que les comprimés, les gélules, les poudres, les granules et les solutions ou suspensions orales, les formes d'administration sublinguale et buccale, les formes d'aministration sous-cutanée, intramusculaire ou intraveineuse et les formes d'aministration rectale.

Afin d'obtenir l'effet désiré, la dose de principe actif peut varier entre 1 et 1000 mg par jour, de préférence entre 2 et 500 mg.

Chaque dose unitaire peut contenir de 1 à 250 mg de principe actif, de préférence de 2 à 125 mg, en combinaison avec un support pharmaceutique. Cette dose unitaire peut être administrée 1 à 4 fois par jour.

Lorsqu'on prépare une composition solide sous forme de comprimés, on mélange le principe actif avec un véhicule pharmaceutique tel que la gélatine, l'amidon, le lactose, le stéarate de magnésium, le talc, la gomme arabique, ou analogues. On peut enrober les comprimés de saccharose ou d'autres matières appropriées ou encore on peut les traiter de telle sorte qu'ils aient une activité prolongée ou retardée et qu'il libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, tels que la polyvinylpyrrolidone et similaires, de même qu'avec des édulcorants ou des correcteurs du goût

Pour une administration rectale on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

Les points de fusion instantanés (F) des produits cristallisés ont été pris au banc chauffant Kofler et sont exprimés en degrés Celsius. Dans les tableaux qui suivent, les abréviations suivantes ont été utilisées :
- CH: cyclohexane
- CH₂Cl₂: dichlorométhane
- EtOH: éthanol
- Et₂O: diéthyléther
- Hx: hexane
- Pn: pentane
- iPr₂O: éther diisopropylique
- iPrOH: isopropanol
- AcOEt: acétate d'éthyle
- MeOH: méthanol
- C*: signifie configuration du carbone asymétrique.

Les abréviations suivantes sont utilisées dans les spectres de RMN :
- M: multiplet
- S: singulet
- SE: singulet élargi
- D: doublet
- Har: H aromatique
o : ortho ; m : méta

### PREPARATION DES INTERMEDIAIRES DE SYNTHESE.

### A. Préparation des dérivés de l'hydrazine (R_{I} NHNH₂).

Un grand nombre de dérivés de l'hydrazine sont des produits commerciaux.

les autres ont été préparés selon des méthodes connues par diazotation de l'amine aromatique correspondante puis réduction du sel de diazonium. Ainsi, on peut citer à titre d'exemple la préparation de :
- la tétrahydro-5,6,7,8 naphtyl-1 hydrazine, selon R. FUSCO et al., Gazz. Chim. Ital., 1974, 104, 813-817 ;
- l'hydrazino-8 quinoléine, selon A. ALBERT et al., J. Chem. Soc., 1967, 1533-1541 ;
- l'hydrazino-5 quinoléine et l'hydrazino-5 isoquinoléine, selon M.G. FERLIN et al., I1 Farmaco, 1989, 44 (12), 1141-1155.

### B. Préparation des acides pyrazole carboxyliques (II) :

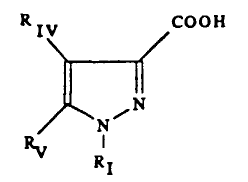

Elle est effectuée selon le mode opératoire décrit précédemment. Le tableau A ci-dessous indique, à titre d'exemples et d'une façon non limitative, les caractéristiques d'acides de formule (II).

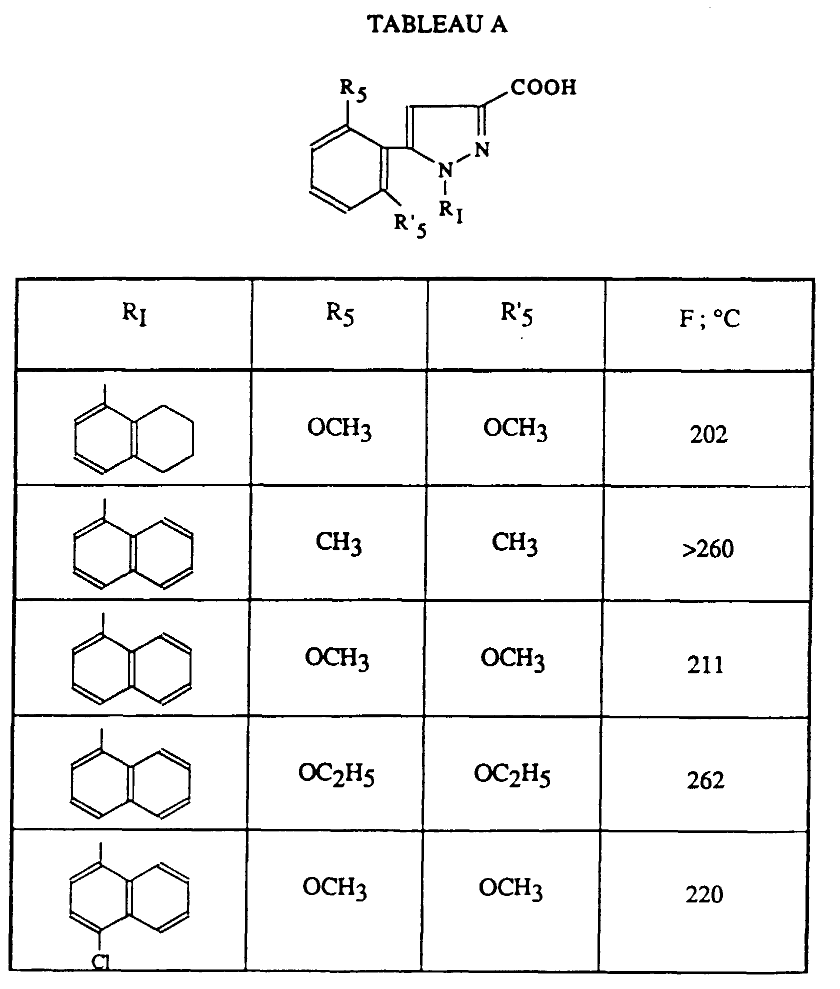

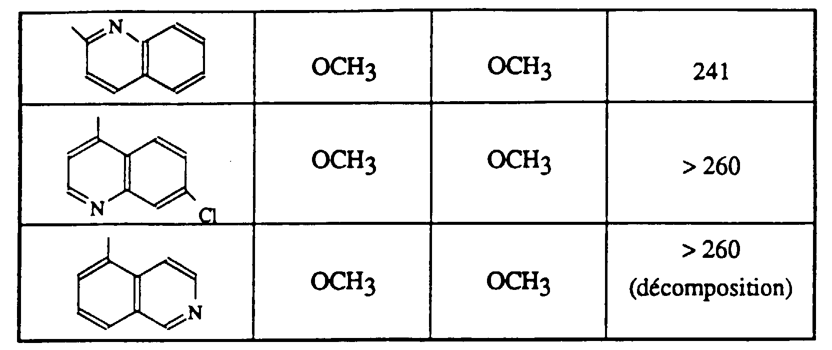

### C. Préparation des aminoacides.

Les produits non commerciaux sont préparés selon la synthèse de STRECKER (Ann., 75, 27, 1850) ou selon la synthèse de H.T.BUCHERER et al., J. Pract. Chem., 1934, 141, 5, suivie d'une hydrolyse pour conduire aux aminoacides ; par exemple, l'acide amino-2 adamantane carboxylique-2 est préparé selon H.T. NASANTA et al. J. Med. Chem., 1973, 16 (7), 823.

Les acides α-aminocycloalcane carboxyliques sont préparés selon J.W. TSANG et al., J. Med. Chem., 1984, 27, 1663.

Les cyclopentylglycines R et S sont préparées par résolution de la benzyloxycarbonylcyclopentylglycine.
1) Préparation de la benzyloxycarbonylcyclopentylglycine racémique
   Ce composé est préparé en opérant selon le schéma réactionnel ci-après (schéma 2) 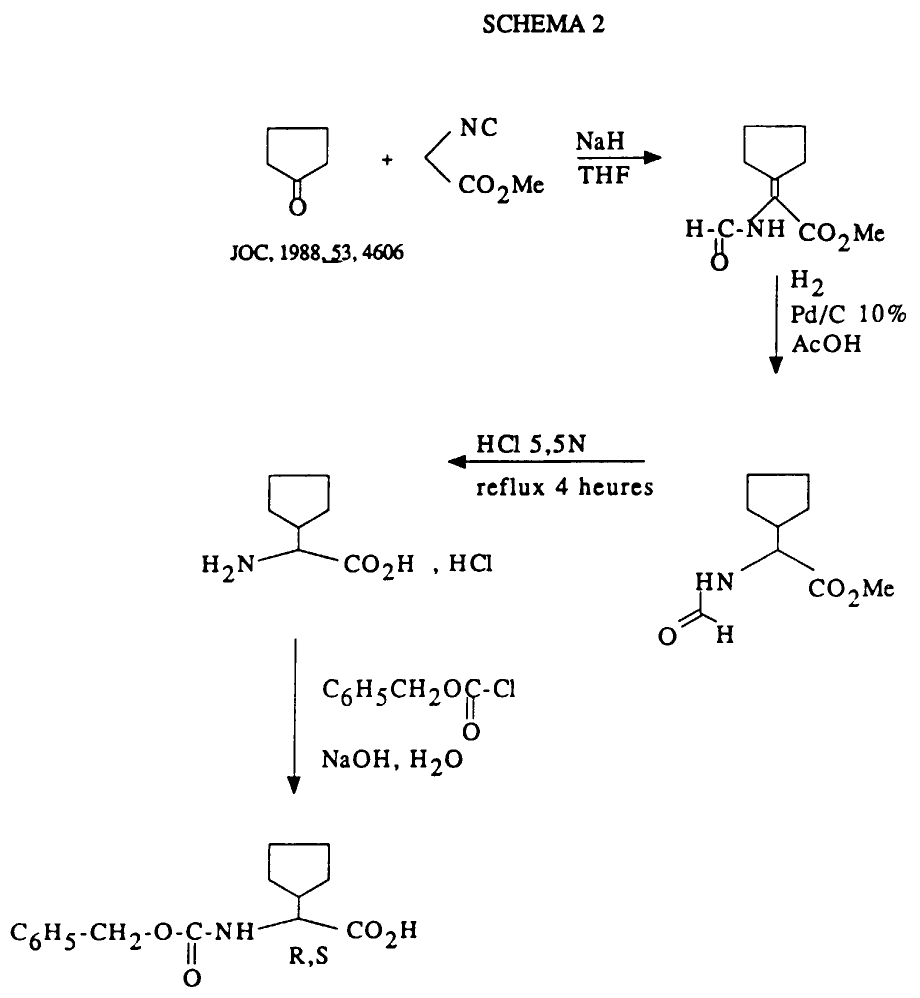
2) Chlorhydrate de la cyclopentylglycine R,S.
   On dissout du NaH à 80% (1,8 g) dans du THF anhydre (50 ml). On ajoute goutte à goutte en agitant un mélange de cyclopentanone (4,2 g) et d'isocyanoacétate de méthyle (5 g) dans du THF (50 ml). L'addition terminée, on laisse 2 heures . On refroidit à 5°C et on ajoute lentement de l'acide acétique en solution aqueuse à 10% (50 ml). On évapore le THF sous vide. Le résidu aqueux est extrait par du chloroforme (3 x 120 ml). On sèche sur Na₂SO₄ et concentre sous vide.
   Le résidu est repris au pentane, filtré et lavé au pentane.
   Le solide (7,6 g) est dissous dans de l'acide acétique (100 ml). On ajoute du palladium sur charbon à 10% (3 g) et on agite à pression atmosphérique et température ambiante sous hydrogène pendant 24 heures (on absorbe 1 litre d'hydrogène). On filtre sur celite, lave à l'acide acétique plusieurs fois. On évapore sous vide. Le résidu est repris dans de l'acide chlorhydrique 5,5 N (70 ml). On chauffe à reflux 4 heures. On concentre à sec, on azéotrope plusieurs fois au toluène et on sèche sous vide. On obtient le produit attendu.
   m = 7,2 g
   RMN D₂O : 8 H à 1,6 (M, CH₂ cycle); 1 H à 2,20 (M, CH cycle) ; 1 H à 3,80 (D, J=7 CHCO₂H); 3 H à 8,60 (SE, NH₃ ⁺)
3) Acylation avec le chloroformiate de benzyle.
   On dissout le chlorhydrate de cyclopentylglycine R,S (7,2 g) dans une solution d'hydroxyde de sodium 2 N (65 ml). On ajoute goutte à goutte le chloroformiate de benzyle (8,5 g) dans le THF (30 ml) en refroidissant à 5°C. On laisse agiter la nuit à température ambiante. On refroidit dans de la glace. On acidifie avec HCl concentré jusqu'à pH=2 (T ≤ 5°C). On extrait au chloroforme, sèche et évapore.
   Le résidu est repris au pentane. On obtient la benzyloxycarbonylcyclopentylclycine R,S.
   F = 110°C.
4) Résolution de la benzyloxycarbonylcyclopentylglycine.
   On dissout la benzyloxycarbonylcyclopentylglycine (5,54 g) dans de l'éthanol absolu (65 ml). On ajoute la diphényl-1,2 éthanol-1 amine-2 (1 R, 2 S) (-), préparée selon J. WEIJLARD et al., J. Am. Chem. Soc. 1951, 73, 1216. On chauffe jusqu'à dissolution. On laisse précipiter la nuit et on filtre. On obtient 2.8 g du sel (F = 175°C). On garde les eaux mères.
   Le sel obtenu est repris par de l'eau (20 ml), de l'HCl (30 ml) et de l'éther (100 ml). On agite jusqu'à dissolution. La phase organique est décantée, séchée, évaporée. On obtient la benzyloxycarbonylcyclopentylglycine que l'on traite tout de suite par HCl concentré (15 ml), AcOH (15 ml). On chauffe à reflux 3 heures. On évapore à sec. Le résidu est repris par de l'éther sec filtré et séché. On obtient le chlorhydrate de la (S) cyclopentylglycine.
   [α]_{D}²⁵ = + 10°,4 (c=0,5 HCI N)
   m = 0,6 g.
   Les eaux mères sont évaporées à sec et reprises par H₂O (50 ml) HCl (60 ml) Et₂O (300 ml). On agite, tout est dissous. On décante la phase éthérée, sèche et évapore. On récupère la benzyloxycarbonylcyclopentylglycine (4,3 g), on la met dans de l'éthanol absolu (50 ml) avec de la diphényl-1,2 éthanol-1 amino-2 (1 S, 2 R) (+) (3,30 g). On chauffe jusqu'à dissolution, on laisse reposer la nuit, on filtre.
   On obtient 4,15 g de sel.
   F = 175°C.
   Ce sel est repris par de l'eau (20 ml), de l'HCl N (40 ml), de l'éther (200 ml). On agite. La phase éthérée est séchée, évaporée, puis on traite le résidu par de l'HCl concentré (10 ml), de l'acide acétique (100 ml). On chauffe le mélange pendant 3 heures à reflux, concentre sous vide, reprend à l'éther anhydre pour obtenir le chlorhydrate de la (R) cyclopentylglycine.
   m = 1,2 g.
   [α]_{D}²⁵ = -10,5 (c= 0,85 HCl N)
   Pureté optique de la R cyclopentylglycine :
   0,10 g du chlorhydrate ci-dessus sont dissous dans du méthanol absolu. On refroidit à -40°C, ajoute 0,5 ml de chlorure de thionyle et laisse le mélange pendant 24 heures à température ambiante . On concentre sous vide, reprend le résidu dans le chloroforme anhydre (20 ml), on ajoute de la triéthylamine (0,2 ml) et le (S) phénylméthylisocyanate (0,074 ml). On laisse 24 heures puis évapore le chloroforme. Le résidu est chromatographié sur gel de silice, éluant : acétate d'éthyle. La concentration des fractions pures fournit 0,1 g de l'ester méthylique.
   Le spectre de RMN dans le CDCl₃ montre, autour de 3,8 ppm, la présence de deux signaux pour le -CO₂CH₃. L'intégration montre que le signal le plus faible réprésente 4%, le signal le plus intense 96%.
   L'excès énantiomérique est donc de 92%.

On peut aussi préparer les cycloalkyl-α-amino acides de configuration R ou S par hydrolyse enzymatique, stéréospécifique, des dérivés N-acétylés racémiques correspondants, selon J. HILL et al. J. Org. Chem., 1965, 1321.

### EXEMPLE 1

### Ester méthylique de l'acide { [phényl-1 (pyridyl-4)-5 pyrazolyl-3] carbonylamino}-2 méthyl-4 pentanoïque (S)..

(I) : R=H; X'=H ; X=-CH₂-CH-(CH₃)₂ ; Z=OCH₃ ; R_{I}=C₆H₅ ; R_{IV}=H ;

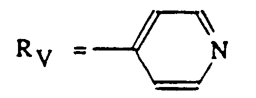

0,35 g d'acide phényl-1 (pyridyl-4)-5 pyrazole carboxylique-3 sont mis en solution dans 5 ml de diméthylformamide en présence de 0,45 ml de diisopropyléthylamine (DIPEA) et de 0,59 g d'hexafluorophosphate de benzotriazolyl N-oxytrisdiméthylaminophosphonium (BOP). On ajoute alors 0,23 g (1 équivalent) de chlorhydrate de l'ester méthylique de la (S) leucine en solution dans 0,4 ml de DIPEA et le mélange réactionnel est abandonné pendant une nuit à température ambiante. Les solvants sont concentrés sous vide, l'huile résiduelle est extraite au dichlorométhane, cette solution est lavée à l'eau puis avec une solution de bicarbonate de sodium et encore à l'eau. La phase organique est séchée sur sulfate de sodium puis concentrée sous vide. Le résidu est chromatographié sur gel de silice, éluannt : acétate d'éthyle.

m = 0,18 g.

Spectre de RMN H du composé 1 : 3H à 8,82 (M, Har o N et CONH) ; 5H à 7,50 (M, Har Phe) ; 3H à 7,27 (Har m N et H₄ pyrazole); 1H à 4,60 (M, H α Leu) ; 3H à 3,77 (S, CO₂CH₃) ; 1H à 2,00 (M, H γ Leu) ; 2H à 1,70 (M, H β Leu) ; 6H à 1,00 (2D, CH₃ Leu).

### EXEMPLE 2

### Acide {[phényl-1 (naphtyl-2)-5 pyrazolyl-3] carbonylamino}-2 phényl-3 propanoïque (S).

(I) : R=H; X'=H ; X=-CH₂-C₆H₅ ; Z=OH ; R_{I}=C₆H₅ ; R_{IV}=H ; 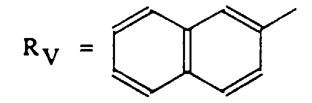 Préparation du chlorure de l'acide (naphtyl-2)-5 phényl-1 pyrazole carboxylique-3.
5 g de l'acide (naphtyl-2)-5 phényl-1 pyrazole carboxylique-3 sont mis en solution dans 56 ml de toluène, et 3,5 ml de chlorure de sulfinyle sont ajoutés goutte à goutte à cette solution. Le mélange est chauffé à 90° pendant 2 h 1/2, puis concentré sous vide. L'huile résiduelle est reprise deux fois dans le toluène et concentrée sous vide.
m = 5 g.
Préparation du composé 2.

A 60 ml d'une solution d'hydroxyde de sodium 2N on ajoute 4,9 g de (S) phénylalanine, puis goutte à goutte une solution de 4 g de chlorure d'acide préparé précédemment en solution dans 65 ml de tétrahydrofuranne. Le mélange réactionnel est laissé une nuit à température ambiante puis concentré sous vide. Le résidu est repris dans l'eau et le pH est ajusté à 1 par addition d'acide chlorhydrique. La solution est extraite au dichlorométhane et la phase organique est lavée à l'eau, avec une solution saturée de chlorure de sodium, séchée sur sulfate de sodium, filtrée et concentrée sous vide. Le résidu est recristallisé du pentane.

m = 2 g.

F = 226°C.

### EXEMPLE 3

### N,N-diéthyl{[phényl-1 (naphtyl-2)-5 pyrazol-3] carbonylamino}-2 phényl-3 propanamide (S).

(I) : R=H; X'=H ; X=-CH₂-C₆H₅ ; Z=-N-(C₂H₅)₂ ; R_{I}=C₆H₅ ; R_{IV}=H ;

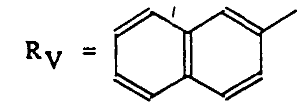

2 g du produit obtenu selon l'exemple 2, 0,88 g de dicyclohexylcarbodiimide (DCCI) et 1,14 g d'hydroxy-1 benzotriazole (HOBT) sont mis en solution dans 68 ml de tétrahydrofuranne et le mélange est agité pendant 3/4 d'heure à température ambiante. On ajoute ensuite 0,4 g de diéthylamine et laisse le mélange réactionnel à température ambiante pendant 24 heures.

La dicyclohexylurée est séparée par filtration et les eaux mères sont concentrées sous vide. Le résidu est chromatographié sur gel de silice, éluant : acétate d'éthyle. Les fractions de produit pur sont concentrées sous vide et le résidu est recristallisé du pentane.

m = 1,46 g.

F = 70°C.

### EXEMPLE 4

### Acide {[phényl-1 dihydro-4,5 benz (g) indazolyl-3] carbonylamino}-2 méthyl-4 pentanoïque (S).

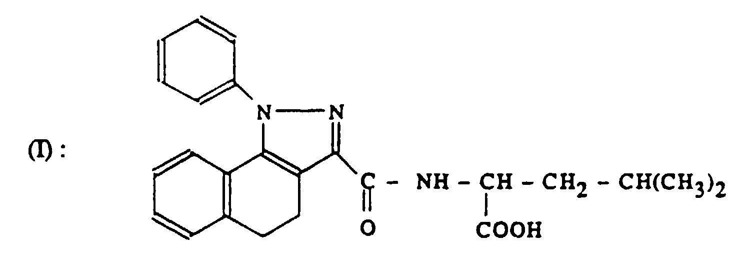

A) Sel de sodium de β-cétocarbéthoxy α-tétralone.
   Cet intermédiaire est préparé selon la méthode décrite par D. RAMESH et al. Indian Journal of Chemistry, 1989, 28B, 76-78.
B) Ester éthylique de l'acide phényl-1 dihydro-4,5 benz (g) indazole carboxylique-3.
   8,04 g du sel de sodium obtenu précédemment sont mis en solution dans 100 ml d'acide acétique. On ajoute 3,3 ml de phénylhydrazine et chauffe le mélange réactionnel à reflux pendant 8 heures. Le mélange refroidi est versé dans l'eau glacée, un précipité est séparé par filtration, lavé à l'eau puis au pentane.
   m = 10,5 g.
C) Acide phényl-1 dihydro-4,5 benz (g) indazole carboxylique-3.
   9,5 g du produit obtenu précédemment sont dissous dans 100 ml de méthanol et 100 ml d'eau. On ajoute 4,2 g d'hydroxyde de potassium et chauffe le mélange réactionnel à reflux pendant 5 heures. Le mélange est versé dans l'eau glacée puis on lave à l'acétate d'éthyle. La phase aqueuse est acidifiée à pH = 2 par addition d'acide chlorhydrique et un précipité est séparé par filtration, lavé à l'eau puis au pentane.
   m = 7,3 g
D) Chlorure de l'acide phényl-1 dihydro-4,5 benz (g) indazole carboxylique-3.
   2,8 g de l'acide obtenu précédemment sont mis en solution dans 100 ml de toluène puis on ajoute 2,2 ml de chlorure de sulfinyle et chauffe à 100°C pendant 5 heures. La solution est concentrée sous vide, on ajoute 20 ml de toluène et concentre sous vide. On répète deux fois la même opération.
E) Composé 4
   0,88 g de S-leucine est dissous dans une solution de 1,33 g d'hydroxyde de sodium dans 20 ml d'eau. Cette solution est refroidie, puis on y ajoute 0,99 g du chlorure de l'acide préparé précédemment en solution dans 16 ml de tétrahydrofuranne et laisse le mélange réactionnel sous agitation à température ambiante pendant 18 heures. La solution est concentrée sous vide, le résidu est repris dans la glace et acidifié à pH = 2 par addition d'acide chlorhydrique, puis extrait à l'acétate d'éthyle. La phase organique est séchée sur sulfate de sodium, filtrée et concentrée sous vide. Le résidu est recristallisé de l'éther isopropylique.
   m = 1 g
   F = 100°C

### EXEMPLE 5

### Acide {[benzyl-1 (naphtyl-2)-3 pyrazolyl-5] carbonylamino}-2 phényl-3 propanoïque (S).

(I'):R=H ; X'=H ; X=-CH₂-C₆H₅ ; Z=OH ; R_{Ia}=CH₂-C₆H₅ ; R_{IV}=H ; 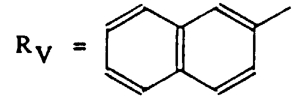
A) La réaction du naphtoyle-2 pyruvate de méthyle avec le chlorhydrate de benzylhydrazine fournit un mélange des esters suivants : ester méthylique de l'acide benzyl-1 (naphtyl-2)-5 pyrazole carboxylique-3 et ester méthylique de l'acide benzyl-1 (naphtyl-2)-3 pyrazole carboxylique-5.
   Une chromatographie sur gel de silice permet la séparation des deux isomères. L'ester méthylique de l'acide benzyl-1 (naphtyl-2)-5 pyrazole carboxylique-3 est élué en premier par un mélange acétate d'éthyle/hexane 50/50 (v/v). L'ester méthylique de l'acide benzyl-1 (naphtyl-2)-3 pyrazole carboxylique-5 est élué en deuxième fraction.
B) Acide benzyl-1 (naphtyl-2)-3 pyrazole carboxylique-5.
   L'acide a été préparé par saponification de l'ester précédemment obtenu.
C) Chlorure de l'acide benzyl-1 (naphtyl-2)-3 pyrazole carboxylique-5
   Le chlorure d'acide est préparé par action du chlorure de sulfinyle sur l'acide précédent et n'est pas isolé.
D) Composé 5.
   0,28 g de phénylalanine (S) est dissous dans une solution d'hydroxyde de sodium refroidie. On ajoute alors une solution de 0,3 g du chlorure d'acide préparé précédemment dans 5 ml de THF et on laisse le mélange réactionnel à température ambiante pendant 24 heures. Le THF est concentré sous vide, le résidu est repris dans l'eau, neutralisé par addition d'acide chlorhydrique concentré. On extrait à l'acétate d'éthyle, sèche sur sulfate de sodium et concentre sous vide. Le résidu est recristallisé du cyclohexane.
   m = 1 g
   F = 100°C

### EXEMPLE 6

### Ester méthylique de l'acide {[(méthoxy-4' cinnamyl)-1 (pyridyl-4)-5 pyrazolyl-3] carbonylamino}-2 méthyl-4 pentanoïque (S).

(I): R=H ; X'=H ; X=-CH₂-CH-(CH₃)₂ ; Z=OCH₃ ; 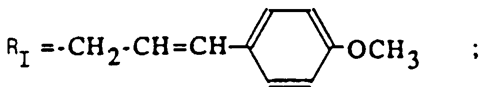 R_{IV}=H ; 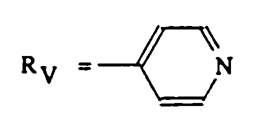
A) Ester méthylique de l'acide (méthoxy-4' cinnamyl)-1 (pyridyl-4)-5 pyrazole carboxylique-3.
   4,6 g de l'ester méthylique de l'acide (pyridyl-4)-5 (1H) pyrazole carboxylique-3 sont mis en solution dans 60 ml de diméthylformamide puis on ajoute 0,63 g d'hydrure de sodium en suspension dans l'huile à 80 %, et chauffe le mélange réactionnel à 40°C pendant 1 heure. On ajoute alors au mélange refroidi une solution de 5,2 g de méthoxy-4' bromure de cinnamyl-1 en solution dans 60 ml de diméthylformamide et laisse le mélange réactionnel à température ambiante pendant 12 heures. On concentre le diméthylformamide sous vide, reprend le résidu dans l'eau, extrait à l'acétate d'éthyle, sèche la phase organique sur sulfate de sodium, filtre et concentre sous vide. L'huile résiduelle est chromatographiée sur gel de silice, éluant : acétate d'éthyle/cyclohexane 50/50 (v/v). Les fractions de produit pur sont concentrées sous vide.
   m = 2,6 g
   F= 118°C
B) Composé 6
   0,4 g de l'acide obtenu précédemment est mis en solution dans 12 ml de diméthylformamide en présence de 0,63 ml de DIPEA et 0,53 g de BOP. On ajoute alors 0,22 g de chlorhydrate de l'ester méthylique de la (S)-leucine en solution dans 0,63 ml de DIPEA et le mélange réactionnel est abandonné pendant une nuit à température ambiante. Le diméthylformamide est concentré sous vide et le résidu est repris dans l'eau. On extrait à l'acétate d'éthyle, sèche la phase organique sur sulfate de sodium, filtre et concentre sous vide. Le résidu est concrétisé dans l'éther diisopropylique.
   m= 0,15 g
   F=172°C

### EXEMPLE 7

### Sel de sodium de l'acide {[(méthoxy-4' cinnamyl)-1 (pyridyl-4)-5 pyrazolyl-3] carbonylamino}-2 phényl-3 propanoïque (S).

(I): R=H ; X'=H ; X=-CH₂-C₆H₅ ; Z=O⁻ Na⁺ 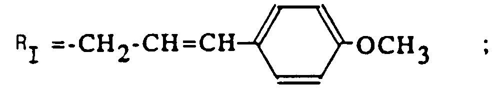 R_{IV}=H ;

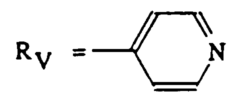

En procédant comme pour l'exemple 6 et en remplaçant le chlorhydrate de l'ester méthylique de la (S)-leucine par le chlorhydrate de l'ester méthylique de la (S)-phénylalanine, on obtient l'ester méthylique que l'on hydrolyse en sel de sodium avec 0,9 équivalent d'hydroxyde de sodium dans 10 ml d'éthanol 96°. Le mélange est laissé une nuit à température ambiante, concentré sous vide et le résidu est lavé à l'éther. Après filtration, on obtient le composé 7.
F = 137°C.

### EXEMPLE 8 Acide {[(isoquinoléinyl-5)-1 (diméthoxy-2,6 phényl)-5 pyrazolyl-3] carbonylamino}-2 adamantanecarboxylique-2.

(I) : R=H ; 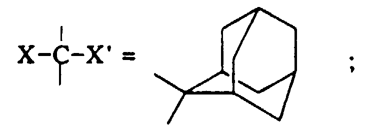 Z = OH ; 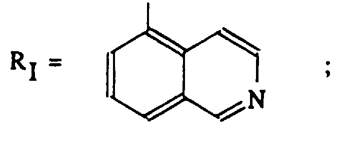 R_{IV} = H ;

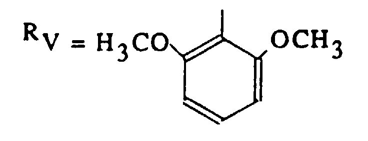

0,75 g de l'acide amino-2 adamantanecarboxylique-2 est mis en solution dans 20 ml de pyridine. On ajoute 1,4 g de chlorure de l'acide (isoquinoléinyl-5)-1 (diméthoxy-2,6 phényl)-5 pyrazolecarboxylique-3 en solution dans 20 ml de dichlorométhane et laisse le mélange réactionnel pendant une nuit à température ambiante. On concentre sous vide, reprend le résidu par du tampon pH=2, agite, filtre le précipité et rince à l'éther diisopropylique.

m = 0,4 g

F > 260°C

### EXEMPLE 9

### Acide{[(quinoléinyl-5)-1 (diméthoxy-2,6 phényl)-5 pyrazolyl-3] carbonylamino}-2 adamantanecarboxylique-2.

(I) : R=H ; 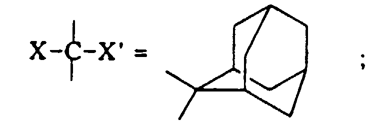 Z = OH ; 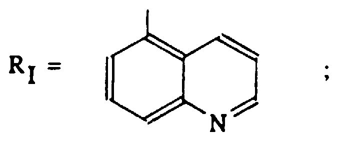 R_{IV} = H ;

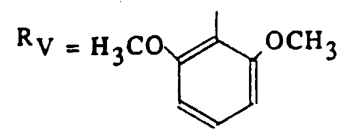

0,23 g de l'acide amino-2 adamantanecarboxylique-2, 0,5 g du chlorure de l'acide (quinoléinyl-5)-1 (diméthoxy-2,6 phényl)-5 pyrazolecarboxylique-3, et 0,7 g d'hydroxyde de potassium sont mis en solution dans 25 ml de dichlorométhane en présence de 0,1 g d'Aliquat 336®

Le mélange réactionnel est agité pendant une nuit à température ambiante, on ajoute 0,7 g d'hydroxyde de potassium et agite pendant 4 heures. Le mélange est filtré et on obtient 0,2 g du produit attendu.

F > 260°C

### EXEMPLE 10

### Acide{[(chloro-4 naphtyl-1)-1 (dihydroxy-2,6 phényl)-5 pyrazolyl-3] carbonylamino}-2 hexanoïque (S).

(I) : R=H ; X'= H ; X = (CH₂)₃-CH₃ Z = OH ; 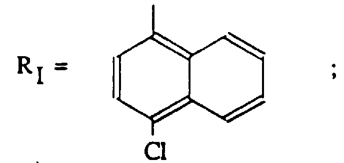 R_{IV} = H ;

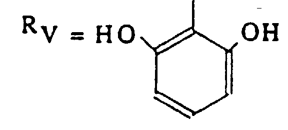

0,3 g d'acide [(chloro-4 naphtyl-1)-1 (diméthoxy-2,6 phényl)-5 pyrazolyl]-3 carbonylamino-2 hexanoïque est mis en solution dans 6,7 ml de dichlorométhane et on refroidit à -70°C. On ajoute goutte à goutte 5,7 ml de tribromure de bore en solution dans 20 ml de dichlorométhane et laisse le mélange réactionnel 2 heures à -70°C. On laisse revenir à température ambiante puis ajoute, en refroidisssant, 12 ml d'eau. On additionne NaOH concentrée jusqu'à pH=14. On lave la phase aqueuse à l'éther, et on l'amène à pH=₂, on extrait à l'acétate d'éthyle, sèche sur sulfate de sodium, filtre et évapore. On cristallise le résidu de l'éther diisopropylique.
m = 0,13 g.
F > 260°C

### EXEMPLE 11

### Acide {[(naphtyl-1)-1 (diméthoxy-2,6 phényl)-5 pyrazolyl-3] carbonylamino}-2 adamantanecarboxylique-2.

(I) : R=H ; 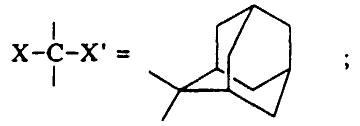 Z = OH ; 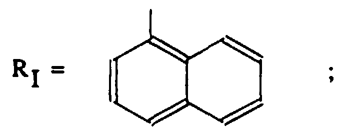 R_{IV} = H ;

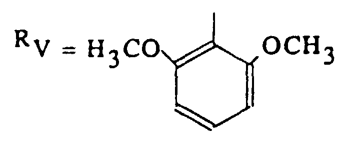

0,107 g d'hydroxyde de sodium dans 1,36 ml d'eau, et 0,51 ml de tétrahydrofuranne sont refroidis à 0°C. On ajoute en une seule portion 0,52 g d'acide amino-2 adamantane carboxylique-2 puis goutte à goutte 0,53 g du chlorure de l'acide (naphtyl-1)-1 (diméthoxy-2,6 phényl)-5 pyrazolecarboxylique-3 en solution dans 3 ml de tétrahydrofuranne. Le mélange est laissé pendant 10 minutes et on ajoute à nouveau la même quantité du chlorure d'acide précédent dans 3 ml de tétrahydrofuranne ; simultanément on ajoute 1,32 ml d'hydroxyde de sodium 2 N. Le mélange réactionnel est abandonné 4 jours à température ambiante ; on ajoute successivement de l'eau glacée, de l'acide chlorhydrique concentré jusqu'à pH=1 et filtre le précipité. Les cristaux sont lavés à l'éther diisopropylique.

m = 0,48 g

F > 260°C

### EXEMPLE 12

### {[(Naphtyl-1)-1 (diméthoxy-2,6 phényl)-5 pyrazolyl-3] carbonylamino}-2 adamantanecarboxylate-2 de méthyle.

(I) : R=H ; 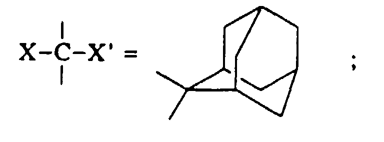 Z = OCH₃ ; 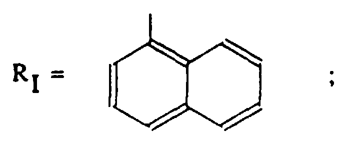 R_{IV} = H ;

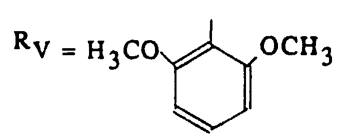

0,5 g du composé préparé à l'exemple 11 est dissous dans 34,6 ml de tétrahydrofuranne anhydre et 4 ml de diméthylformamide. On ajoute 3,5 ml d'eau et 0,208 g de carbonate de césium et laisse le mélange réactionnel à température ambiante pendant 1 heure. On concentre sous vide et azéotrope avec du toluène. Le résidu est repris dans 5 ml de tétrahydrofuranne. On ajoute 0,6 ml d'iodure de méthyle et laisse le mélange réactionnel pendant 1 heure à température ambiante. On concentre sous vide, reprend le résidu dans l'eau, agite et sépare le précipité par filtration. Le précipité est lavé à l'eau et au pentane.

m = 0.38 g

F = 242-244°C

### EXEMPLE 13

### Acide {[(chloro-7 quinoléinyl-4)-1 (diméthoxy-2,6 phényl)-5 pyrazolyl-3] carbonylamino}-2 adamantanecarboxylique-2,

(I) : R=H ; 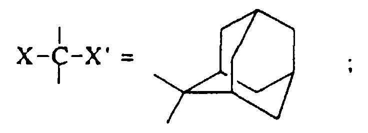 Z = OH ; 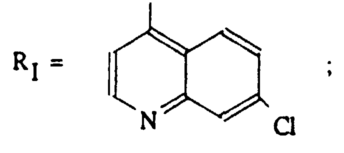 R_{IV} = H ;

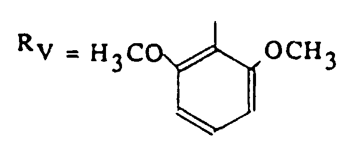

En procédant selon l'exemple 8 et en remplaçant le chlorure d'acide par le chlorure de l'acide (chlore-7 quinoléinyl-4)-1 (diméthoxy-2,6 phényl)-5 pyrazolecarboxylique-3, on obtient le composé intermédiaire de formule : 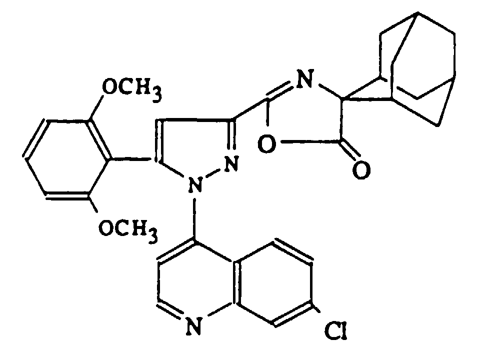 dont le point de fusion est 249°C.

0,1 g de cet intermédiaire est mis en solution dans 5 ml de dichlorométhane ; on ajoute 5 ml d'acide trifluoroacétique et laisse le mélange une demi-heure à température ambiante. On concentre sous vide pour obtenir le composé attendu.

m = 0,080 g

F > 260°C

En répétant l'un quelconque des modes opératoires décrits aux exemples 1 à 13 on a préparé les composés indiqués dans les tableaux 1 à 15 ci-après. Dans ces tableaux, R₃ lorsqu'il est utilisé, représente le groupe :

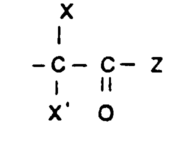

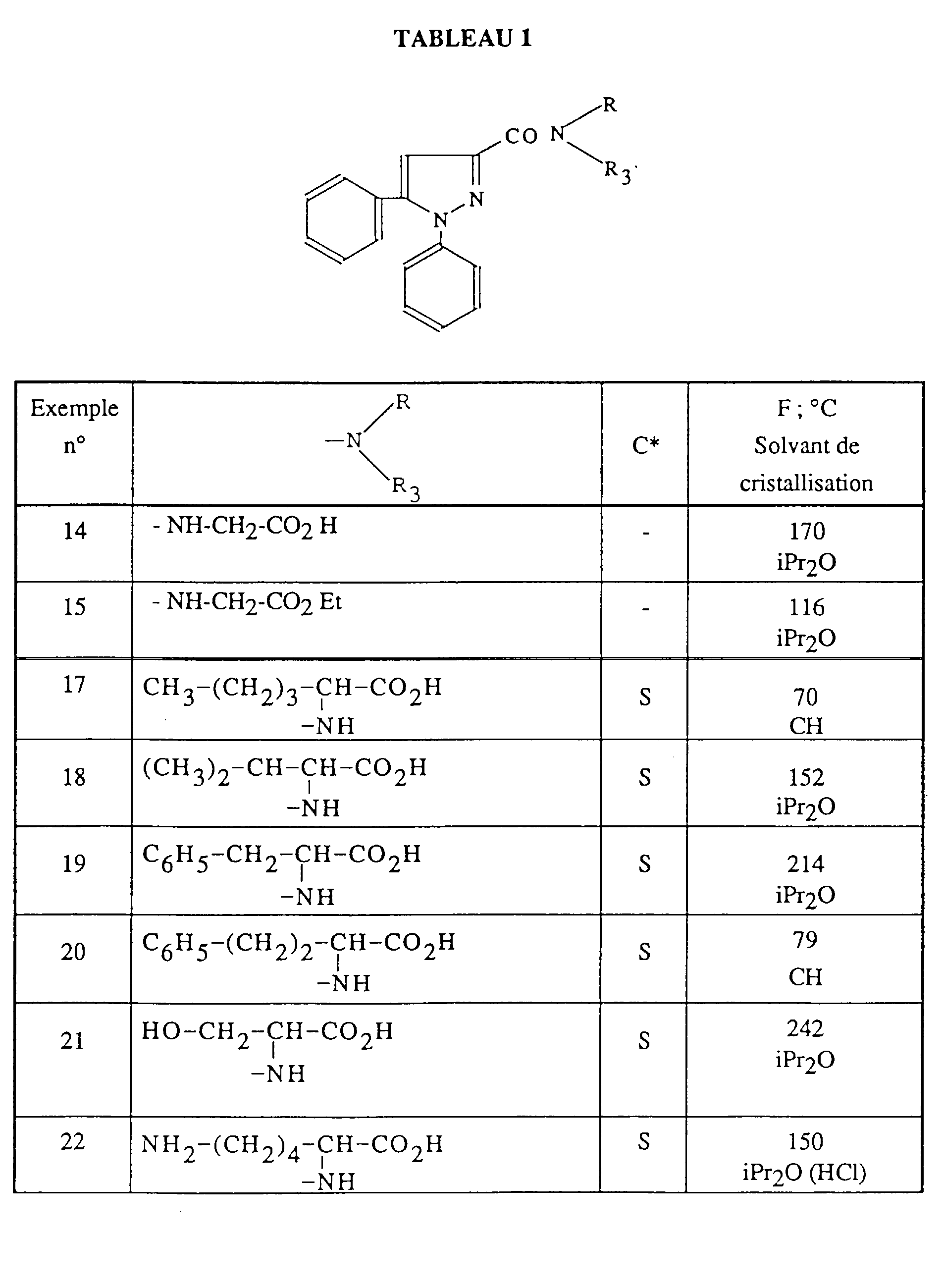

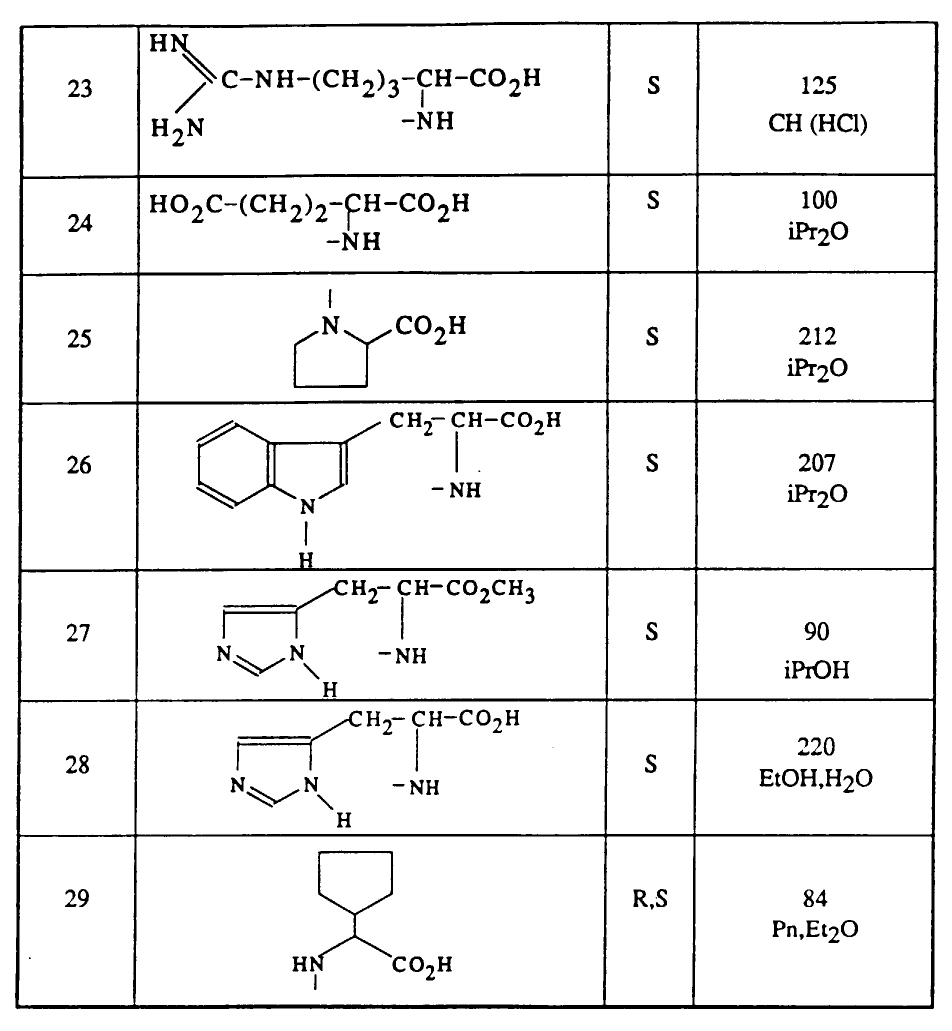

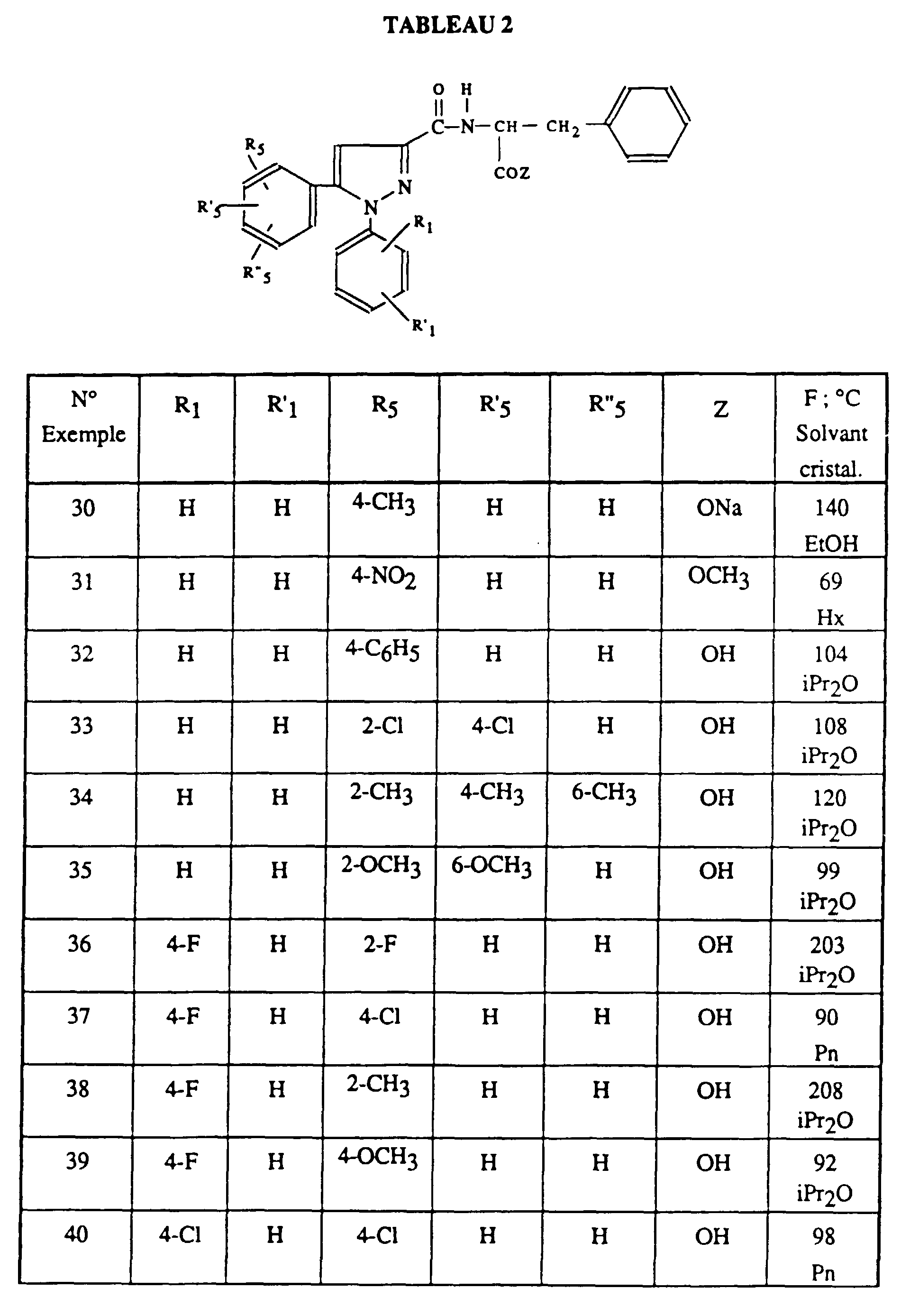

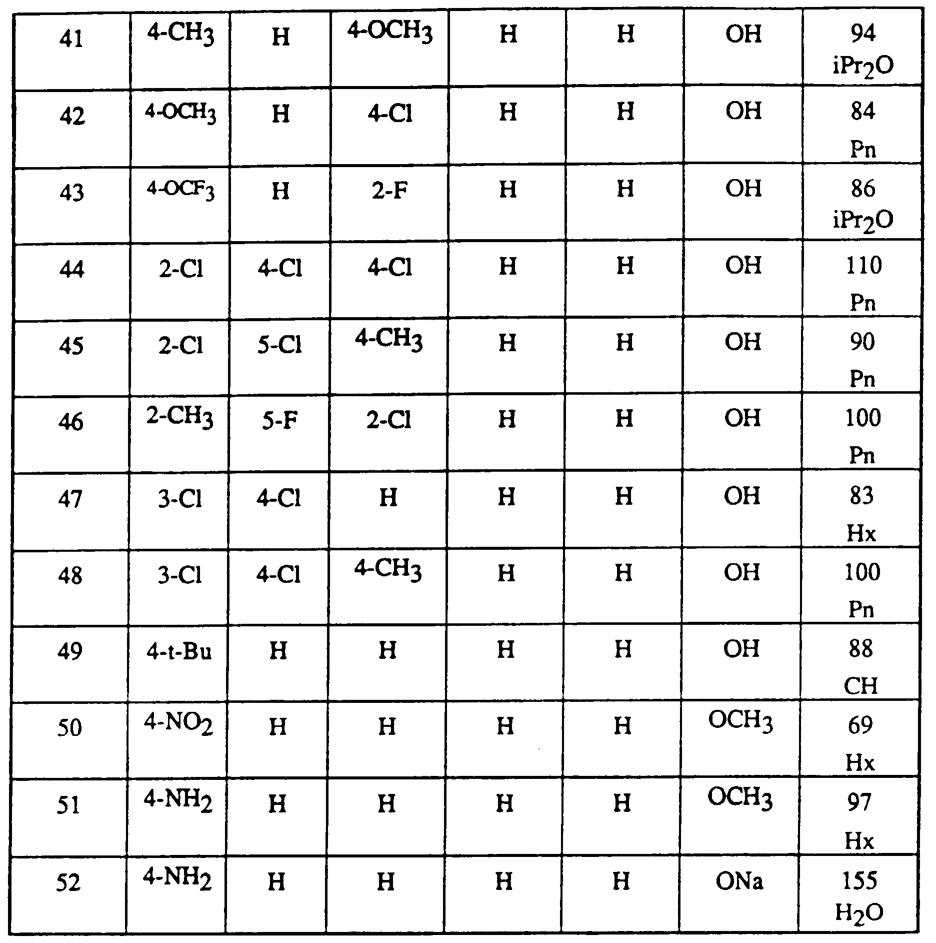

Les composés du tableau 2 sont tous de configuration (S).

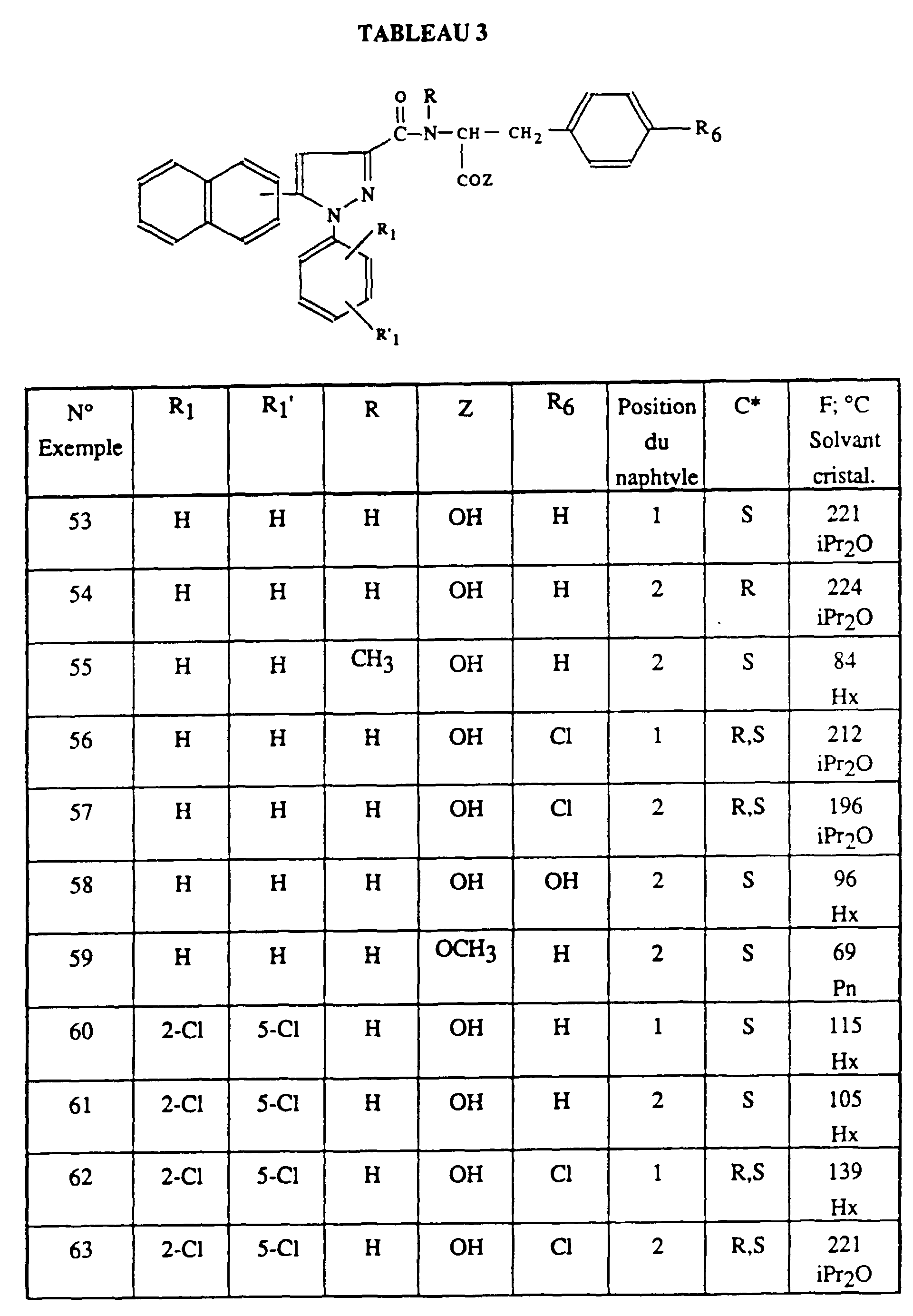

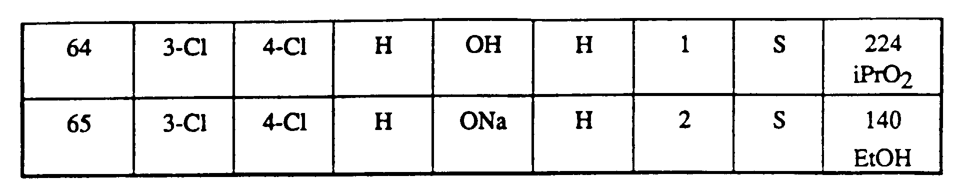

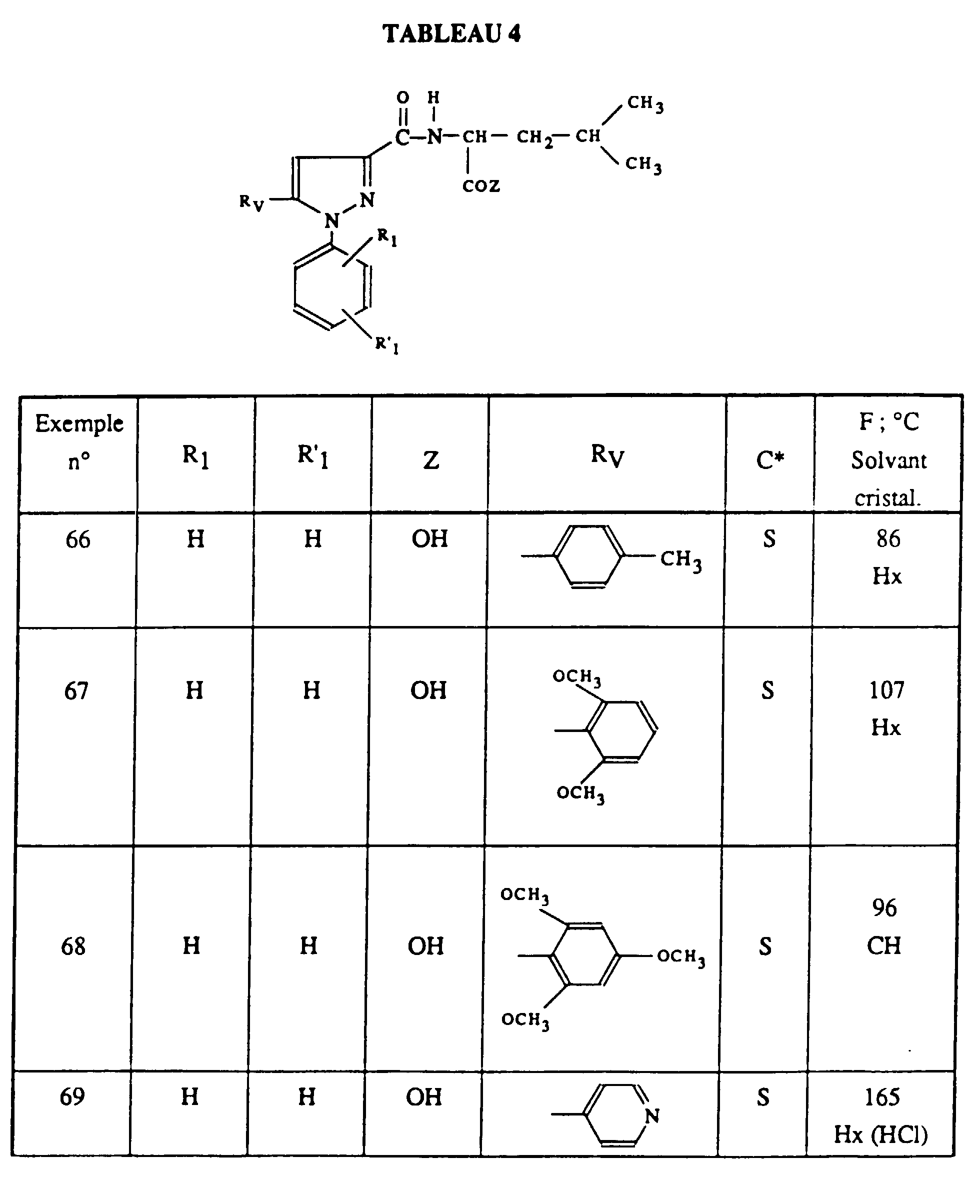

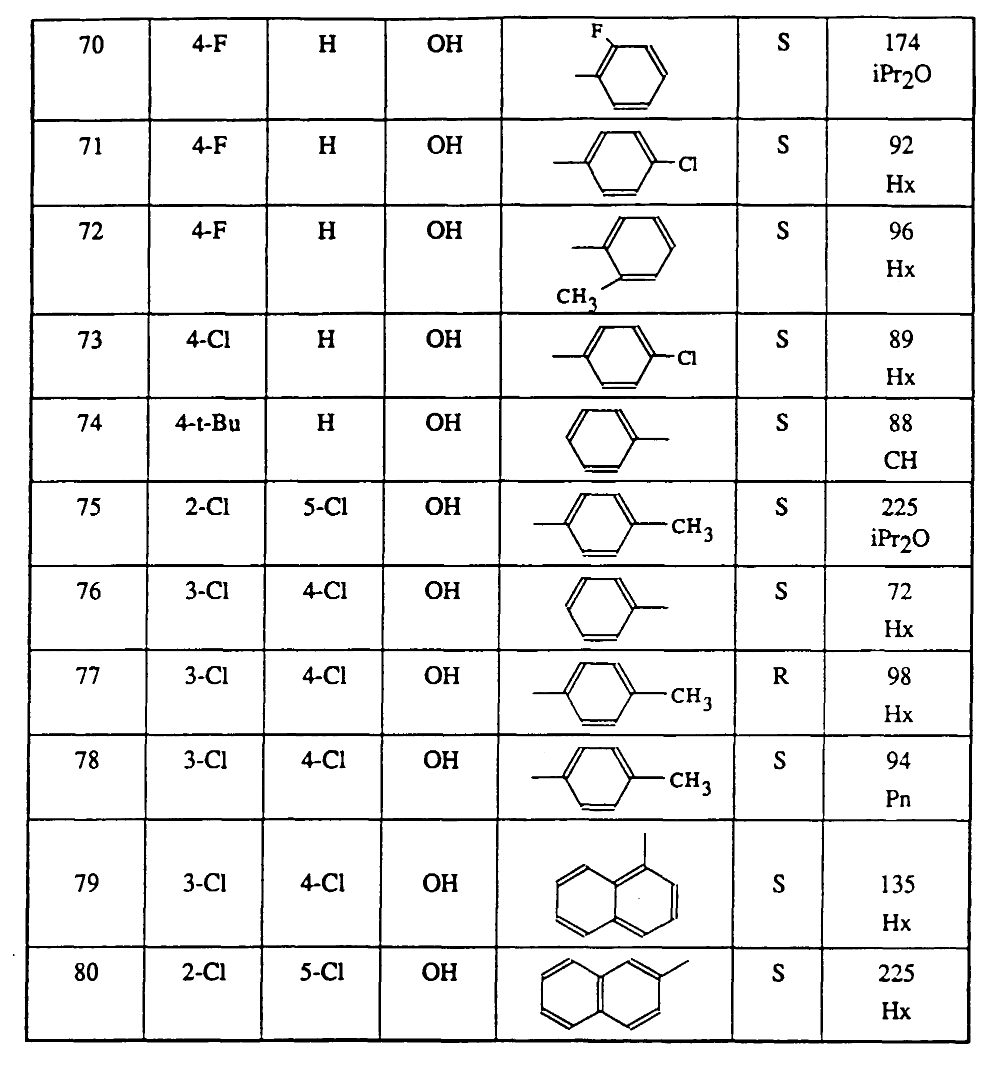

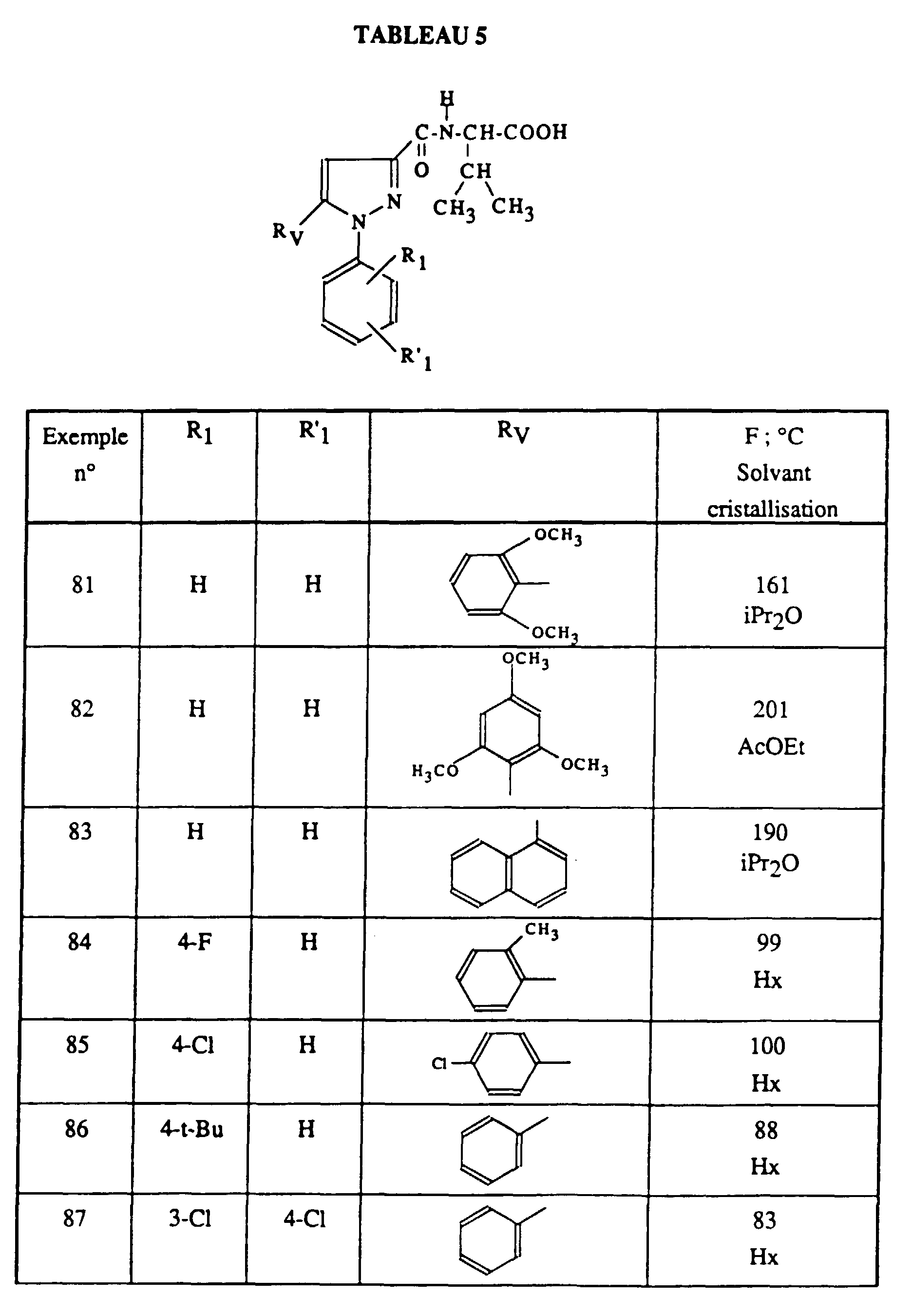

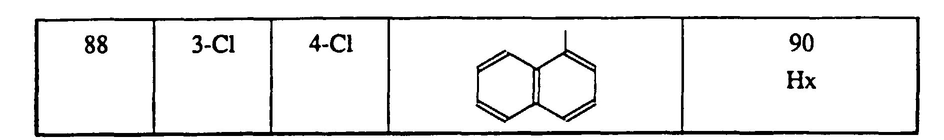

Les composés de 81 à 88 sont de configuration S.

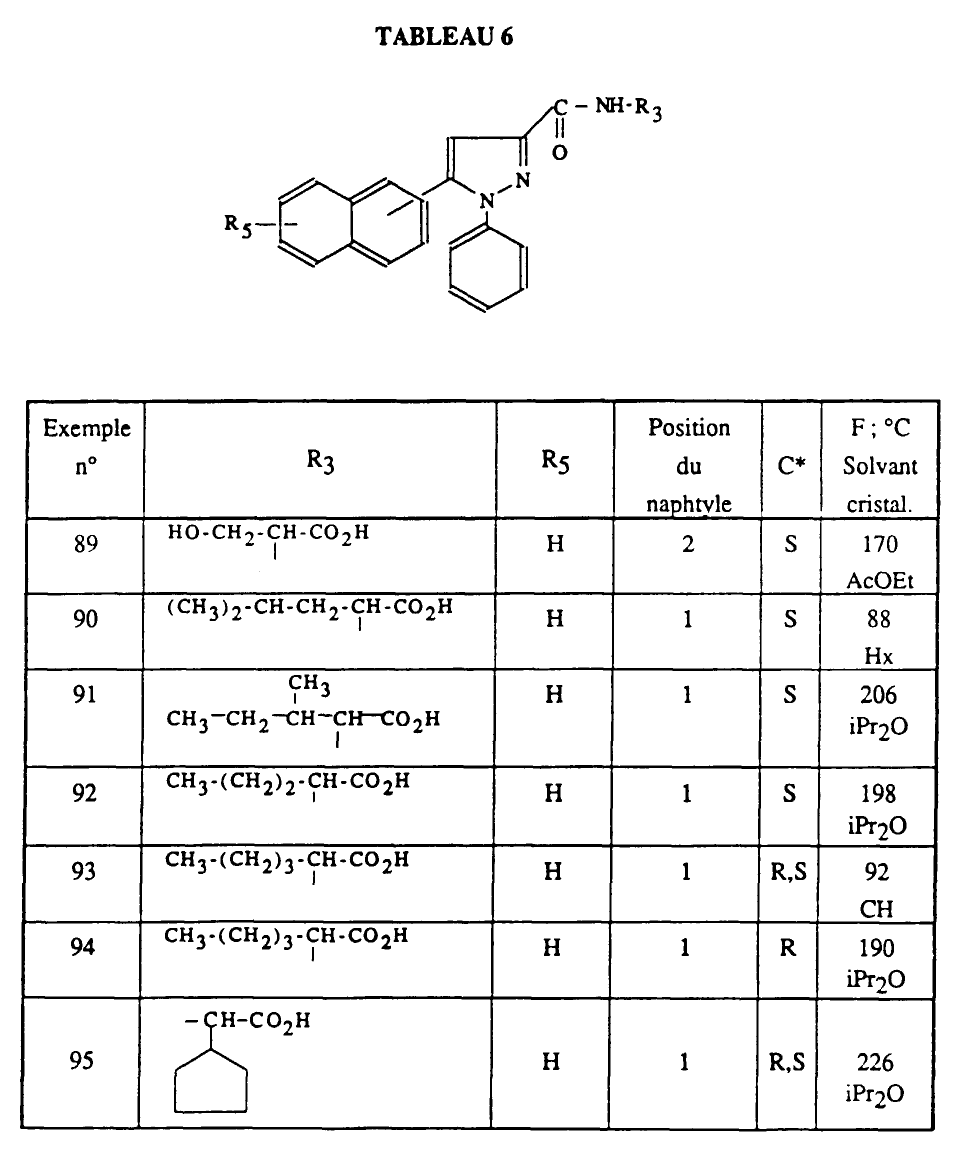

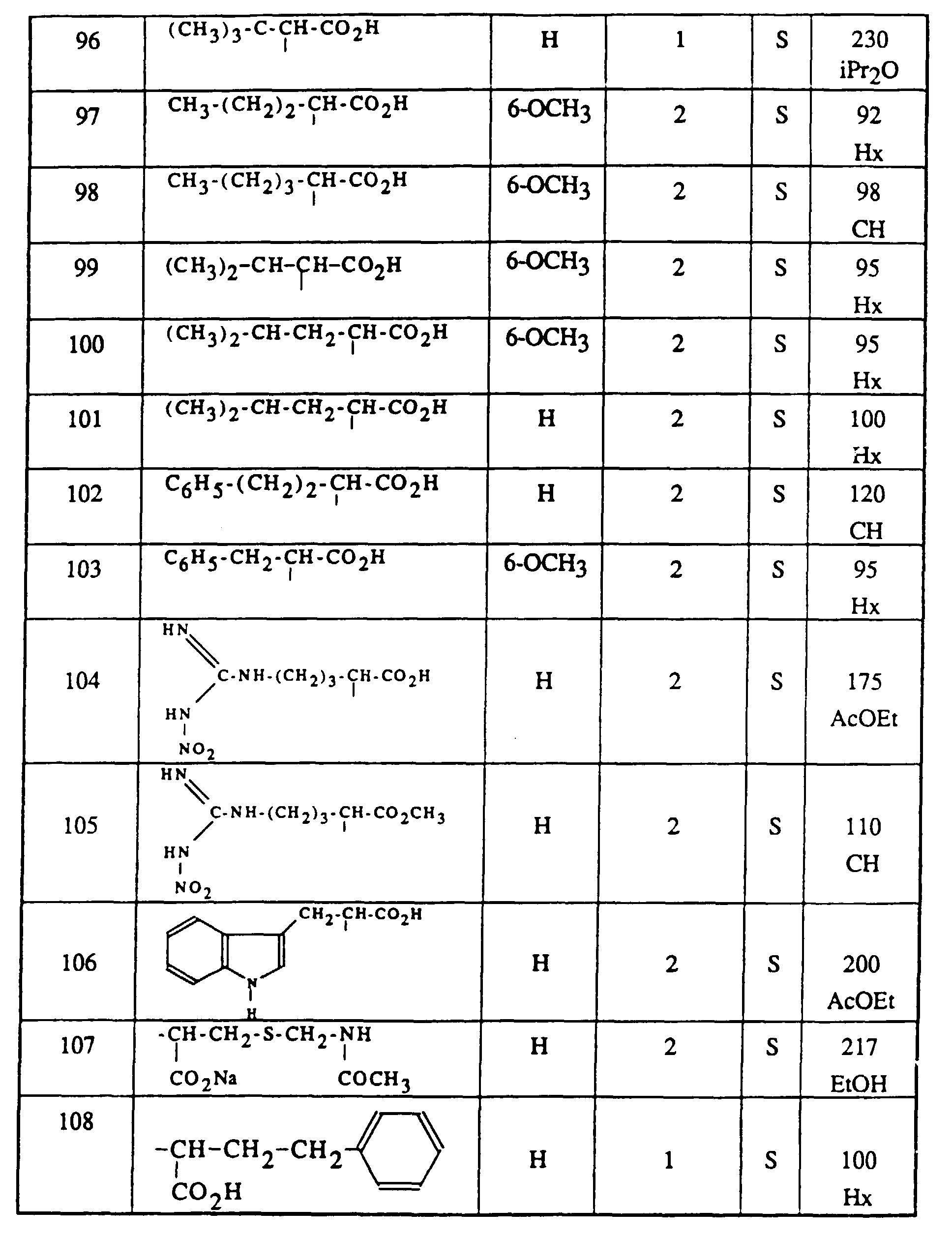

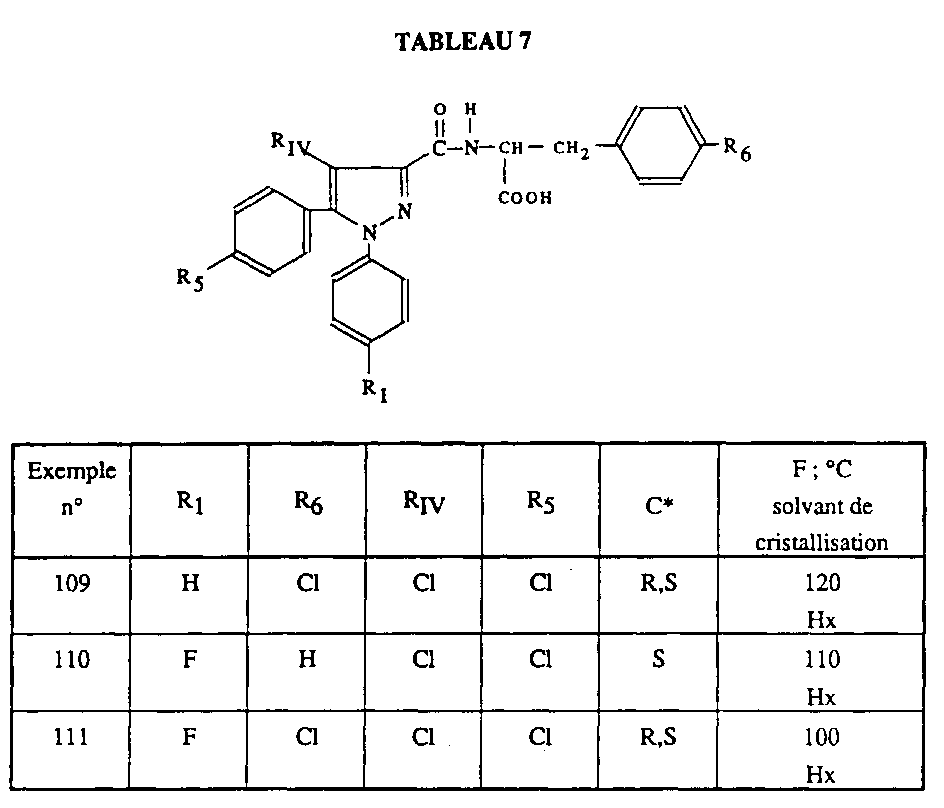

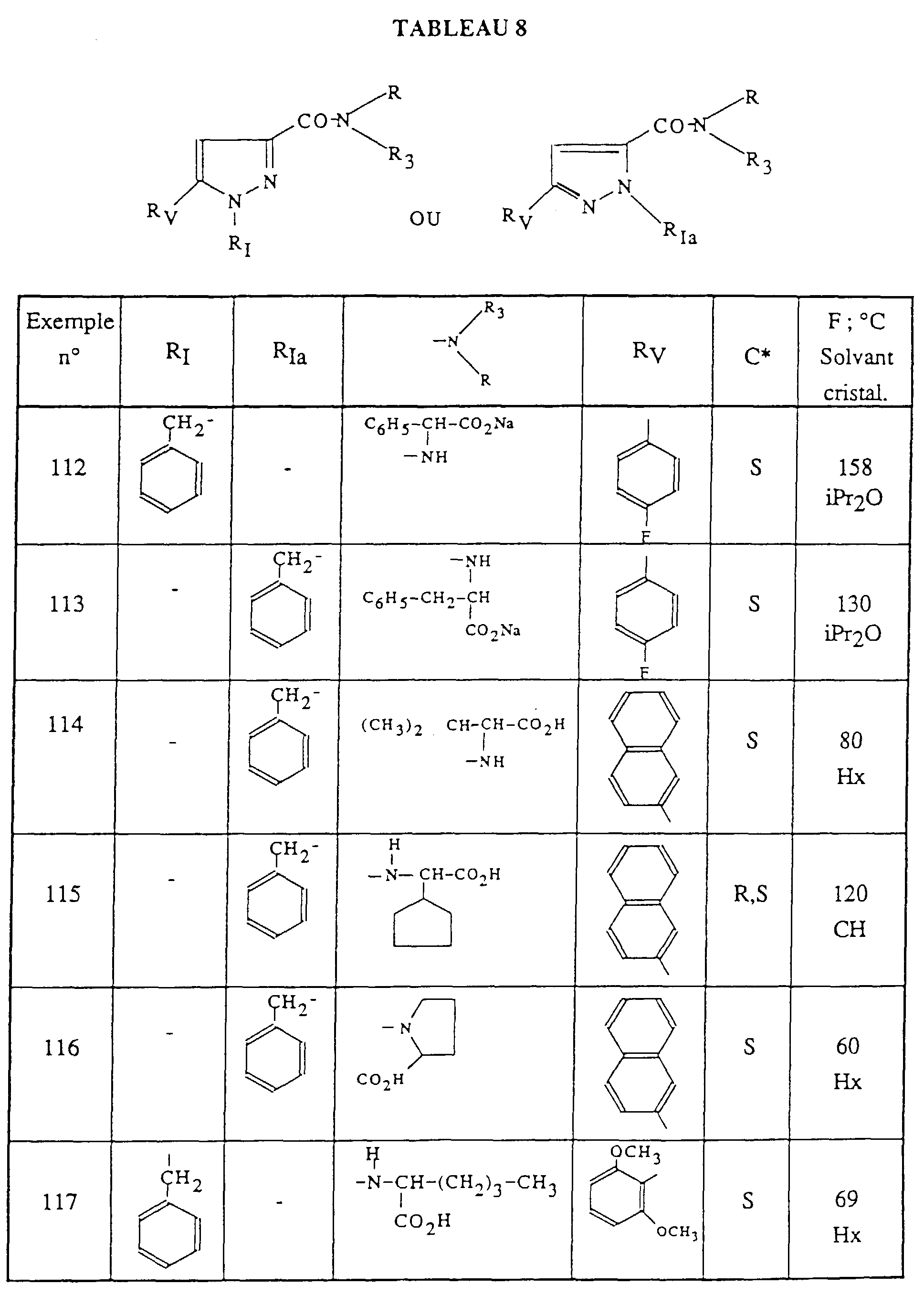

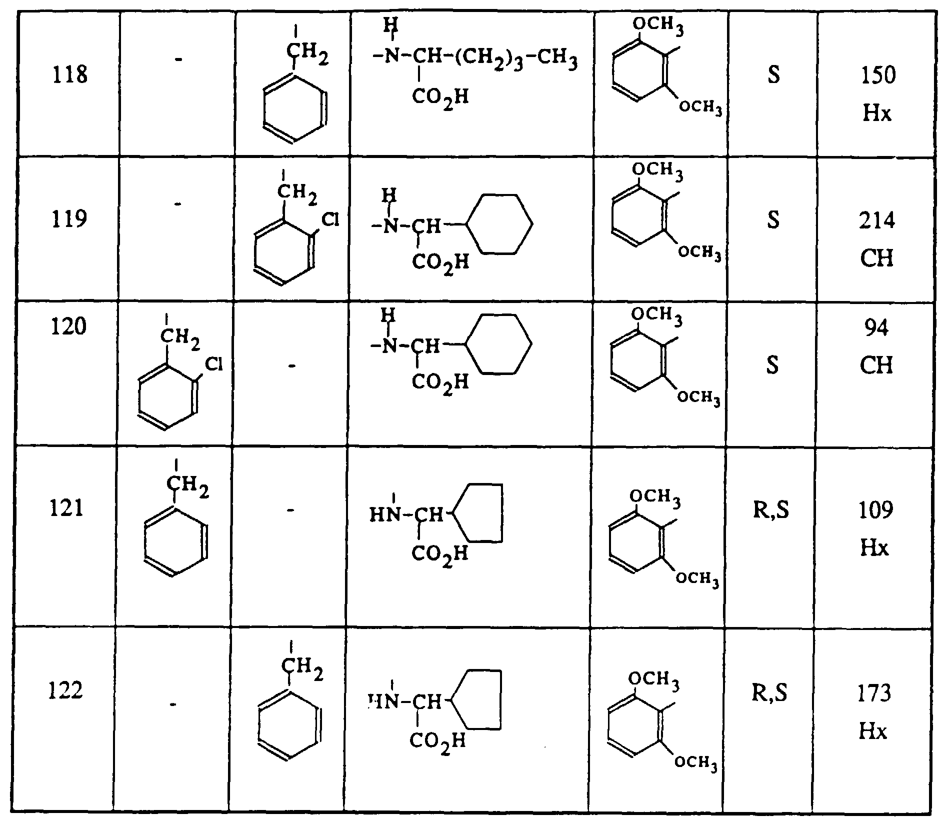

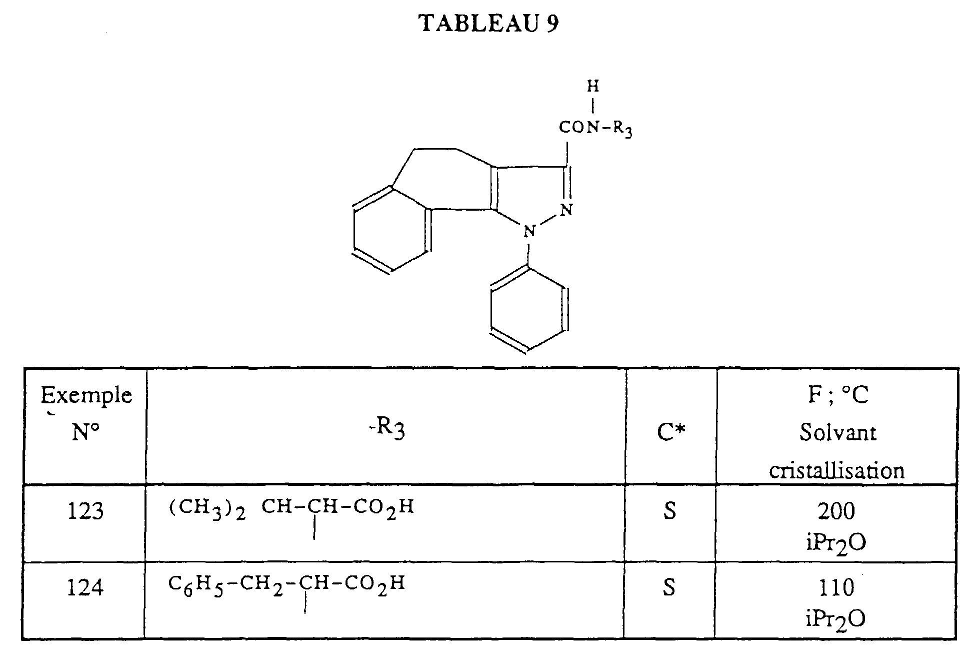

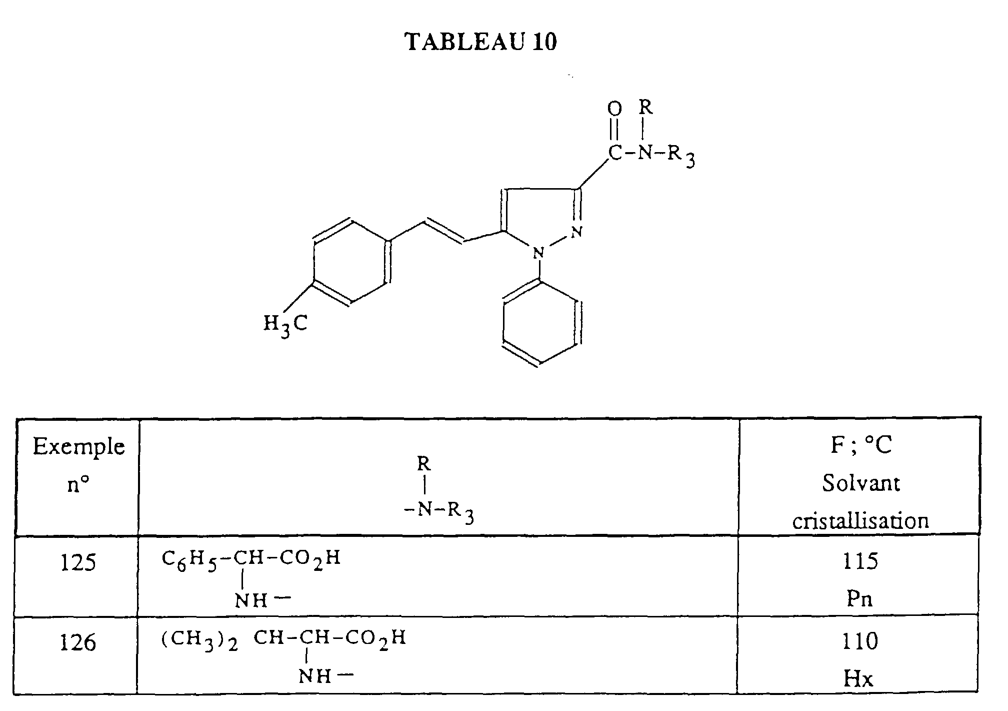

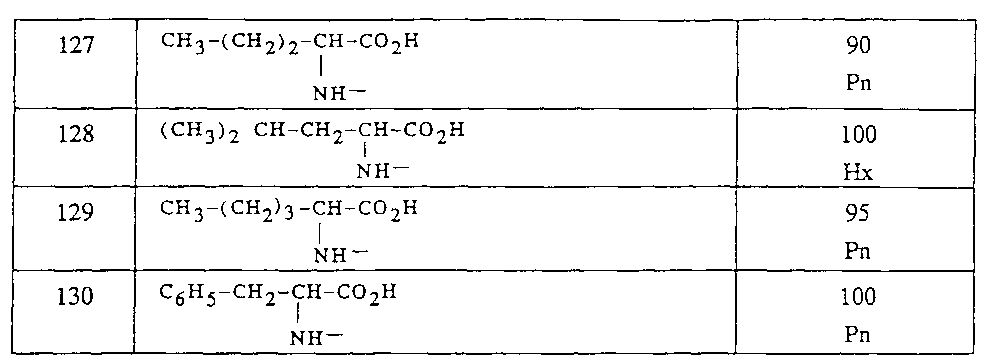

Les composés 125 à 130 sont de configuration S.

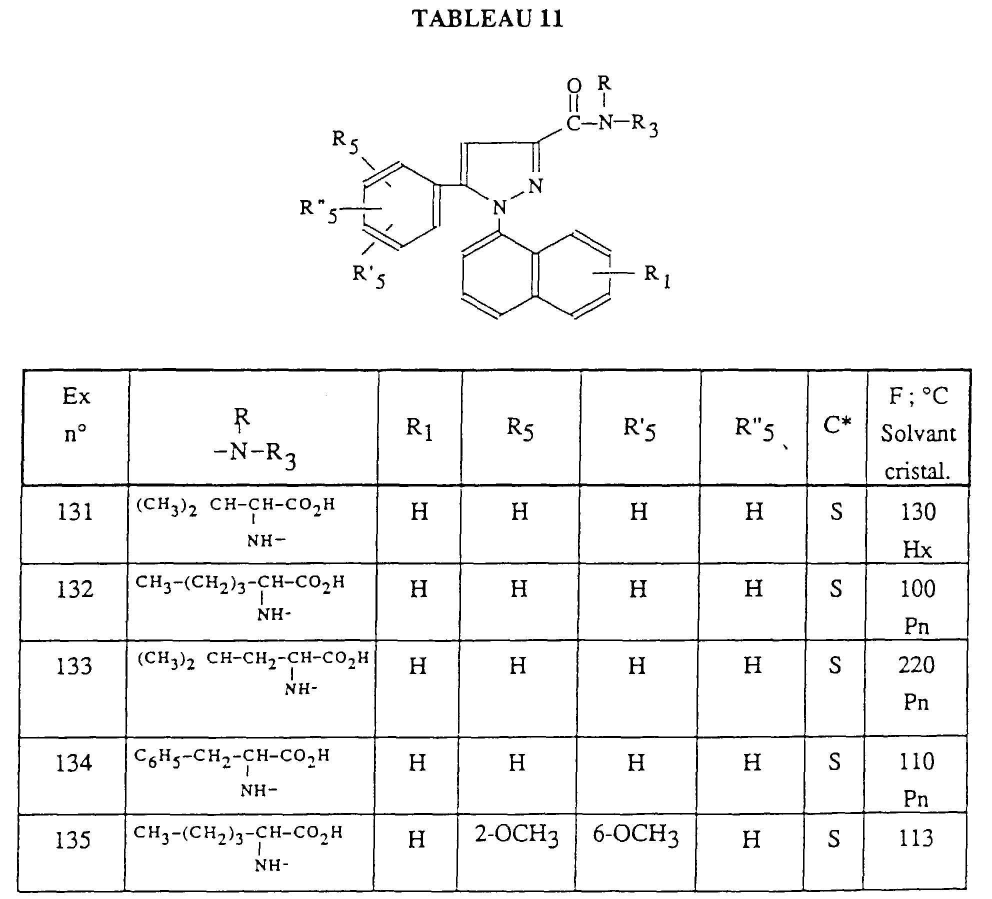

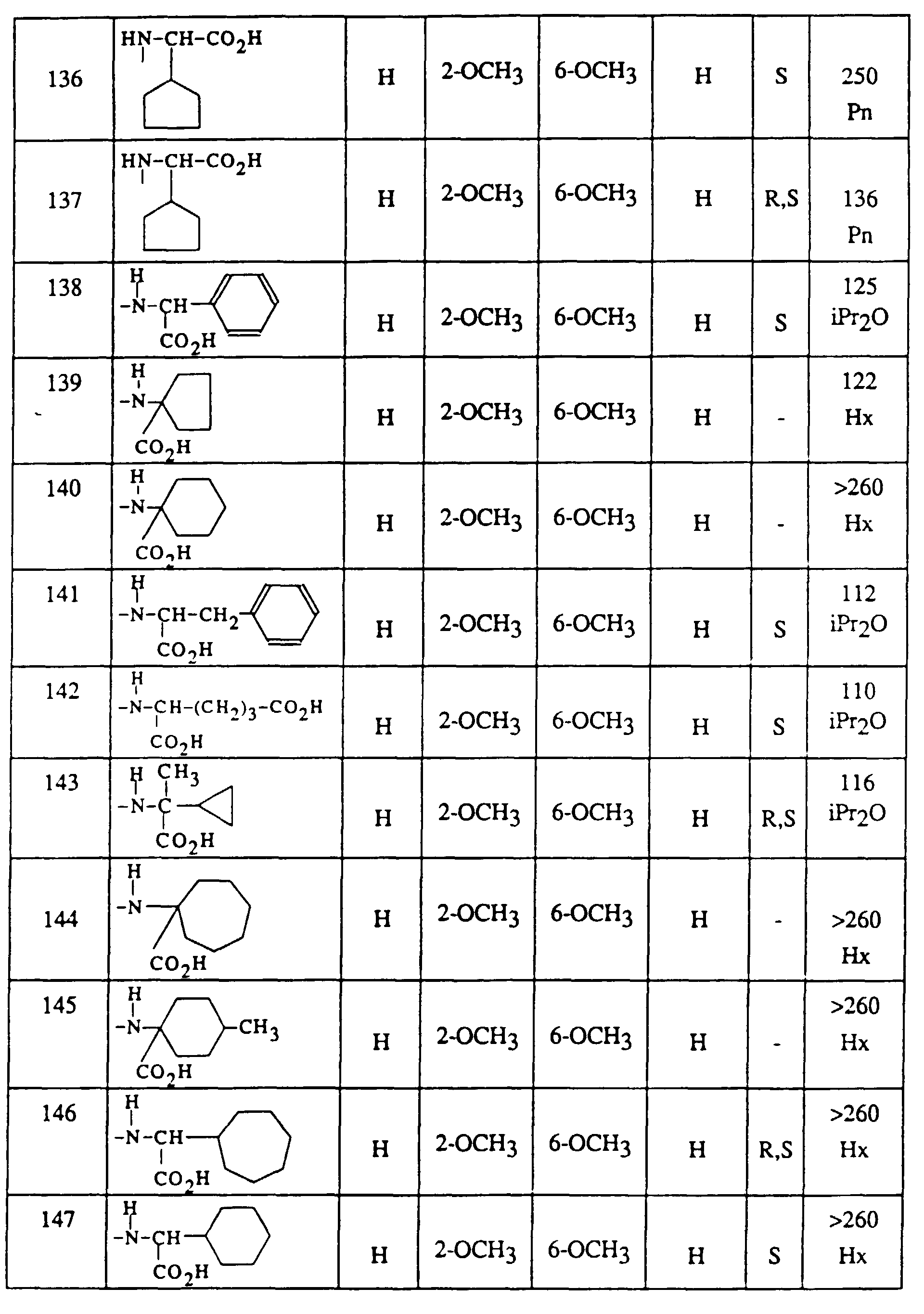

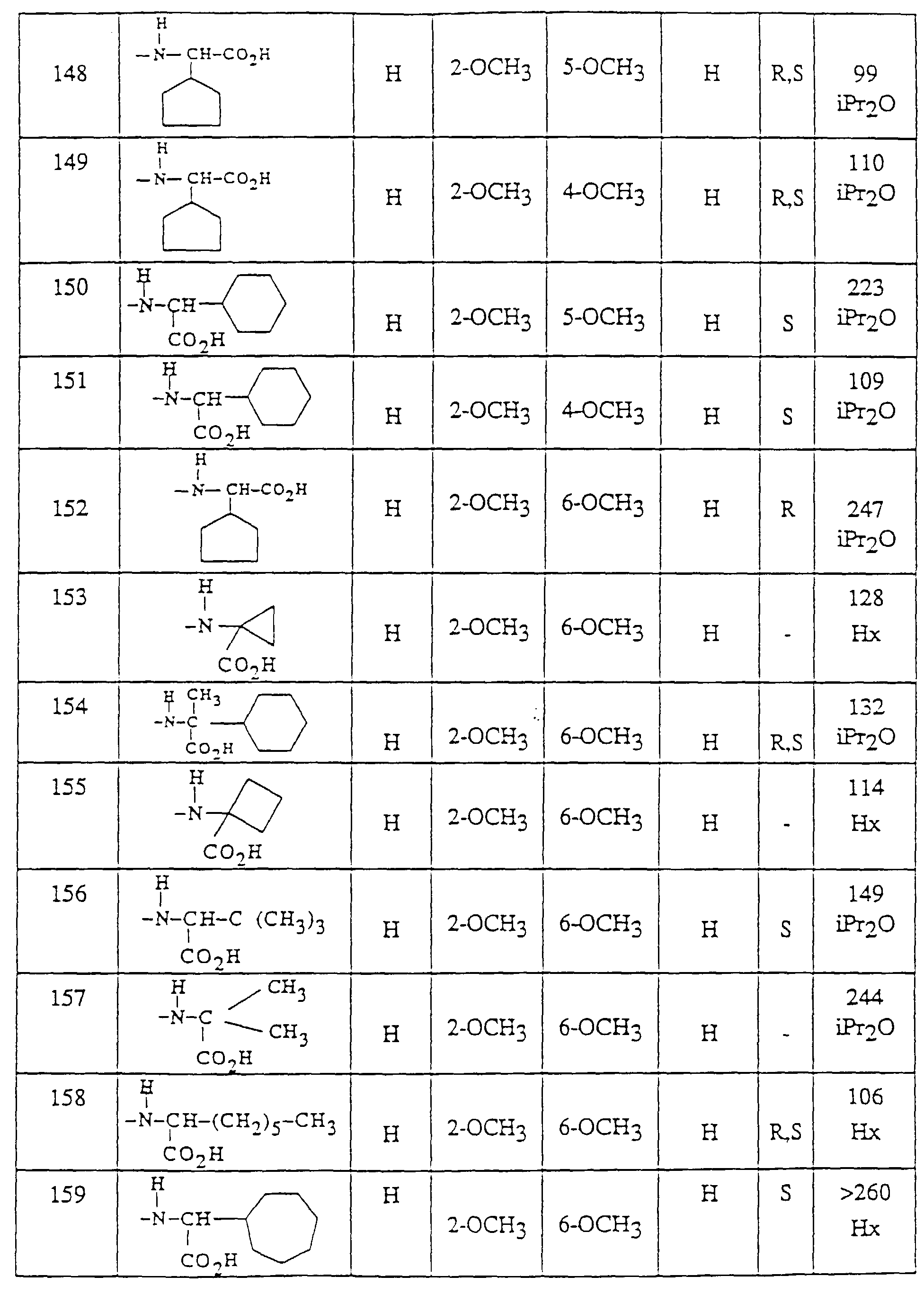

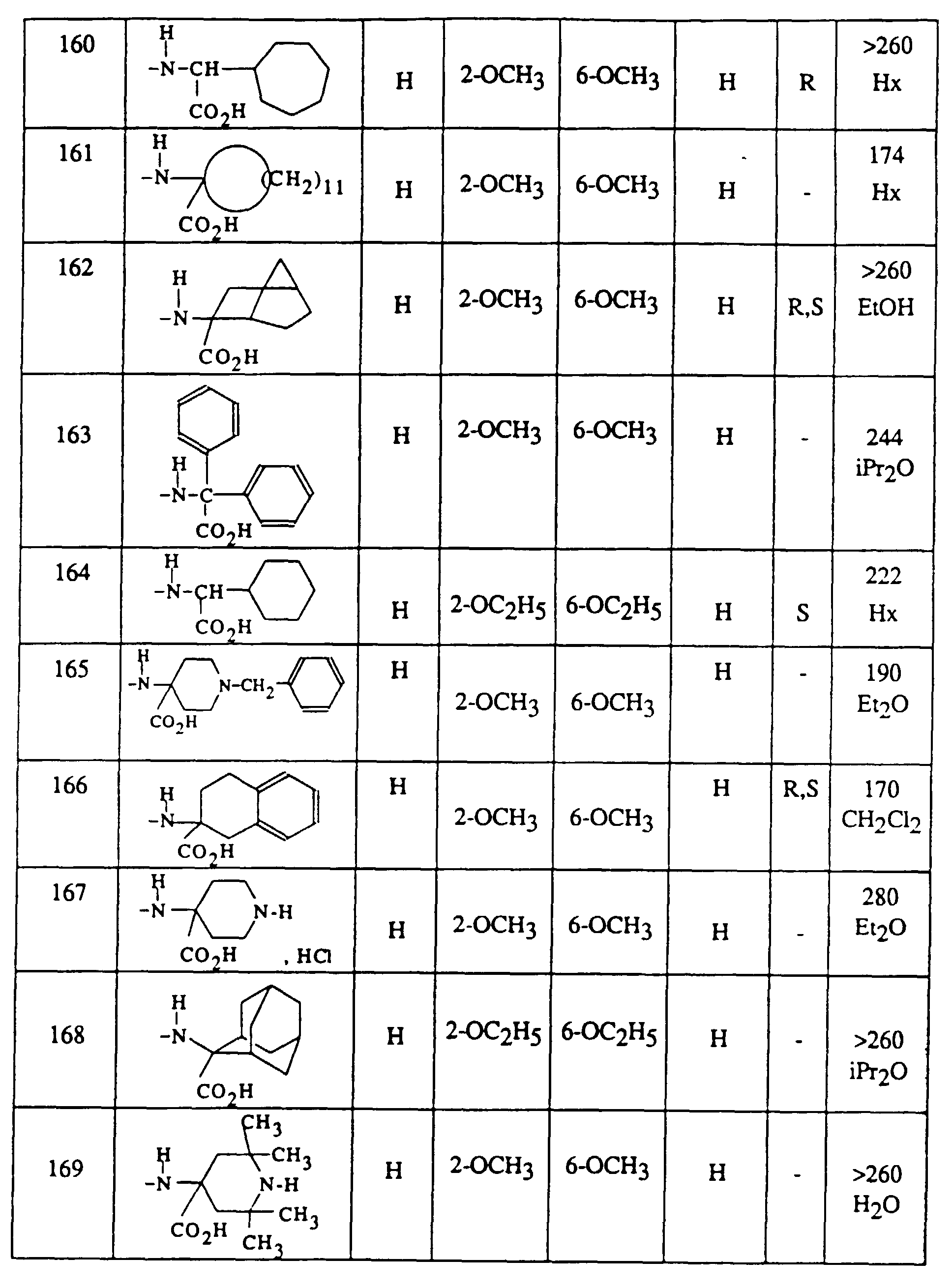

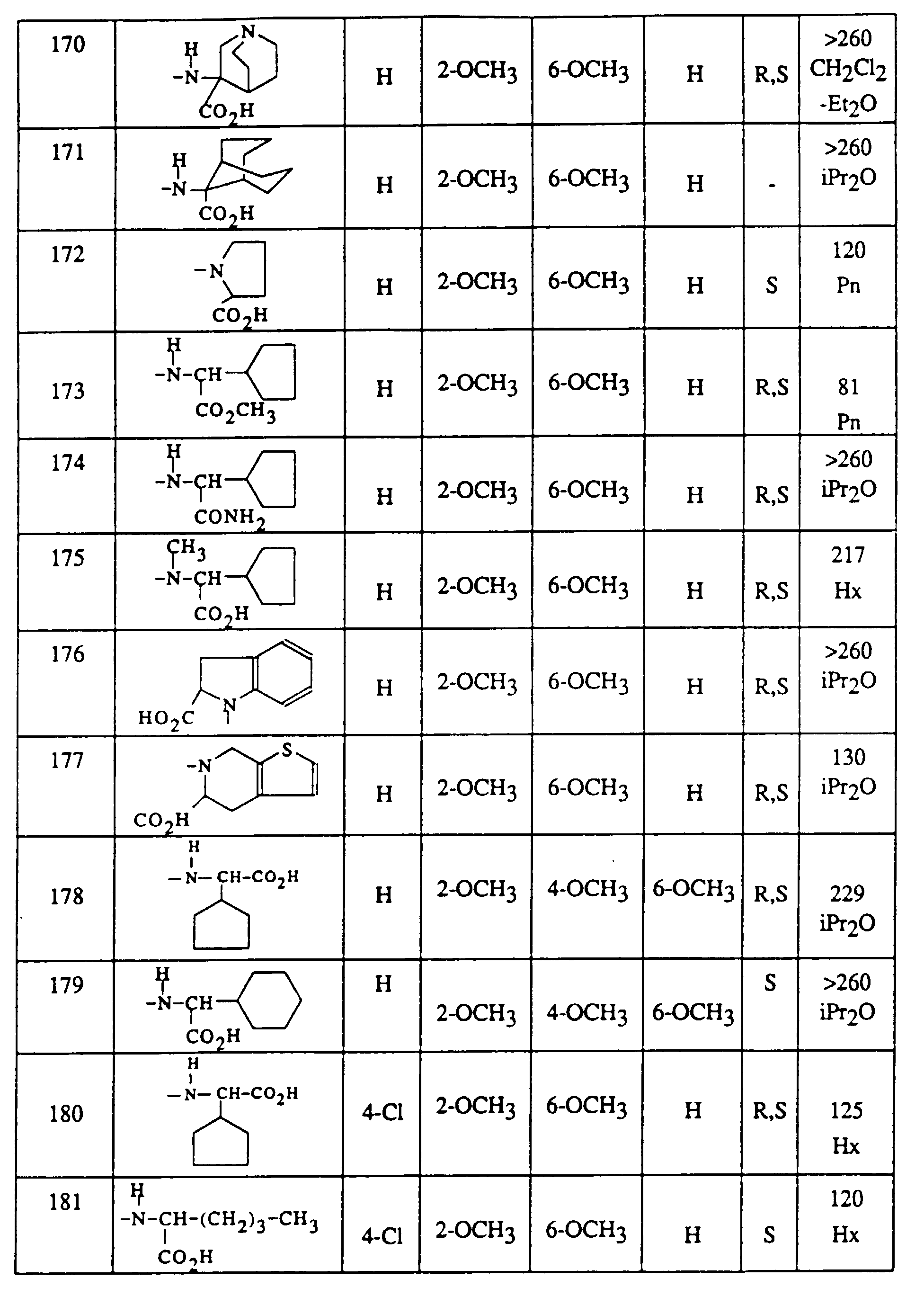

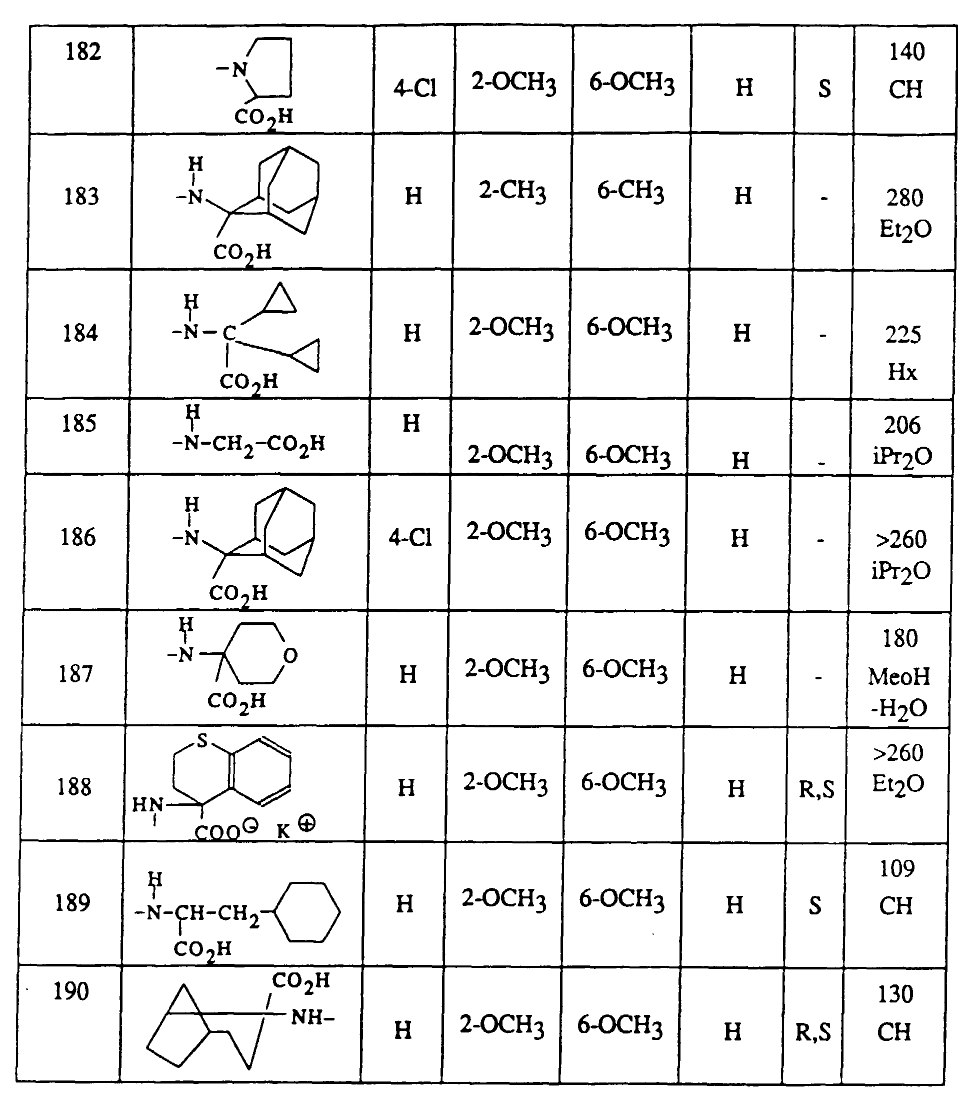

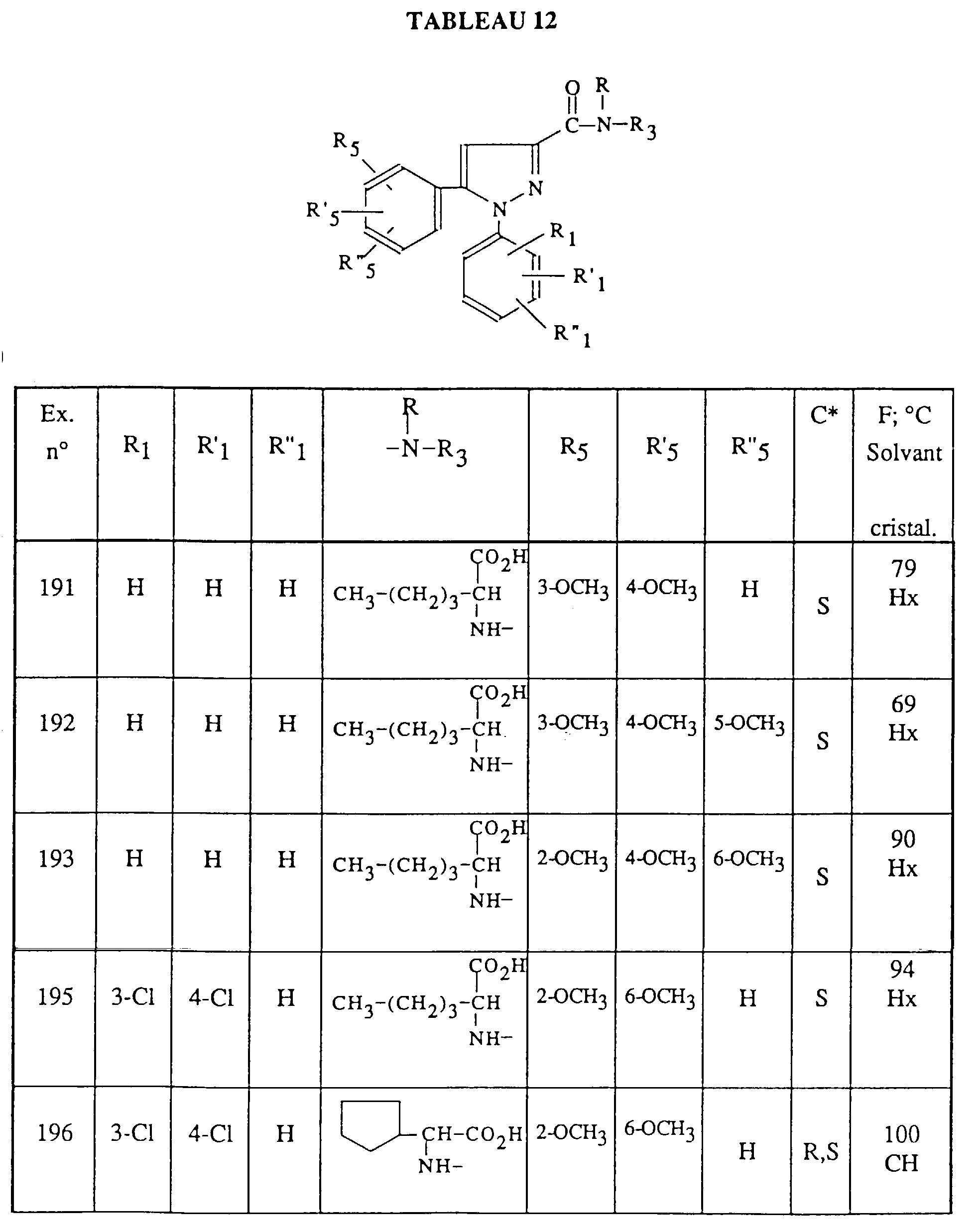

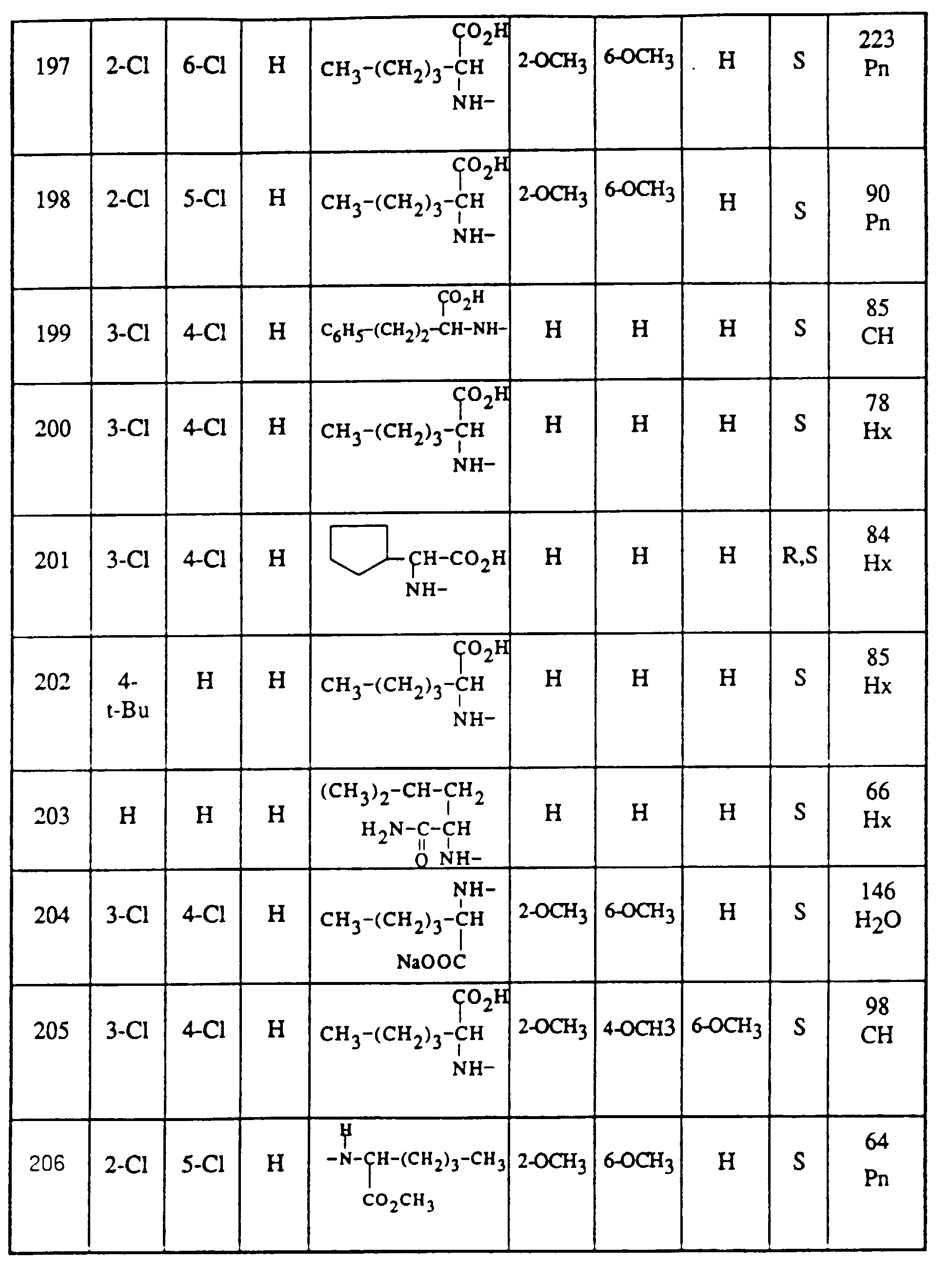

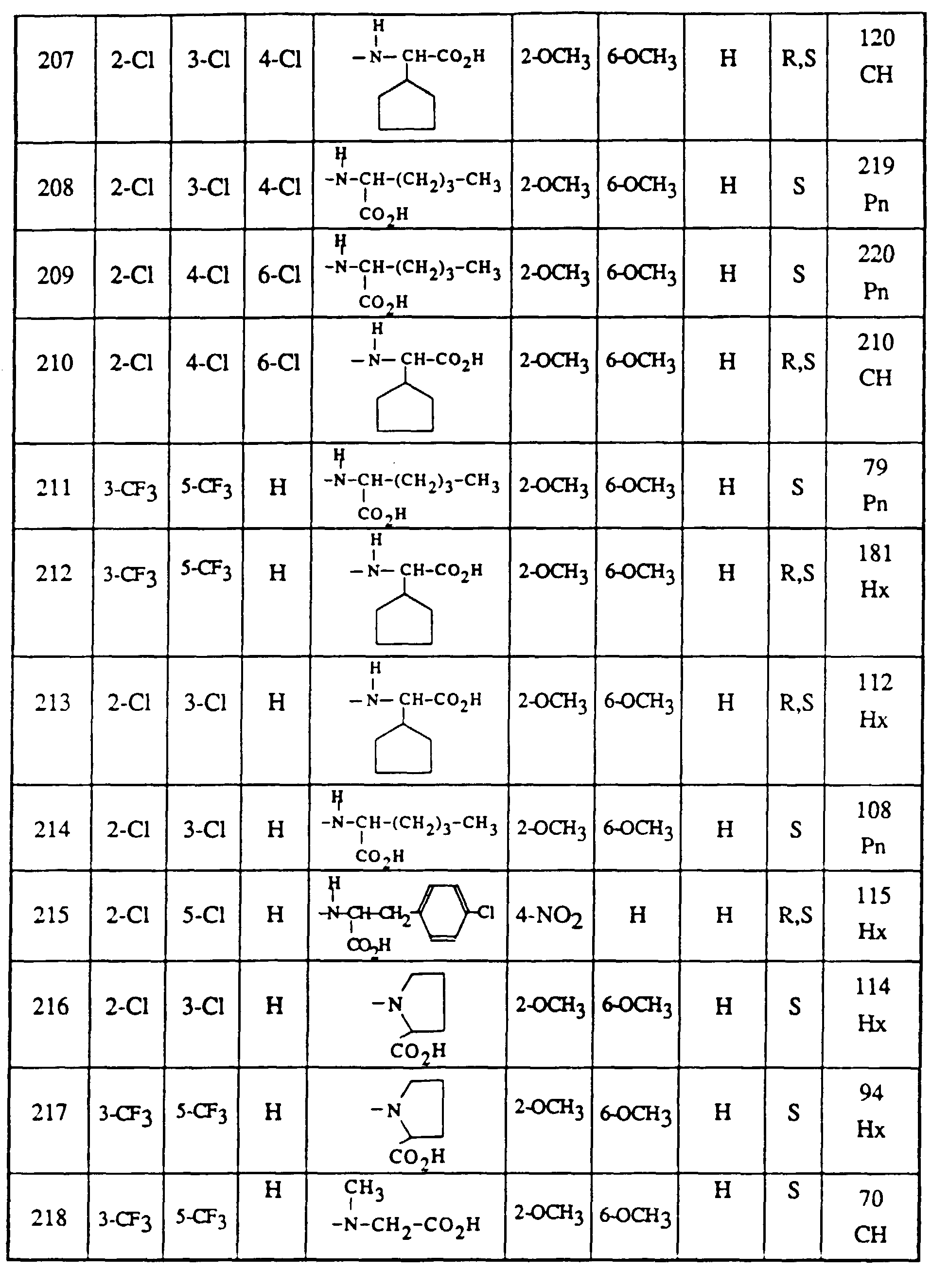

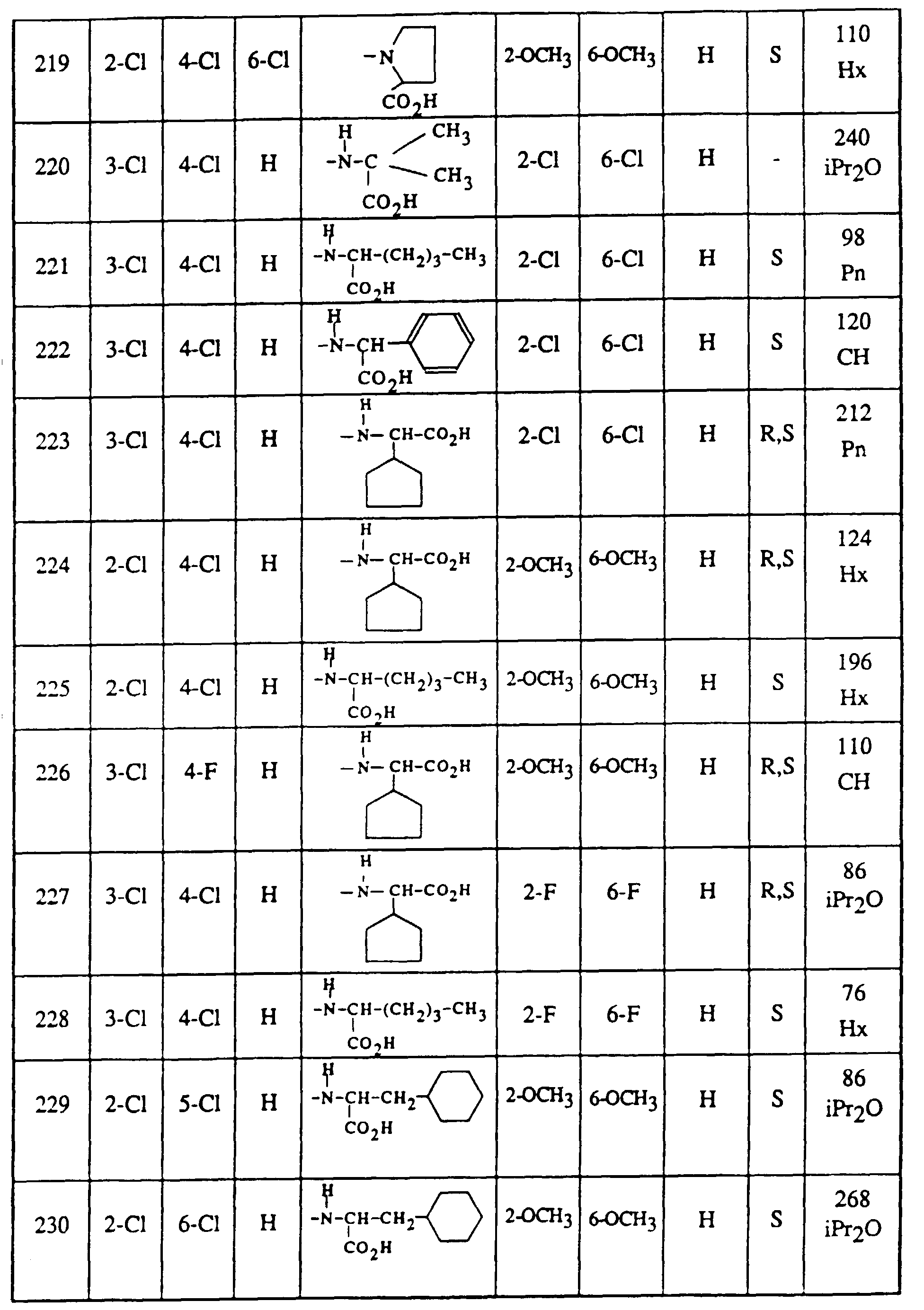

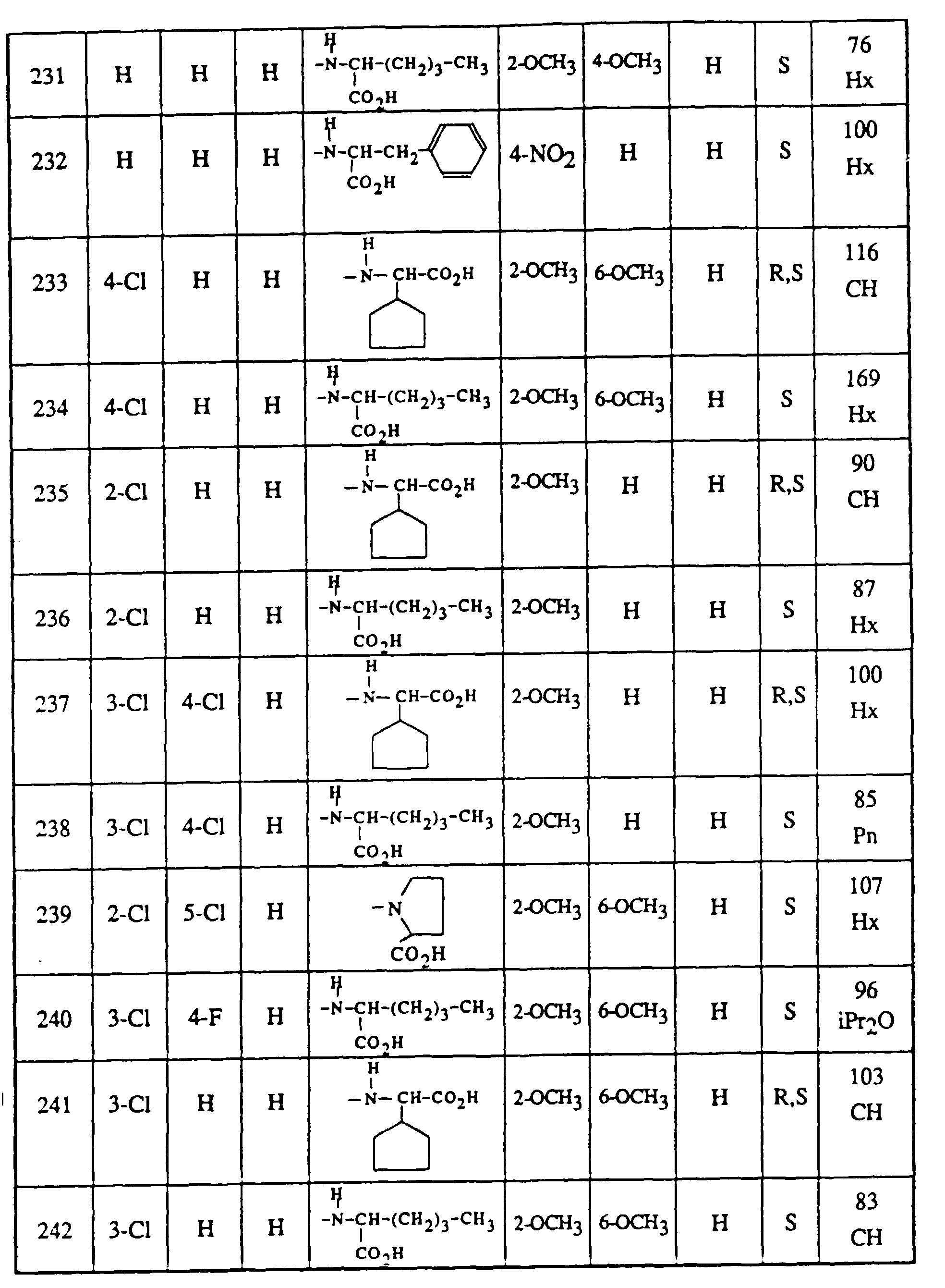

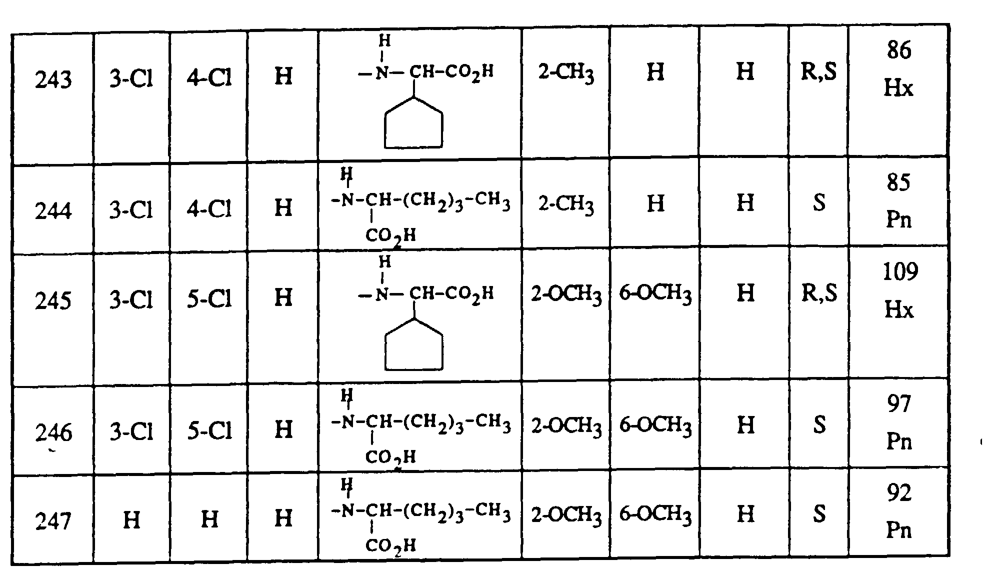

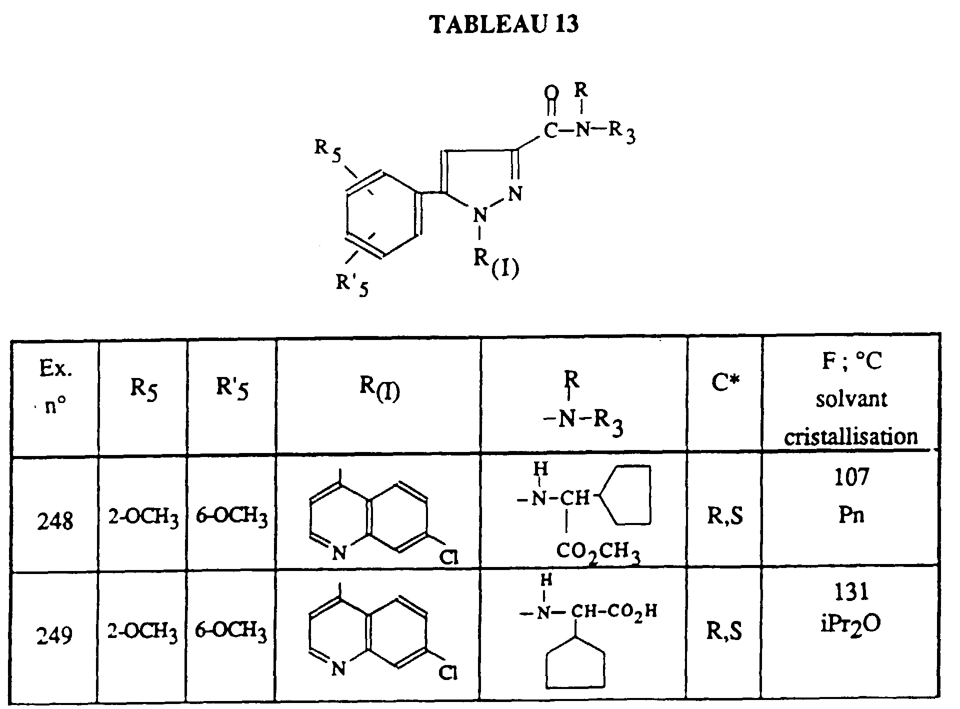

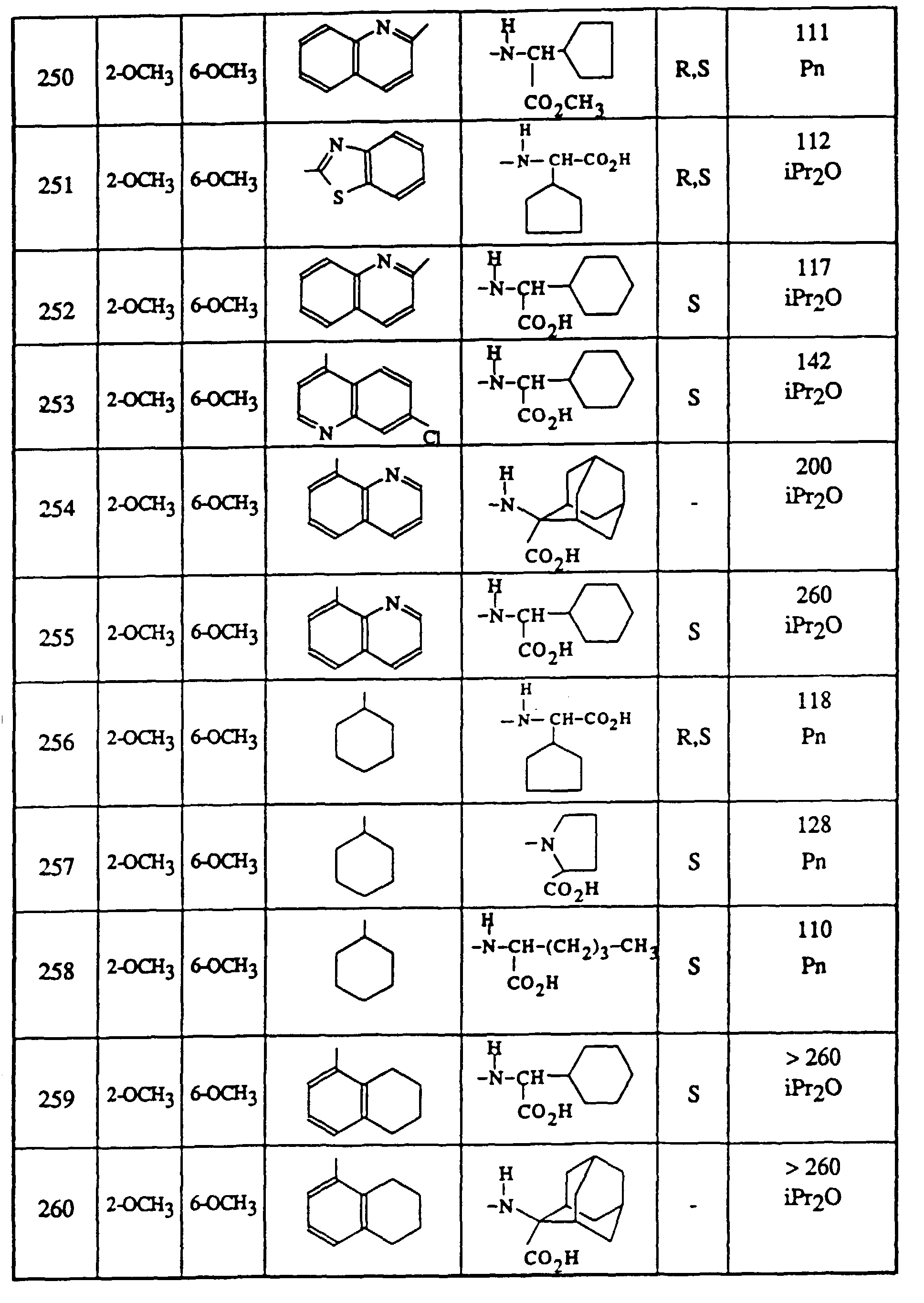

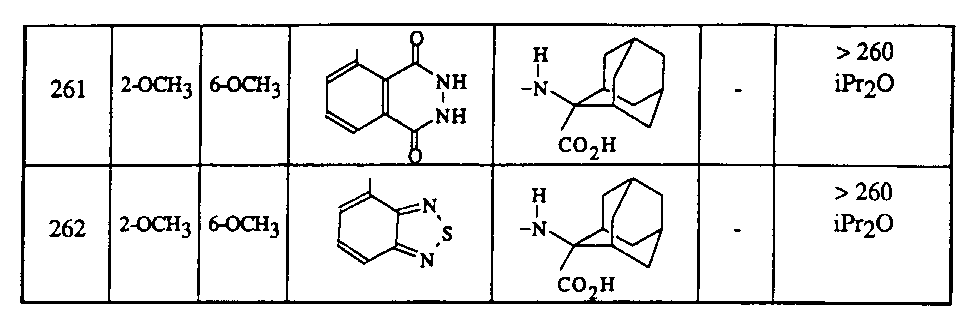

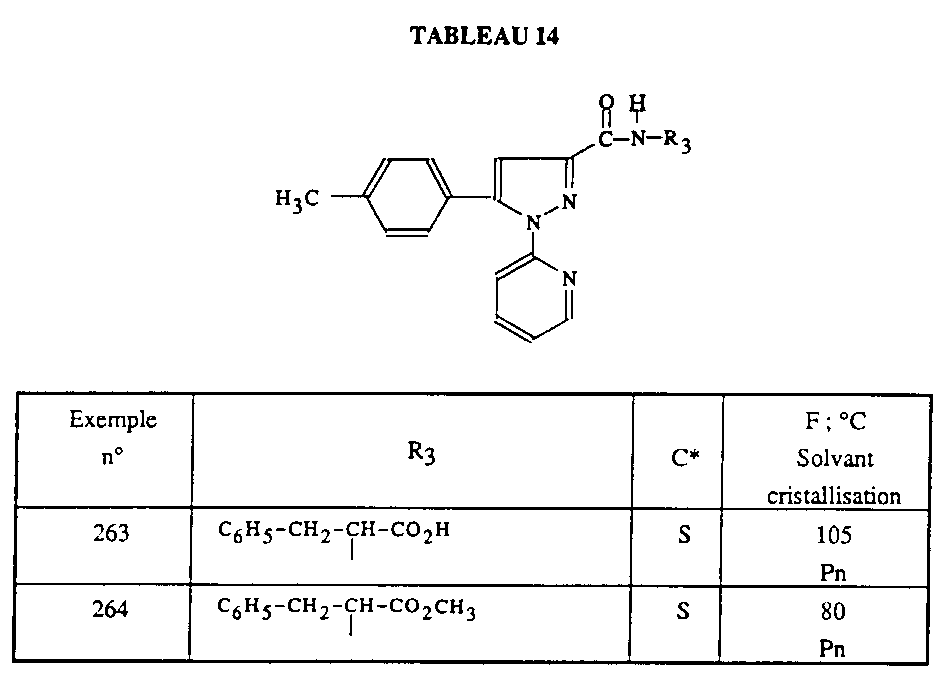

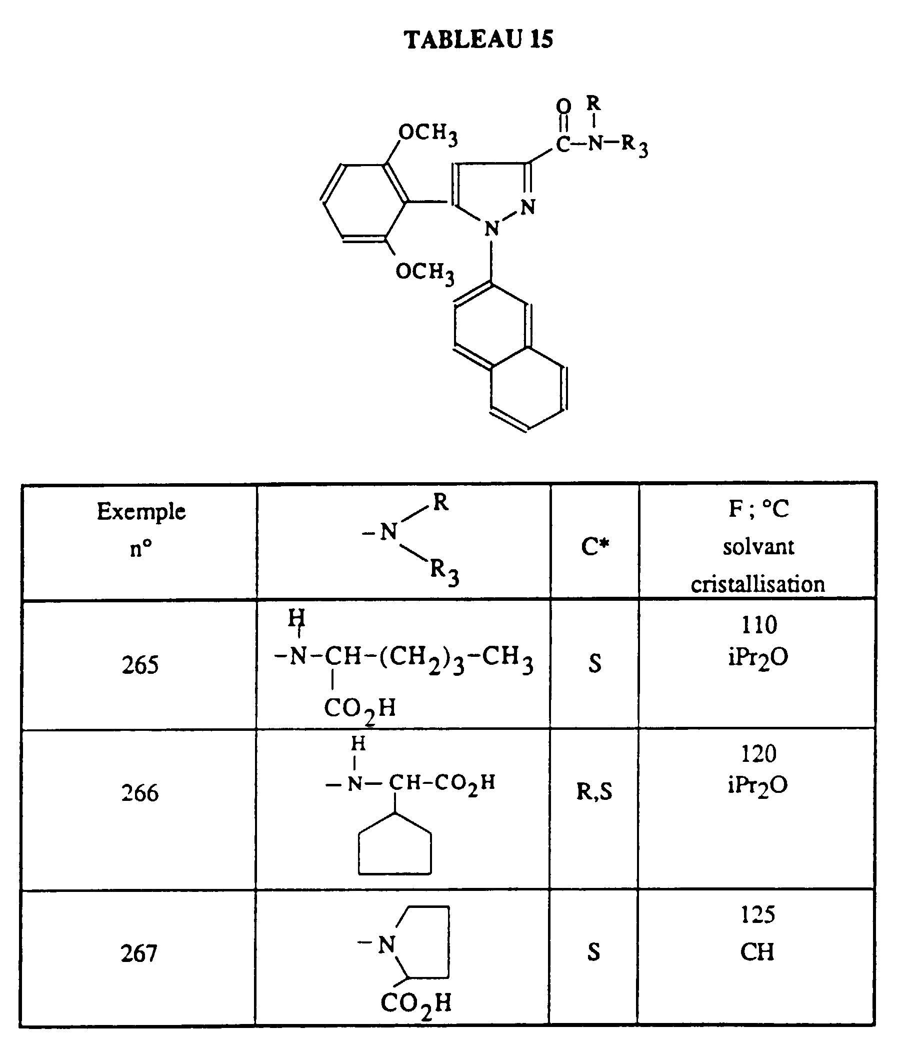

## Revendications (Revendications pour l'(les) Etat(s) contractant(s) suivant(s): AT, BE, CH, LI, DE, DK, FR, GB, IT, LU, NL, SE)

1. Amido-3 pyrazole de formule (I) ou (I'): 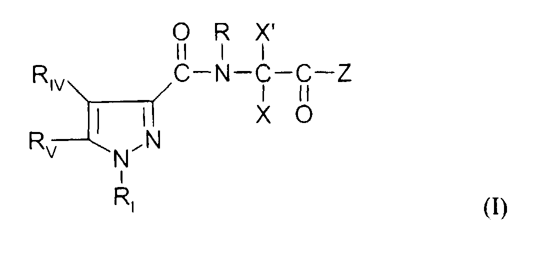 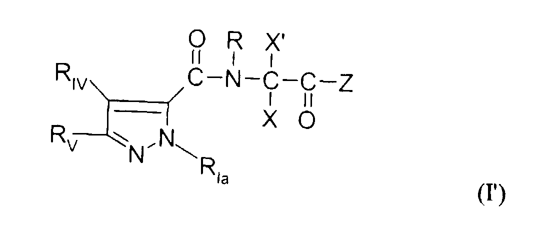 dans laquelle
- R_{I} représente
. un groupe 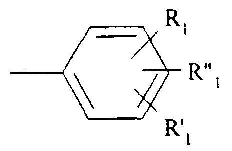 où R₁, R'₁ et R"₁ représentent chacun indépendamment un atome d'hydrogène. un atome d'halogène, un hydroxyle, un groupe alkyle droit ou ramifié en C₁-C₄, un groupe alcoxy en C₁-C₄, un groupe trifluorométhyle, un groupe trifluorométhoxy, un groupe nitro, un groupe carboxy, un groupe amino ;
. un groupe carboxyalkyle ou alcoxycarbonylalkyle dans lequel les alkyles sont en C₁-C₄ ;
. un groupe cycloalkyle dans lequel les alkyles sont en C₃-C₆ ;
. un groupe tétrahydronaphtyle ;
. un groupe pyridyle ;
. un naphtyle substitué par R₁, R'₁ et R"₁ tels que définis précédemment ;
. un groupe benzyle substitué par R₁, R'₁ et R"₁ tels que définis précédemment ;
. un groupe cinnamyle éventuellement substitué sur le noyau aromatique par un halogène, un hydroxyle, un alcoxy en C₁-C₄ ;
. un groupe quinolyle ou isoquinolyle, éventuellement substitué par R₁, R'₁ et R"₁ tels que définis ci-dessus ;
. un groupe benzothiazolyle-2 ;
. un groupe quinoxalinyldione ;
. un groupe phtalazinyl-1 ;
. un groupe benzothiadiazolyle ;
. un groupe méthylène substitué par un groupement hétérocyclique choisi parmi les groupes pyridyle et thiényle ;
- R_{Ia} représente un groupe benzyle substitué par R₁, R'₁ et R"₁ tels que définis ci-dessus ;
- R_{IV} représente un atome d'hydrogène, un atome d'halogène, un alkyle en C₁-C₆ ;
- R_{V} représente :
. un groupe 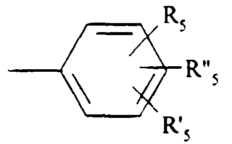
- où R₅, R'₅ et R"₅ représentent chacun indépendamment un atome d'hydrogène, un atome d'halogène, un alkyle droit ou ramifié en C₁-C₄, un hydroxyle, un alcoxy en C₁-C₄, un nitro, un trifluorométhyle, un trifluorométhoxy, un cyano, un amino, un carboxy, un carboxyalkyle en C₁-C₄, un phényle ;
. un groupe naphtyle non substitué ou substitué par un alkyle en C₁-C₄ ;
. un groupe pyridyle;
. un groupe styryle non substitué ou substitué par un alkyle en C₁-C₄ ;
- ou bien R_{IV} et R_{V} considérés ensemble représentent :
. un groupe 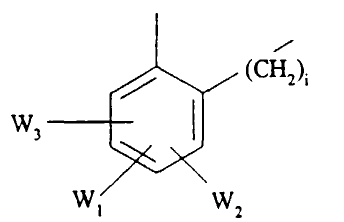 dans lequel le groupe phényle substitue le pyrazole en position 5 et le groupe -(CH₂)ᵢ- dans lequel i = 1 à 3 substitue le pyrazole en position 4, W₁, W₂ et W₃ substituent le cycle benzénique et représentent indépendamment l'hydrogène, un halogène ou un groupe hydroxyle ;
- R représente l'hydrogène, un alkyle en C₁-C₄ droit ou ramifié ;
- Z représente un groupe hydroxyle, un groupe alcoxy en C₁-C₆ ; un atome d'oxygène substitué par un groupe choisi parmi les groupes tertio-butyle, benzyle, benzyle substitué par un atome d'halogène, alkyle en C₁-C₆, trifluorométhyle, trifluorométhoxy et carboxy ; un groupe amino ;
- soit X représente l'hydrogène et X' représente l'hydrogène, un alkyle droit ou ramifié en C₁-C₆ ; un phényle ; un aminoalkyle en C₁-C₄ ; un hydroxyalkyle en C₁-C₄ ; un carboxyalkyle dans lequel le groupe alkyle est en C₁-C₄ ; un guanidinoalkyle dont le groupe alkyle est en C₁-C₄ ; un nitroguanidinoalkyle dont le groupe alkyle est en C₁-C₄ ; un cycloalkyle en C₃-C₇ ; un phénylalkyle dans lequel l'alkyle est en C₁-C₄ et dans lequel le phényle est éventuellement substitué par un halogène, un hydroxyle ou par un alkyle en C₁-C₃ ; un hétéroarylalkyle dans lequel l'hétéroaryle représente un imidazolyle, un indolyle non substitué ou substitué par un alkyle en C₁-C₄ ; par un hydroxy ou par un alcoxy en C₁-C₄ et dans lequel l'alkyle est en C₁-C₄ ; un groupe adamantyle ; un groupe adamantyle substitué par un ou deux méthyles, par un hydroxyle, un alcoxy en C₁-C₃ ; un atome d'halogène ; un groupe aza-1 adamantyle ;
- soit X représente l'hydrogène, X' et R considérés ensemble forment avec l'atome d'azote auquel R est lié et avec l'atome de carbone auquel X est lié un cycle , non substitué ou substitué par un hydroxyle, de formule 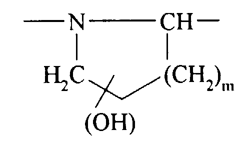 avec m = 0, 1 ou 2
ou un cycle de formule : 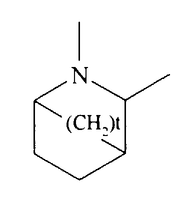 avec t = 1 ou 2
ou un cycle de formule : 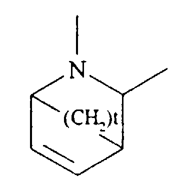 avec t = 1 ou 2
ou un cycle indolinyle ; perhydroindole, tétrahydro-4,5,6,7 thiéno [2,3-c]pyridyl-6;
- soit X et X' représentent chacun indépendamment un alkyle en C₁-C₄ ; un cycloalkyle en C₃-C₆ ; un phényle ;
- soit X, X' et l'atome de carbone auquel ils sont liés forment un groupe cycloalkylidène ayant au maximum 12 atomes de carbone, éventuellement substitué par un alkyle en C₁-C₃ ; un groupe adamantylidène ; un groupe adamantylidène substitué par un ou deux groupes méthyle ou par un hydroxyle, un alcoxy en C₁-C₃, un atome d'halogène ; un groupe aza-1 adamantylidène ; un groupe quinuclidinylidène ; un groupe pipéridinylidène-4 éventuellement N- substitué par un groupe benzyle ; un groupe tétraméthyl-2,2,6,6 pipéridinylidène ; un groupe tétrahydronaphtylidène ; un groupe tétrahydropyranilidène-4 ou tétrahydrothiopyranylidène-4 ; un groupe dihydro-2,3 (4H) benzopyranylidène-4; un groupe dihydro-2,3(4 H) benzothiopyranylidène-4 ;
un groupe bicyclo [2,2,1] heptane-5 ylidène-2 ; un groupe oxa-8 bicyclo [3,2,1] octèn-6-ylidène-3 ; un groupe thia-8 bicyclo [3,2,1]octanylidène-3 ; ou un de ses sels éventuels avec des acides organiques ou minéraux ou avec des bases minérales ou organiques.

2. Amido-3 pyrazole selon la revendication 1, de formule (I) dans laquelle R, X, X', Z, R_{IV} et R_{V} sont tels que définis dans la revendication 1, caractérisé en ce que R_{I}' représente un groupe phényle ou naphtyle, substitué par R₁, R'₁ et R"₁ tels que définis dans la revendication 1, ou un de ses sels éventuels avec des acides minéraux ou organiques ou avec des bases organiques ou minérales.

3. Amido-3 pyrazole selon la revendication 1, de formule (I) ou (I') dans lequel R, X, X', Z, R_{I}, ou R_{Ia} et R_{IV} sont tels que définis dans la revendication 1, caractérisé en ce que R_{V} représente un phényle ou un naphtyle substitué par R₅, R'₅ et R"₅ tels que définis dans la revendication 1, R₅, R'₅ et R"₅ étant de préférence l'hydrogène ou un alcoxy en C₁-C₄ ; ou un de ses sels éventuels avec des acides minéraux ou organiques ou avec des bases organiques ou minérales.

4. Amido-3 pyrazole selon la revendication 1, de formule I ou I', dans lequel X, X'et l'atome de carbone auquel ils sont liés forment un groupe adamantylidène.

5. Amido-3 pyrazole selon la revendication 4 de formule : 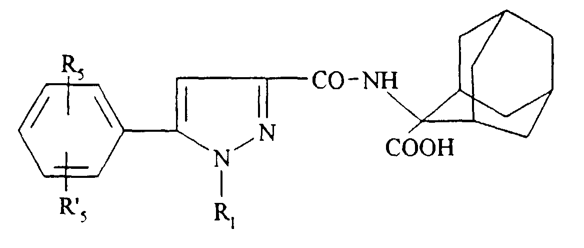 dans laquelle R_{I} est naphtyl-1, chloro-4 naphtyl-1, tétrahydro-5,6,7,8 naphtyl-1, isoquinolyl-5, quinolyl-8, chloro-7 quinolyl-4,2,1,3-benzothiadiazolyl-4, R₅ et R'₅ représentant respectivement 2- et 6-méthoxy.

6. Amido-3 pyrazole selon la revendication 1, de formule I dans laquelle R_{I} est chloro-7 quinolyl-4, R_{IV} est l'hydrogène, R_{V} est 2,6-diméthoxyphényle, R est l'hydrogène, Z est hydroxyle et X, X' et l'atome de carbone auquel ils sont liés forment un groupe adamantylidène.

7. Procédé pour la préparation des composés de formule (I) et (I') selon la revendication 1, caractérisé en ce que l'on traite un dérivé fonctionnel d'un acide pyrazolecarboxylique de formule (II) ou (II') : 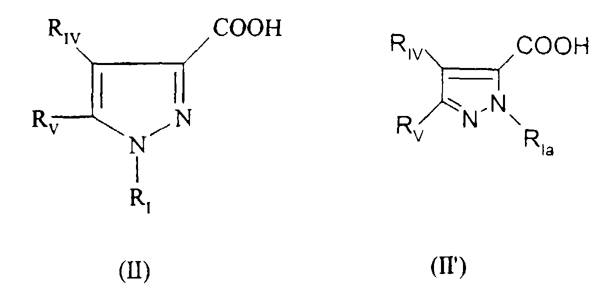 dans laquelle R_{I}, R_{IV}, R_{V} et R_{Ia} sont tels que définis ci-dessus, avec un aminoacide, éventuellement protégé par les groupements protecteurs habituels en synthèse peptidique, de formule : 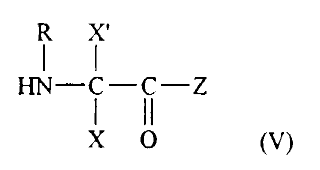 dans laquelle R, X, X' et Z sont tels que définis ci-dessus et on transforme éventuellement le composé ainsi obtenu en un de ses sels.

8. Composition pharmaceutique contenant en tant que principe actif un composé selon l'une quelconque des revendications 1 à 6 ou un de ses sels éventuels pharmaceutiquement acceptables.

9. Composition pharmaceutique selon la revendication 8 sous forme d'unité de dosage.

10. Composition pharmaceutique selon la revendication 9, caractérisée en ce qu'elle contient de 0,25 à 250 mg de principe actif en mélange avec au moins un excipient pharmaceutique.

11. N,N-diéthyl-{[phényl-1(naphtyl-2)-5 pyrazol-3] carbonylamino}-2 phényl-3 propanamide.

12. - acide {[(naphtyl-1)-1(diméthoxy-2,6 phényl)-5 pyrazolyl-3]carbonylamino}-2 bicyclo[2.2.1]heptanecarboxylique-2 ;
- acide {[(naphtyl-1)-1(diméthoxy-2,6 phényl)-5 pyrazolyl-3]carbonylamino}-9 bicyclo[3.3.1]nonanecarboxylique-9 ;
- acide {[(naphtyl-1)-1(diméthoxy-2,6-phényl)-5 pyrazolyl-3]carbonylamino}-2 bicyclo[3.2.1]octanecarboxylique-2 ;
- sel de sodium de l'acide {[(naphtyl-2)-5 phényl-1 pyrazolyl-3] carbonylamino}-2(S)-(acétamidométhylthio)-3 propanoïque.
- acide {[(dihydro-2,3 phtalazinyl-8 dione-1,4)-1 (diméthoxy-2,6 phényl)-5 , pyrazolyl-3]carbonylamino}-2 adamantanecarboxylique-2.

## Revendications (Revendications pour l'(les) Etat(s) contractant(s) suivant(s): ES)

1. Procédé de préparation d'un amido-3 pyrazole de formule (I) ou (I'): 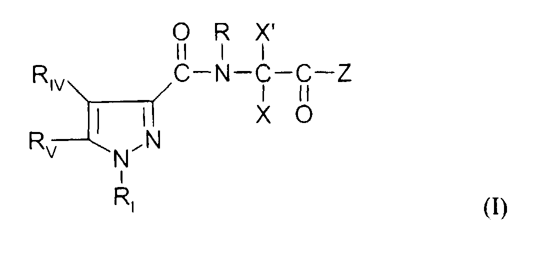 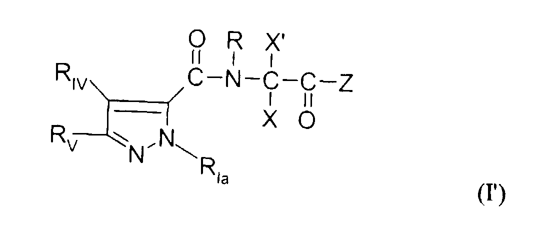 dans laquelle
- R_{I} représente
. un groupe 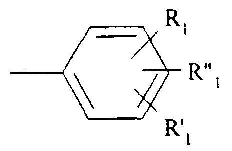 où R₁, R'₁ et R"₁ représentent chacun indépendamment un atome d'hydrogène, un atome d'halogène, un hydroxyle, un groupe alkyle droit ou ramifié en C₁-C₄, un groupe alcoxy en C₁-C₄, un groupe trifluorométhyle, un groupe trifluorométhoxy, un groupe nitro, un groupe carboxy, un groupe amino ;
. un groupe carboxyalkyle ou alcoxycarbonylalkyle dans lequel les alkyles sont en C₁-C₄ ;
. un groupe cycloalkyle dans lequel les alkyles sont en C₃-C₆ ;
. un groupe tétrahydronaphtyle ;
. un groupe pyridyle ;
. un naphtyle substitué par R₁, R'₁ et R"₁ tels que définis précédemment ;
. un groupe benzyle substitué par R₁, R'₁ et R"₁ tels que définis précédemment ;
. un groupe cinnamyle éventuellement substitué sur le noyau aromatique par un halogène, un hydroxyle, un alcoxy en C₁-C₄ ;
. un groupe quinolyle ou isoquinolyle, éventuellement substitué par R₁, R'₁ et R"₁ tels que définis ci-dessus ;
. un groupe benzothiazolyle-2 ;
. un groupe quinoxalinyldione ;
. un groupe phtalazinyl-1 ;
. un groupe benzothiadiazolyle ;
. un groupe méthylène substitué par un groupement hétérocyclique choisi parmi les groupes pyridyle et thiényle ;
- R_{Ia} représente un groupe benzyle substitué par R₁, R'₁ et R"₁ tels que définis ci-dessus ;
- R_{IV} représente un atome d'hydrogène, un atome d'halogène, un alkyle en C₁-C₆ ;
- R_{V} représente :
. un groupe 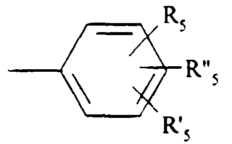
- où R₅, R'₅ et R"₅ représentent chacun indépendamment un atome d'hydrogène, un atome d'halogène, un alkyle droit ou ramifié en C₁-C₄, un hydroxyle, un alcoxy en C₁-C₄, un nitro, un trifluorométhyle, un trifluorométhoxy, un cyano, un amino, un carboxy, un carboxyalkyle en C₁-C₄, un phényle ;
. un groupe naphtyle non substitué ou substitué par un alkyle en C₁-C₄ ;
. un groupe pyridyle ;
. un groupe styryle non substitué ou substitué par un alkyle en C₁-C₄ ;
. ou bien R_{IV} et R_{V} considérés ensemble représentent :
. un groupe 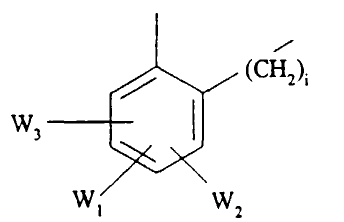 dans lequel le groupe phényle substitue le pyrazole en position 5 et le groupe -(CH₂)ᵢ- dans lequel i = 1 à 3 substitue le pyrazole en position 4, W₁, W₂ et W₃ substituent le cycle benzénique et représentent indépendamment l'hydrogène, un halogène ou un groupe hydroxyle ;
- R représente l'hydrogène, un alkyle en C₁-C₄ droit ou ramifié ;
- Z représente un groupe hydroxyle, un groupe alcoxy en C₁-C₆ ; un atome d'oxygène substitué par un groupe choisi parmi les groupes tertio-butyle, benzylc, benzyle substitué par un atome d'halogène, alkyle en C₁-C₆, trifluorométhyle, trifluorométhoxy et carboxy ; un groupe amino ;
- soit X représente l'hydrogène et X' représente l'hydrogène, un alkyle droit ou ramifié en C₁-C₆ ; un phényle ; un aminoalkyle en C₁-C₄ ; un hydroxyalkyle en C₁-C₄ ; un carboxyalkylc dans lequel le groupe alkyle est en C₁-C₄ ; un guanidinoalkyle dont le groupe alkyle est en C₁-C₄ ; un nitroguanidinoalkyle dont le groupe alkyle est en C₁-C₄ ; un cycloalkylc en C₃-C₇ ; un phénylalkyle dans lequel l'alkyle est en C₁-C₄ et dans lequel le phényle est éventuellement substitué par un halogène, un hydroxyle ou par un alkyle en C₁-C₃ ; un hétéroarylalkyle dans lequel l'hétéroaryle représente un imidazolyle, un indolyle non substitué ou substitué par un alkyle en C₁-C₄ ; par un hydroxy ou par un alcoxy en C₁-C₄ et dans lequel l'alkyle est en C₁-C₄ ; un groupe adamantyle ; un groupe adamantyle substitué par un ou deux méthyles, par un hydroxyle, un alcoxy en C₁-C₃ ; un atome d'halogène ; un groupe aza-1 adamantyle ;
- soit X représente l'hydrogène, X' et R considérés ensemble forment avec l'atome d'azote auquel R est lié et avec l'atome de carbone auquel X est lié un cycle, non substitué ou substitué par un hydroxyle, de formule 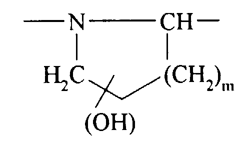 avec m = 0, 1 ou 2
ou un cycle de formule : 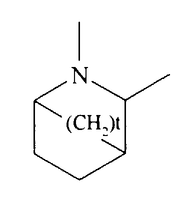 avec t = 1 ou 2
ou un cycle de formule : 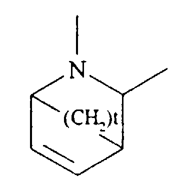 avec t = 1 ou 2
ou un cycle indolinyle ; perhydroindole, tétrahydro-4,5,6,7 thiéno[2,3-c]pyridyl-6 ;
- soit X et X' représentent chacun indépendamment un alkyle en C₁-C₄ ; un cycloalkyle en C₃-C₆ ; un phényle ;
- soit X, X' et l'atome de carbone auquel ils sont liés forment un groupe cycloalkylidène ayant au maximum 12 atomes de carbone, éventuellement substitué par un alkyle en C₁-C₃ ; un groupe adamantylidène ; un groupe adamantylidène substitué par un ou deux groupes méthyle ou par un hydroxyle, un alcoxy en C₁-C₃, un atome d'halogène ; un groupe aza-1 adamantylidène ; un groupe quinuclidinylidène ; un groupe pipéridinylidène-4 éventuellement N-substitué par un groupe benzyle ; un groupe tétraméthyl-2,2,6,6 pipéridinylidène ; un groupe tétrahydronaphtylidène ; un groupe tétrahydropyranilidène-4 ou tétrahydrothiopyranylidène-4 ; un groupe dihydro-2,3 (4H) benzopyranylidène-4 ; un groupe dihydro-2,3(4 H) benzothiopyranylidène-4 ;
un groupe bicyclo [2,2,1] heptène-5 ylidène-2 ; un groupe oxa-8 bicyclo [3,2,1] octèn-6-ylidène-3 ; un groupe thia-8 bicyclo [3,2,1] octanylidène-3 ; ou d'un de ses sels éventuels avec des acides organiques ou minéraux ou avec des bases minérales ou organiques, caractérisé en ce que l'on traite un dérivé fonctionnel d'un acide pyrazolecarboxylique de formule (II) ou (II'): 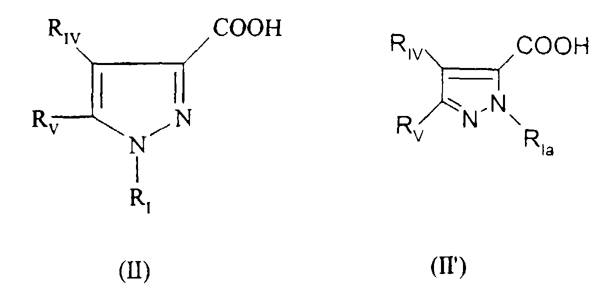 dans laquelle R_{I}, R_{IV}, R_{V} et R_{Ia} sont tels que définis ci-dessus, avec un aminoacide, éventuellement protégé par les groupements protecteurs habituels en synthèse peptidique, de formule: 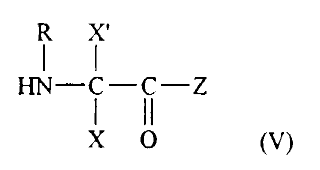 dans laquelle R, X, X' et Z sont tels que définis ci-dessus et on transforme éventuellement le composé ainsi obtenu en un de ses sels.

2. Procédé de préparation d'un amido-3 pyrazole de formule (I) selon la revendication 1, caractérisé en ce que l'on traite un composé de formule (II), dans laquelle R_{I} représente un groupe phényle ou naphtyle, substitué par R₁, R'₁ et R"₁ tels que définis dans la revendication 1, et R_{IV} et R_{V} sont tels que définis dans la revendication 1, avec un composé de formule (V) dans laquelle R, X, X' et Z sont tels que définis dans la revendication 1, et on transforme éventuellement en un de ses sels éventuels avec des acides minéraux ou organiques ou avec des bases organiques ou minérales.

3. Procédé de préparation d'un amido-3 pyrazole de formule (I) ou (I') selon la revendication 1, caractérisé en ce que l'on traite un composé de formules (II) ou (II') dans lesquelles R_{V} représente un phényle ou un naphtyle substitué par R₅, R'₅ et R"₅ tels que définis dans la revendication 1, R₅, R'₅ et R"₅ étant de préférence l'hydrogène ou un alcoxy en C₁-C₄, et R₁, ou R_{Ia}, et R_{IV} sont tels que définis dans la revendication 1, avec un composé de formule (V) dans laquelle R, X, X' et Z sont tels que définis dans la revendication 1, et on transforme éventuellement en un de ses sels éventuels avec des acides minéraux ou organiques ou avec des bases organiques ou minérales.

4. Procédé de préparation d'un amido-3-pyrazole de formule (I) ou (I') selon la revendication 1, caractérisé en ce qu'on utilise un composé de formule (V) dans laquelle X, X' et l'atome de carbone auquel ils sont liés fonnent un groupe adamantylidène.

5. Procédé de préparation selon la revendication 4 d'un amido-3 pyrazole de formule: 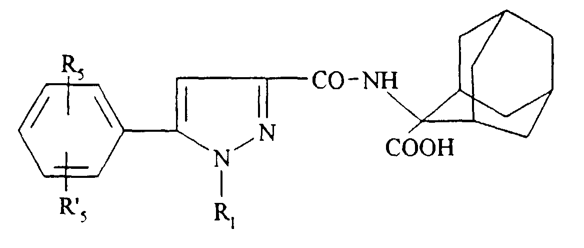 caractérisé en ce que l'on traite un composé de formule (II) dans laquelle R_{IV} est un hydrogène, R_{V} est un phényle substitué par R₅ et R'₅ représentant respectivement 2- et 6-méthoxy, et R_{I} est naphtyl-1, chloro-4 naphtyl-1, tétrahydro-5,6-7,8 naphtyl-1, isoquinolyl-5, quinolyl-8, chloro-7 quinolyl-4,2,1,3-benzothiadiazolyl-4, avec un composé de formule (V) dans laquelle R est l'hydrogène, X, X' et l'atome de carbone auquel ils sont liés forment un groupe adamantylidène, et Z est un groupe hydroxyle.

6. Procédé de préparation d'un amido-3 pyrazole de formule (I) selon la revendication 1, caractérisé en ce que l'on traite un composé de formule (Il) dans laquelle R_{I} est chloro-7 quinolyl-4, R_{IV} est l'hydrogène, et R_{V} est 2,6-diméthoxyphényle avec un composé de formule (V) dans laquelle R est l'hydrogène, Z est hydroxyle et X, X' et l'atome de carbone auquel ils sont liés forment un groupe adamantylidène.

7. Procédé de préparation d'une composition pharmaceutique, caractérisé en ce qu'il consiste à mélanger, en tant que principe actif, un composé obtenu par le procédé selon l'une quelconque des revendications 1 à 6 ou un de ses sels éventuels pharmaceutiquement acceptables avec au moins un excipient pharmaceutique.

8. Procédé de préparation d'une composition pharmaceutique selon la revendication 7, caractérisé en ce que la composition est transformée en une unité de dosage.

9. Procédé de préparation d'une composition pharmaceutique selon la revendication 8, caractérisé en ce qu'il consiste à mélanger de 0,25 à 250 mg de principe actif avec au moins un excipient pharmaceutique.

10. Procédé de préparation du N,N-diéthyl-{[phényl-1(naphtyl-2)-5 pyrazol-3]carbonylamino}-2 phényl-3 propanamide, caractérisé en ce que l'on traite un dérivé fonctionnel d'un acide pyrazolecarboxylique de formule (II) : 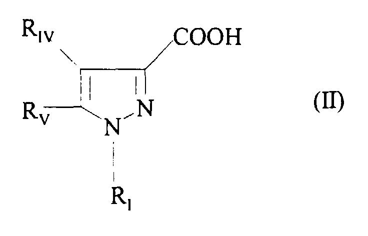 dans laquelle R_{I} est C₆H₅, R_{IV} est H et Rv est 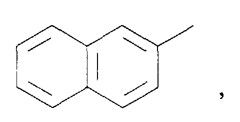 avec un aminoacide, éventuellement protégé par les groupements protecteurs habituels en synthèse peptidique, de formule: 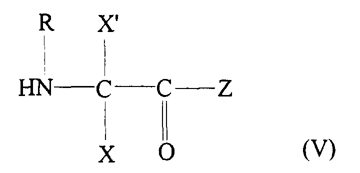 dans laquelle R est H, X' est H, X est -CH₂-C₆H₅ et Z est -N-(C₂H₅)₂.

11. Procédé de préparation de l'acide {[(naphtyl-1)-1(diméthoxy-2,6 phényl)-5 pyrazolyl-3]carbonylamino}-2 bicyclo[2.2.1]heptanecarboxylique-2, caractérisé en ce que l'on traite un dérivé fonctionnel d'un acide pyrazolecarboxylique de formule (II) : 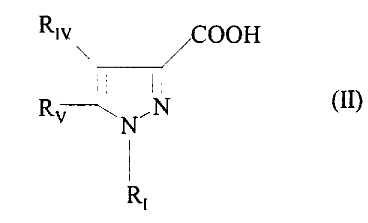 dans laquelle R_{I} est 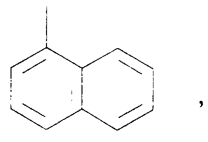 R_{IV} est H et R_{V} est 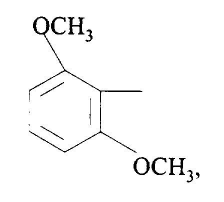 avec un aminoacide, éventuellement protégé par les groupements protecteurs habituels en synthèse peptidique, de formule: 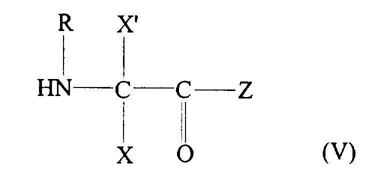 dans laquelle R est H, X, X' et l'atome de carbone auquels ils sont liés forment le cycle 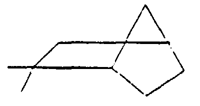 et Z est OH.

12. Procédé de préparation de l'acide {[(naphtyl-1)-1 (diméthoxy-2,6 phényl)-5 pyrazolyl-3]carbonylamino}-9 bicyclo[3.3.1]nonanecarboxylique-9, caractérisé en ce que l'on traite un dérivé fonctionnel d'un acide pyrazolecarboxylique de formule (II) : 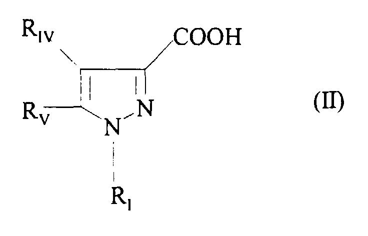 dans laquelle R_{I} est 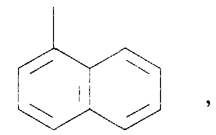 R_{IV} est H et R_{V} est 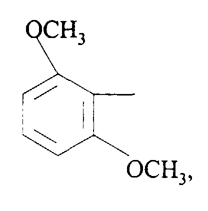 avec un aminoacide, éventuellement protégé par les groupements protecteurs habituels en synthèse peptidique, de formule : 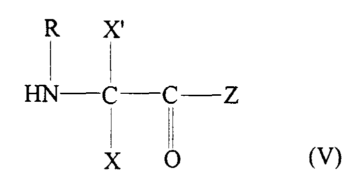 dans laquelle R est H, X', X et l'atome de carbone auquel ils sont liés forment le cycle 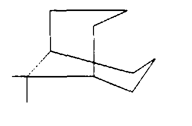 et Z est OH.

13. Procédé de préparation de l'acide {[(naphtyl-1)-1 (diméthoxy-2,6 phényl)-5 pyrazolyl-3]carbonylamino}-2 bicyclo[3.2.1]oetanecarboxylique-2, caractérisé en ce que l'on traite un dérivé fonctionnel d'un acide pyrazolecarboxylique de formule (II) : 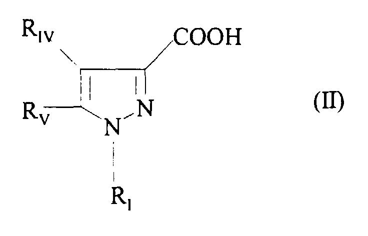
dans laquelle R_{I} est 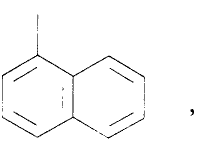 R_{IV} est H et R_{V} est 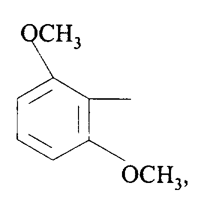 avec un aminoacide, éventuellement protégé par les groupements protecteurs habituels en synthèse peptidique, de formule : 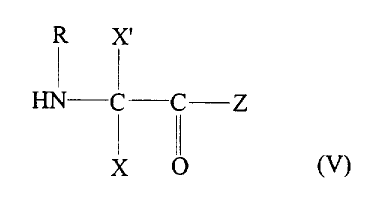 dans laquelle R est H, X, X' et l'atome de carbone auquels ils sont liés forment le cycle 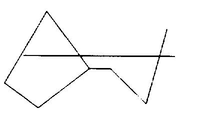 et Z et OH.

14. Procédé de préparation du sel de sodium de l'acide {[(naphtyl-2)-5 phényl-1 pyrazolyl-3]carbonylamino}-2(S)-(acétamidométhylthio)-3 propanoïque, caractérisé en ce que l'on traite un dérivé fonctionnel d'un acide pyrazolecarboxylique de formule (II) : 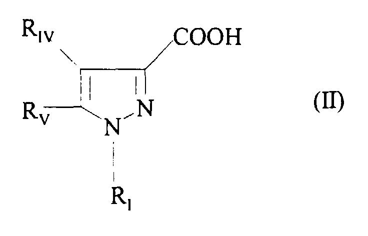 dans laquelle R_{I} est le phényle, R_{IV} est H et R_{V} est un naphtyle en position 2, avec un aminoacide, éventuellement protégé par les groupements protecteurs habituels en synthèse peptidique, de formule : 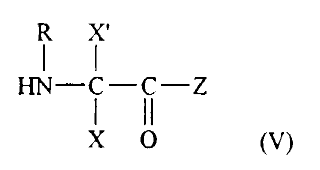 dans laquelle R est H, X est H, X' est un acétamidométhylthiométhyle et Z est OH, et on transforme le composé en son sel sodique.

15. Procédé de préparation de l'acide {[(dihydro-2,3 phtalazinyl-8 dione-1,4)-1 (diméthoxy-2,6 phényl)-5 pyrazolyl-3]carbonylamino}-2 adamantanecarboxyiique-2, caractérisé en ce que l'on traite un dérivé fonctionnel d'un acide pyrazolecarboxylique de formule (II) : 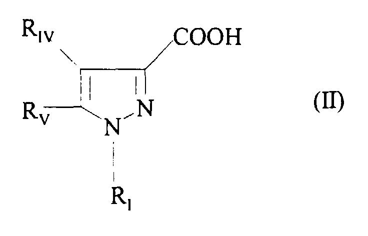 dans laquelle R_{I} est 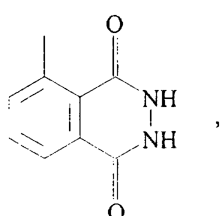 R_{IV} est H et R_{V} est 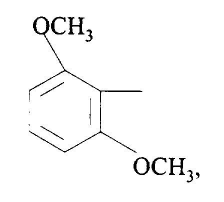 avec un aminoacide, éventuellement protégé par les groupements protecteurs habituels en synthèse peptidique, de formule : 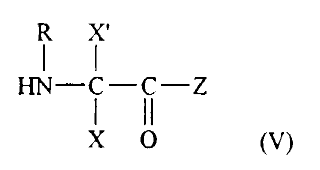 dans laquelle R est H, X, X' et l'atome de carbone auquel ils sont liés forment le cycle 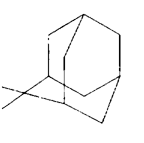 et Z est OH.

## Revendications (Revendications pour l'(les) Etat(s) contractant(s) suivant(s): GR)

1. Amido-3-pyrazole de formule (I) ou (I') : 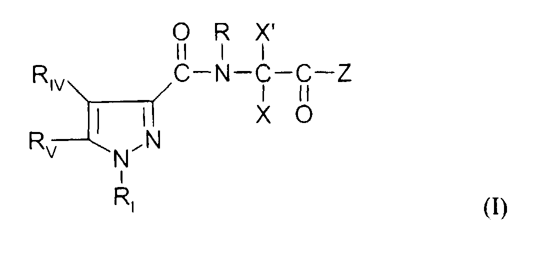 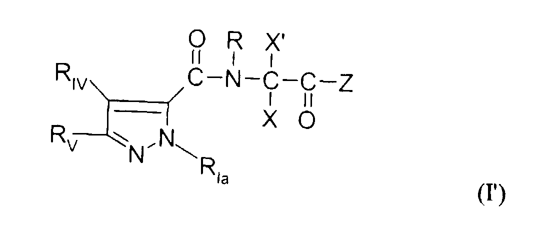 dans laquelle
- R_{I} représente
. un groupe 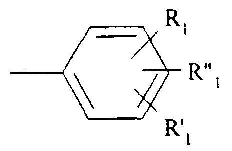 où R₁, R'₁ et R"₁ représentent chacun indépendamment un atome d'hydrogène, un atome d'halogène, un hydroxyle, un groupe alkyle droit ou ramifié en C₁-C₄, un groupe alcoxy en C₁-C₄, un groupe trifluorométhyle, un groupe trifluorométhoxy, un groupe nitro, un groupe carboxy, un groupe amino ;
. un groupe carboxyalkyle ou alcoxycarbonylalkyle dans lequel les alkyles sont en C₁-C₄ ;
. un groupe cycloalkyle dans lequel les alkyles sont en C₃-C₆ ;
. un groupe tétrahydronaphtyle ;
. un groupe pyridyle ;
. un naphtyle substitué par R₁, R'₁ et R"₁ tels que définis précédemment ;
. un groupe benzyle substitué par R₁, R'₁ et R"₁ tels que définis précédemment ;
. un groupe cinnamyle éventuellement substitué sur le noyau aromatique par un halogène, un hydroxyle, un alcoxy en C₁-C₄ ;
. un groupe quinolyle ou isoquinolyle, éventuellement substitué par R₁, R'₁ et R"₁ tels que définis ci-dessus ;
. un groupe benzothiazolyle-2 ;
. un groupe quinoxalinyldione ;
. un groupe phtalazinyl-1 ;
. un groupe benzothiadiazolyle ;
. un groupe méthylène substitué par un groupement hétérocyclique choisi parmi les groupes pyridyle et thiényle ;
- R_{Ia} représente un groupe benzyle substitué par R₁, R'₁ et R"₁ tels que définis ci-dessus ;
- R_{IV} représente un atome d'hydrogène, un atome d'halogène, un alkyle en C₁-C₆ ;
- R_{V} représente :
. un groupe 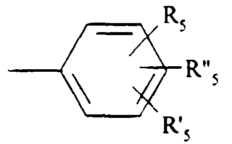
- où R₅, R'₅ et R"₅ représentent chacun indépendamment un atome d'hydrogène, un atome d'halogène, un alkyle droit ou ramifié en C₁-C₄, un hydroxyle, un alcoxy en C₁-C₄, un nitro, un trifluorométhyle, un trifluorométhoxy, un cyano, un amino, un carboxy, un carboxyalkyle en C₁-C₄, un phényle ;
. un groupe naphtyle non substitué ou substitué par un alkyle en C₁-C₄ ;
. un groupe pyridyle ;
. un groupe styryle non substitué ou substitué par un alkyle en C₁-C₄ ;
- ou bien R_{IV} et R_{V} considérés ensemble représentent :
. un groupe 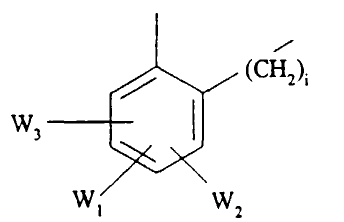 dans lequel le groupe phényle substitue le pyrazole en position 5 et le groupe -(CH₂)ᵢ- dans lequel i = 1 à 3 substitue le pyrazole en position 4, W₁, W₂ et W3 substituent le cycle benzénique et représentent indépendamment l'hydrogène, un halogène ou un groupe hydroxyle ;
- R représente l'hydrogène, un alkyle en C₁-C₄ droit ou ramifié ;
- Z représente un groupe hydroxyle, un groupe alcoxy en C₁-C₆ ; un atome d'oxygène substitué par un groupe choisi parmi les groupes tertio-butyle, benzyle, benzyle substitué par un atome d'halogène, alkyle en C₁-C₆, trifluorométhyle, trifluorométhoxy et carboxy ; un groupe amino ;
- soit X représente l'hydrogène et X' représente l'hydrogène, un alkyle droit ou ramifié en C₁-C₆ ; un phényle ; un aminoalkyle en C₁-C₄ ; un hydroxyalkyle en C₁-C₄ ; un carboxyalkyle dans lequel le groupe alkyle est en C₁-C₄ ; un guanidinoalkyle dont le groupe alkyle est en C₁-C₄ ; un nitroguanidinoalkyle dont le groupe alkyle est en C₁-C₄ ; un cycloalkyle en C₃-C₇ ; un phénylalkyle dans lequel l'alkyle est en C₁-C₄ et dans lequel le phényle est éventuellement substitué par un halogène, un hydroxyle ou par un alkyle en C₁-C₃ ; un hétéroarylalkyle dans lequel l'hétéroaryle représente un imidazolyle, un indolyle non substitué ou substitué par un alkyle en C₁-C₄ ; par un hydroxy ou par un alcoxy en C₁-C₄ et dans lequel l'alkyle est en C₁-C₄ ; un groupe adamantyle ; un groupe adamantyle substitué par un ou deux méthyles, par un hydroxyle, un alcoxy en C₁-C₃ ; un atome d'halogène ; un groupe aza-1 adamantyle ;
- soit X représente l'hydrogène, X' et R considérés ensemble forment avec l'atome d'azote auquel R est lié et avec l'atome de carbone auquel X est lié un cycle, non substitué ou substitué par un hydroxyle, de fonnule 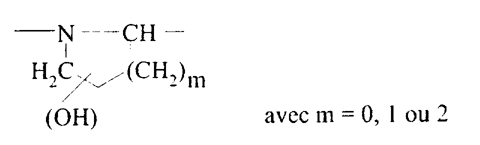 ou un cycle de formule : 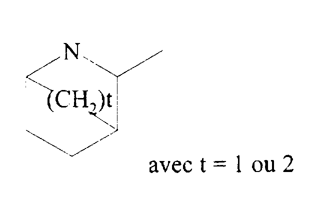 ou un cycle de formule : 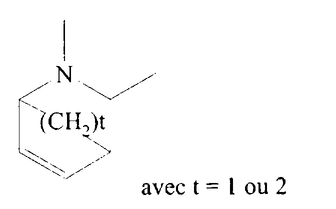 ou un cycle indolinyle ; perhydroindole, tétrahydro-4,5,6,7 thiéno[2,3-c]pyridyl-6 ;
- soit X et X' représentent chacun indépendamment un alkyle en C₁-C₄ ; un cycloalkyle en C₃-C₆ ; un phényle ;
- soit X, X' et l'atome de carbone auquel ils sont liés fonnent un groupe cycloalkylidène ayant au maximum 12 atomes de carbone, éventuellement substitué par un alkyle en C₁-C₃ ; un groupe adamantylidène ; un groupe adamantylidène substitué par un ou deux groupes méthyle ou par un hydroxyle, un alcoxy en C₁-C₃, un atome d'halogène ; un groupe aza-1 adamantylidène ; un groupe quinuclidinylidène ; un groupe pipéridinylidène-4 éventuellement N-substitué par un groupe benzyle ; un groupe tétraméthyl-2,2,6,6-pipéridinylidène ; un groupe tétrahydronaphtylidène ; un groupe tétrahydropyranilidène-4 ou tétrahydrothiopyranylidène-4 ; un groupe dihydro-2,3 (4H) benzopyranylidène-4; un groupe dihydro-2,3(4 H) benzothiopyranylidène-4 ;
un groupe bicyclo [2,2,1] heptène-5 ylidène-2 ; un groupe oxa-8 bicyclo [3,2,1] octèn-6-ylidène-3 ; un groupe thia-8 bicyclo [3,2,1] octanylidène-3 ; ou un de ses sels éventuels avec des acides organiques ou minéraux ou avec des bases minérales ou organiques.

2. Amido-3 pyrazole selon la revendication 1, de formule (I) dans laquelle R, X, X', Z, R_{IV} et R_{V} sont tels que définis dans la revendication 1, caractérisé en ce que R_{I} représente un groupe phényle ou naphtyle, substitué par R₁, R'₁ et R"₁ tels que définis dans la revendication 1, ou un de ses sels éventuels avec des acides minéraux ou organiques ou avec des bases organiques ou minérales.

3. Amido-3 pyrazole selon la revendication 1, de formule (I) ou (I') dans lequel R, X, X', Z, R_{I}, ou R_{Ia} et R_{IV} sont tels que définis dans la revendication 1, caractérisé en ce que Rv représente un phényle ou un naphtyle substitué par R₅, R'₅ et R"₅ tels que définis dans la revendication 1, R₅, R'₅ et R"₅ étant de préférence l'hydrogène ou un alcoxy en C₁-C₄ ; ou un de ses sels éventuels avec des acides minéraux ou organiques ou avec des bases organiques ou minérales.

4. Amido-3-pyrazole selon la revendication 1, de formule I ou I', dans lequel X, X' et l'atome de carbone auquel ils sont liés forment un groupe damantylidène.

5. Amido-3 pyrazole selon la revendication 4 de formule : 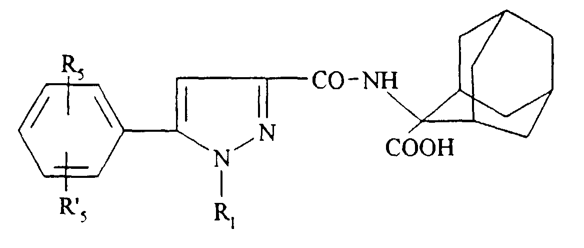 dans laquelle R_{I} est naphtyl-1, chloro-4 naphtyl-1, tétrahydro-5,6,7,8 naphtyl-1, isoquinolyl-5, quinolyl-8, chloro-7 quinolyl-4,2,1,3-benzothiadiazolyl-4, R₅ et R'₅ représentent respectivement 2- et 6-méthoxy.

6. Amido-3 pyrazole selon la revendication 1, de formule I dans laquelle R_{I} est chloro-7 quinolyl-4, R_{IV} est l'hydrogène, R_{V} est 2,6-diméthoxyphényle, R est l'hydrogène, Z est hydroxyle et X, X' et l'atome de carbone auquel ils sont liés forment un groupe adamantylidène.

7. Procédé pour la préparation des composés de formule (I) et (I') selon la revendication 1, caractérisé en ce que l'on traite un dérivé fonctionnel d'un acide pyrazolecarboxylique de formule (II) ou (II'): 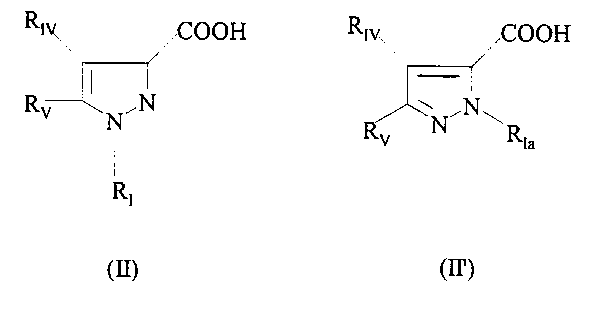 dans laquelle R_{I}, R_{IV}, R_{V} et R_{Ia} sont tels que définis ci-dessus, avec un aminoacide, éventuellement protégé par les groupements protecteurs habituels en synthèse peptidique, de formule : 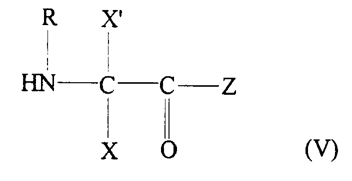 dans laquelle R, X, X' et Z sont tels que définis ci-dessus et on transforme éventuellement le composé ainsi obtenu en un de ses sels.

8. Procédé de préparation d'une composition pharmaceutique, caractérisé en ce qu'il consiste à mélanger, en tant que principe actif, un composé selon l'une quelconque des revendications 1 à 6 ou un de ses sels éventuels pharmaceutiquement acceptables avec au moins un excipient pharmaceutique.

9. Procédé de préparation d'une composition pharmaceutique selon la revendication 8, caractérisé en ce que la composition est transformée en une unité de dosage.

10. Procédé de préparation d'une composition pharmaceutique selon la revendication 9, caractérisé en ce qu'il consiste à mélanger de 0,25 à 250 mg de principe actif avec au moins un excipient pharmaceutique.

11. N,N-diéthyl-{[phényl-1(naphtyl-2)-5 pyrazol-3]carbonylamino}-2 phényl-3 propanamide.

12. - acide {[(naphtyl-1)-1(diméthoxy-2,6 phényl)-5 pyrazolyl-3]-carbonylamino}-2 bicyclo[2.2.1]heptanecarboxylique-2 ;
- acide {[(naphtyl-1)-1(diméthoxy-2,6 phényl)-5 pyrazolyl-3]carbonylamino}-9 bicyclo[3.3.1]nonanecarboxylique-9 ;
- acide {[(naphtyl-1)-1(diméthoxy-2,6-phényl)-5 pyrazolyl-3]carbonylamino}-2 bicyclo[3.2.1]octanecarboxylique-2 ;
- sel de sodium de l'acide {[(naphtyl-2)-5 phényl-1 pyrazolyl-3]carbonylamino}-2(S)-(acétamidométhylthio)-3 propanoïque ;
- acide {[(dihydro-2,3 phtalazinyl-8 dione-1,4)-1 (diméthoxy-2,6 phényl)-5 pyrazolyl-3]carbonylamino}-2 adamantanecarboxylique-2.

## Patentansprüche (Patentansprüche für folgende(n) Vertragsstaat(en): AT, BE, CH, LI, DE, DK, FR, GB, IT, LU, NL, SE)

1. 3-Amidopyrazol der Formel (I) oder (I') 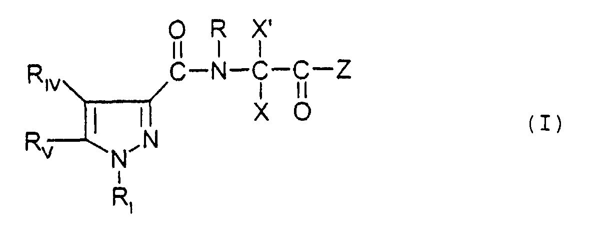 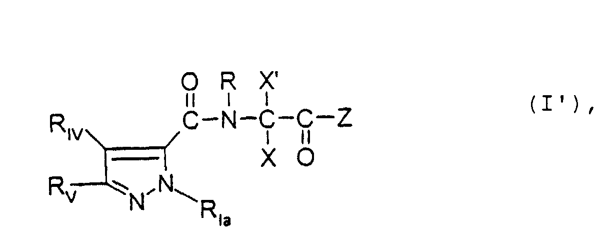 worin bedeuten:
- R_{I}
. eine Gruppe 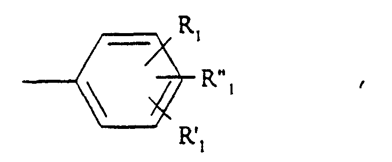 worin R₁, R'₁ und R"₁ unabhängig Wasserstoff, ein Halogenatom, Hydroxy, geradkettiges oder verzweigtes C₁-C₄-Alkyl, C₁-C₄-Alkoxy, Trifluormethyl, Trifluormethoxy, Nitro, Carboxy, Amino bedeuten,
. Carboxyalkyl oder Alkoxycarbonylalkyl, worin Alkyl C₁-C₄-Alkyl bedeutet,
. Cycloalkyl, worin Alkyl C₃-C₆-Alkyl bedeutet,
. Tetrahydronaphthyl,
. Pyridyl,
. Naphthyl, das mit R₁, R'₁ und R"₁, die wie weiter oben definiert sind, substituiert ist,
. Benzyl, das mit R₁, R'₁ und R"₁, die wie weiter oben definiert sind, substituiert ist,
. Cinnamyl, das gegebenenfalls am aromatischen Ring mit einem Halogen, Hydroxy, C₁-C₄-Alkoxy substituiert ist,
. Chinolyl oder Isochinolyl, das gegebenenfalls mit R₁, R'₁ und R"₁, die wie weiter oben definiert sind, substituiert ist,
. 2-Benzothiazolyl,
. Chinoxalinyldion,
. 1-Phthalazinyl,
. Benzothiadiazolyl,
. Methylen, das mit einer heterocyclischen Gruppe substituiert ist, die unter Pyridyl und Thienyl ausgewählt ist,
- R_{Ia} Benzyl, das mit R₁, R'₁ und R"₁, die wie weiter oben definiert sind, substituiert ist,
- R_{IV} Wasserstoff, ein Halogenatom, C₁-C₆-Alkyl,
- R_{V}
. eine Gruppe 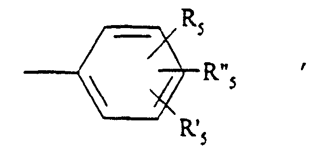 worin R₅, R'₅ und R"₅ unabhängig Wasserstoff, ein Halogenatom, geradkettiges oder verzweigtes C₁-C₄-Alkyl, Hydroxy, C₁-C₄-Alkoxy, Nitro, Trifluormethyl, Trifluormethoxy,Cyano, Amino, Carboxy, C₁-C₄-Carboxyalkyl, Phenyl bedeuten,
. Naphthyl, das nicht substituiert ist oder mit C₁-C₄-Alkyl substituiert ist,
. Pyridyl,
. Styryl, das nicht substituiert ist oder mit C₁-C₄-Alkyl substituiert ist,
- oder R_{IV} und R_{V} zusammen
. eine Gruppe 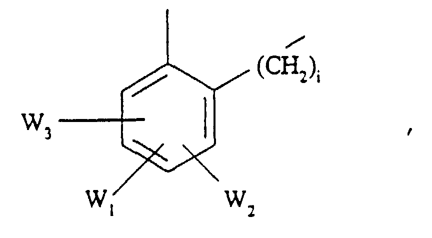 wobei der Phenylrest den Pyrazolring in 5-Stellung substituiert, die Gruppe -(CH₂)ᵢ-, in der i 1 bis 3 bedeutet, den Pyrazolring in 4-Stellung substituiert und die Reste W₁, W₂ und W₃ Substituenten am Benzolring sind und unabhängig Wasserstoff, Halogen oder Hydroxy bedeuten,
- R Wasserstoff, geradkettiges oder verzweigtes C₁-C₄-Alkyl,
- Z Hydroxy, C₁-C₆-Alkoxy, ein Sauerstoffatom, das mit einer Gruppe substituiert ist, die unter tert.-Butyl, Benzyl, Benzyl, das mit einem Halogen, C₁-C₆-Alkyl, Trifluormethyl, Trifluormethoxy, Carboxy substituiert ist, ausgewählt ist, Amino,
- X Wasserstoff und X' Wasserstoff, geradkettiges oder verzweigtes C₁-C₆-Alkyl, Phenyl, C₁-C₄-Aminoalkyl, C₁-C₄-Hydroxyalkyl, Carboxyalkyl, worin Alkyl C₁-C₄-Alkyl bedeutet, Guanidinoalkyl, worin Alkyl C₁-C₄-Alkyl bedeutet, Nitroguanidinoalkyl, worin Alkyl C₁-C₄-Alkyl bedeutet, C₃-C₇-Cycloalkyl, Phenylalkyl, worin Alkyl C₁-C₄-Alkyl bedeutet und der Phenylrest gegebenenfalls mit einem Halogen, einer Hydroxygruppe oder C₁-C₃-Alkyl substituiert ist, Heteroarylalkyl, worin Heteroaryl ein Imidazolyl, ein Indolyl, das nicht substituiert ist oder mit C₁-C₄-Alkyl, einer Hydroxygruppe oder C₁-C₄-Alkoxy substituiert ist, darstellt und Alkyl C₁-C₄-Alkyl bedeutet, Adamantyl, Adamantyl, das mit ein oder zwei Methylgruppen, Hydroxy, C₁-C₃-Alkoxy, einem Halogenatom substituiert ist, 1-Azaadamantyl, oder
- X Wasserstoff und X' und R zusammen mit dem Stickstoffatom, mit dem der Rest R verbunden ist, und mit dem Kohlenstoffatom, mit dem X verbunden ist, einen Ring, der nicht substituiert ist oder mit einer Hydroxygruppe substituiert ist, der Formel 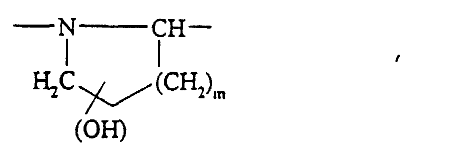 worin m 0,1 oder 2 bedeutet,
oder einen Ring der Formel 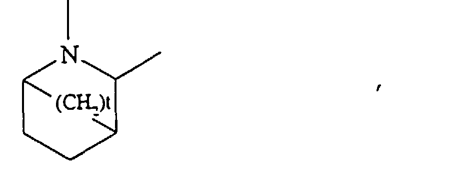 worin t 1 oder 2 bedeutet,
oder einen Ring der Formel 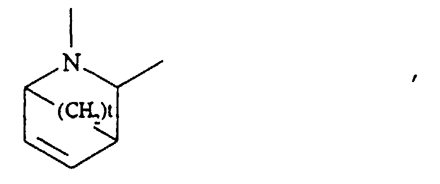 worin t 1 oder 2 bedeutet,
oder einen Indolinylring, Perhydroindolring, 4,5,6,7-Tetrahydrothieno[2,3-c]-6-pyridyl-Ring, oder
- X und X' unabhängig C₁-C₄-Alkyl, C₃-C₆-Cycloalkyl, Phenyl, oder
- X, X' und das Kohlenstoffatom, mit dem sie verbunden sind, eine Cycloalkylidengruppe mit maximal 12 Kohlenstoffatomen, die gegebenenfalls mit C₁-C₃-Alkyl substituiert ist, Adamantyliden, Adamantyliden, das mit ein oder zwei Methylgruppen, Hydroxy, C₁-C₃-Alkoxy, einem Halogenatom substituiert ist, 1-Azaadamantyliden, Chinucli-dinyliden, 4-Piperidinyliden, das gegebenenfalls am N-Atom mit Benzyl substituiert ist, 2,2,6,6-Tetramethylpiperidinyliden, Tetrahydronaphthyliden, 4-Tetrahydropyranyliden oder 4-Tetrahydrothiopyranyliden, (4H)-2,3-Dihydro-4-benzopyranyliden, (4H)-2,3-Dihydro-4-benzothiopyranyliden, Bicyclo[2,2,1]-5-hepten-2-yliden, 8-Oxabicyclo[3,2,1]-6-octen-3-yliden, 8-Thiabicyclo[3,2,1]-3-octanyliden,
oder eines seiner möglichen Salze mit organischen oder anorganischen Säuren oder mit anorganischen oder organischen Basen.

2. 3-Amidopyrazol nach Anspruch 1 der Formel (I), in der R, X, X', Z, R_{IV} und R_{V} wie in Anspruch 1 definiert sind, dadurch gekennzeichnet, daß R₁ Phenyl oder Naphthyl bedeutet, das mit R₁, R'₁ und R"₁, die wie in Anspruch 1 definiert sind, substituiert ist, oder eines seiner möglichen Salze mit anorganischen oder organischen Säuren oder mit organischen oder anorganischen Basen.

3. 3-Amidopyrazol nach Anspruch 1 der Formel (I) oder (I'), in der R, X, X', Z, R_{I} oder R_{Ia} und R_{IV} wie in Anspruch 1 definiert sind, dadurch gekennzeichnet, daß R_{V} Phenyl oder Naphthyl bedeutet, das mit R₅, R'₅ und R"₅, die wie in Anspruch 1 definiert sind, substituiert ist, wobei R₅, R'₅ und R"₅ vorzugsweise Wasserstoff oder C₁-C₄-Alkoxy bedeuten, oder eines seiner möglichen Salze mit anorganischen oder organischen Säuren oder mit organischen oder anorganischen Basen.

4. 3-Amidopyrazol nach Anspruch 1 der Formel (I) oder (I'), in der X, X' und das Kohlenstoffatom, mit dem sie verbunden sind, eine Adamantylidengruppe bilden.

5. 3-Amidopyrazol nach Anspruch 4 der Formel 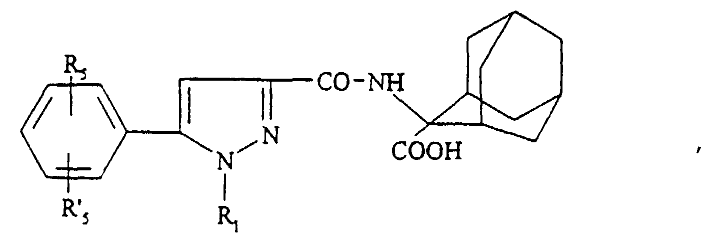 worin R_{I} 1-Naphthyl, 4-Chlor-1-naphthyl, 5,6,7,8-Tetrahydro-1-naphthyl, 5-Isochinolyl, 8-Chinolyl, 7-Chlor-4,2,1,3-chinolyl-4-benzothiadiazolyl bedeutet und R₅ und R'₅ 2-Methoxy bzw. 6-Methoxy darstellen.

6. 3-Amidopyrazol nach Anspruch 1 der Formel(I), worin R_{I} 7-Chlor-4-chinolyl, R_{IV} Wasserstoff, R_{V} 2,6-Dimethoxyphenyl, R Wasserstoff und Z Hydroxy bedeutet und X, X' und das Kohlenstoffatom, mit dem sie verbunden sind, eine Adamantylidengruppe bilden.

7. Verfahren zur Herstellung der Verbindungen der Formeln (I) und (I') nach Anspruch 1, dadurch gekennzeichnet, daß ein funktionelles Derivat einer Pyrazolcarbonsäure der Formel (II) oder (II') 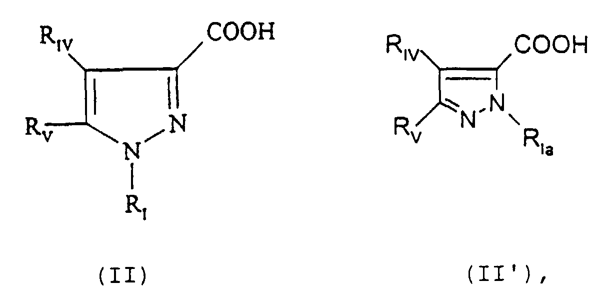 worin R_{I}, R_{IV}, R_{V} und R_{Ia} wie weiter oben definiert sind, mit einer Aminosäure, die gegebenenfalls mit bei der Peptidsynthese üblichen Schutzgruppen geschützt ist, der Formel 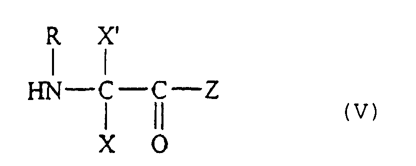 umgesetzt wird, in der R, X, X' und Z wie weiter oben definiert sind, und daß die so hergestellte Verbindung gegebenenfalls in eines ihrer Salze umgewandelt wird.

8. Pharmazeutische Zusammensetzung, die als Wirkstoff eine Verbindung nach einem der Ansprüche 1 bis 6 oder eines ihrer möglichen, pharmazeutisch akzeptablen Salze enthält.

9. Pharmazeutische Zusammensetzung nach Anspruch 8 in Form einer Dosiereinheit.

10. Pharmazeutische Zusammensetzung nach Anspruch 9, dadurch gekennzeichnet, daß sie 0,25 bis 250 mg Wirkstoff im Gemisch mit mindestens einem pharmazeutischen Excipiens enthält.

11. N,N-Diethyl-2-{[1-phenyl-5-(2-naphthyl)-3-pyrazol]-carbonylamino}-3-phenylpropanamid.

12. - 2-{[1-(1-Naphthyl)-5-(2,6-dimethoxyphenyl)-3-pyrazolyl]-carbonylamino}-bicyclo[2,2,1]-2-heptancarbonsäure;
- 2-{[1-(1-Naphthyl)-5-(2,6-dimethoxyphenyl)-3-pyrazolyl]-carbonylamino}-bicyclo[3,3,1]-9-nonancarbonsäure;
- 2-{[1-(1-Naphthyl)-5-(2,6-dimethoxyphenyl)-3-pyrazolyl]-carbonylamino}-bicyclo[3,2,1]-2-octancarbonsäure;
- Natriumsalz der 2(S)-{[5-(2-Naphthyl)-1-phenyl-3-pyrazolyl]-carbonylamino}-3-(acetamidomethylthio)-propansäure,
- 2-{[1-(2,3-Dihydro-8-phthalazinyl-1,4-dion)-5-(2,6-dimethoxyphenyl)-3-pyrazolyl]-carbonylamino}-2-adamantancarbonsäure.

## Patentansprüche (Patentansprüche für folgende(n) Vertragsstaat(en): ES)

1. Verfahren zur Herstellung eines 3-Amidopyrazols der Formel (I) oder (I') 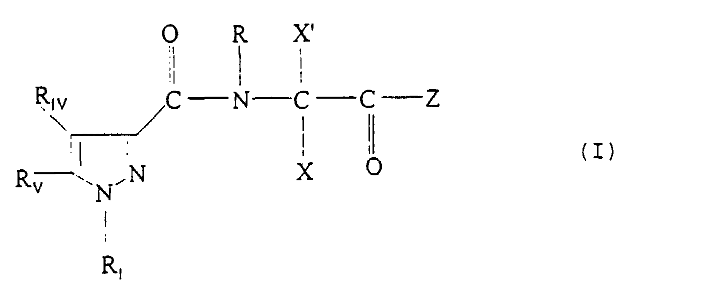 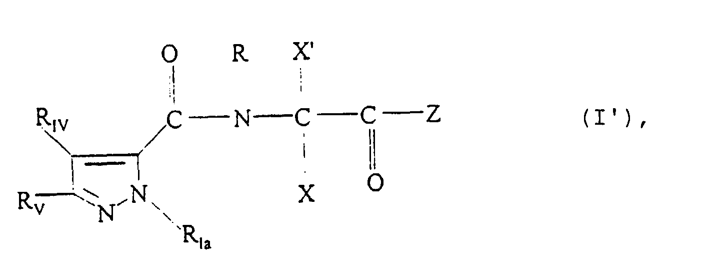 worin bedeuten:
- R_{I}
. eine Gruppe 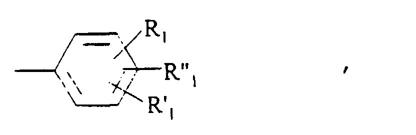 worin R₁, R'₁ und R"₁ unabhängig Wasserstoff, ein Halogenatom, Hydroxy, geradkettiges oder verzweigtes C₁-C₄-Alkyl, C₁-C₄-Alkoxy, Trifluormethyl, Trifluormethoxy, Nitro, Carboxy, Amino bedeuten,
. Carboxyalkyl oder Alkoxycarbonylalkyl, worin Alkyl C₁-C₄-Alkyl bedeutet,
. Cycloalkyl, worin Alkyl C₃-C₆-Alkyl bedeutet,
. Tetrahydronaphtyl,
. Pyridyl,
. Naphtyl, das mit R₁, R'₁ und R"₁, die wie weiter oben definiert sind, substituiert ist,
. Benzyl, das mit R₁, R'₁ und R"₁, die wie weiter oben definiert sind, substituiert ist,
. Cinnamyl, das gegebenenfalls am aromatischen Ring mit einem Halogen, Hydroxy, C₁-C₄-Alkoxy substituiert ist,
. Chinolyl oder Isochinolyl, das gegebenenfalls mit R₁, R'₁ und R"₁, die wie weiter oben definiert sind, substituiert ist,
. 2-Benzothiazolyl,
. Chinoxalinyldion,
. 1-Phthalazinyl,
. Benzothiadiazolyl,
. Methylen, das mit einer heterocyclischen Gruppe substituiert ist, die unter Pyridyl und Thienyl ausgewählt ist,
- R_{Ia} Benzyl, das mit R₁, R'₁ und R"₁, die wie weiter oben definiert sind, substituiert ist,
- R_{IV} Wasserstoff, ein Halogenatom, C₁-C₆-Alkyl,
- R_{V}
. eine Gruppe 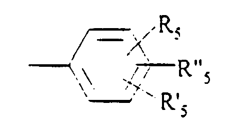 worin R₅, R'₅ und R"₅ unabhängig Wasserstoff, ein Halogenatom, geradkettiges oder verzweigtes C₁-C₄-Alkyl, Hydroxy, C₁-C₄-Alkoxy, Nitro, Trifluormethyl, Trifluormethoxy,Cyano, Amino, Carboxy, C₁-C₄-Carboxyalkyl, Phenyl bedeuten,
. Naphtyl, das nicht substituiert ist oder mit C₁-C₄-Alkyl substituiert ist,
. Pyridyl,
. Styryl, das nicht substituiert ist oder mit C₁-C₄-Alkyl substituiert ist,
- oder R_{IV} und R_{V} zusammen
. eine Gruppe 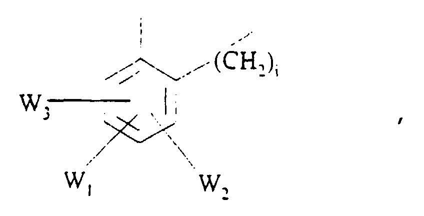 wobei der Phenylrest den Pyrazolring in 5-Stellung substituiert, die Gruppe -(CH₂)ᵢ-, in der i 1 bis 3 bedeutet, den Pyrazolring in 4-Stellung substituiert und die Reste W₁, W₂ und W₃ Substituenten am Benzolring sind und unabhängig Wasserstoff, Halogen oder Hydroxy bedeuten,
- R Wasserstoff, geradkettiges oder verzweigtes C₁-C₄-Alkyl,
- Z Hydroxy, C₁-C₆-Alkoxy, ein Sauerstoffatom, das mit einer Gruppe substituiert ist, die unter tert.-Butyl, Benzyl, Benzyl, das mit einem Halogen, C₁-C₆-Alkyl, Trifluormethyl, Trifluormethoxy, Carboxy substituiert ist, ausgewählt ist, Amino,
- X Wasserstoff und X' Wasserstoff, geradkettiges oder verzweigtes C₁-C₆-Alkyl, Phenyl, C₁-C₄-Aminoalkyl, C₁-C₄-Hydroxyalkyl, Carboxyalkyl, worin Alkyl C₁-C₄-Alkyl bedeutet, Guanidinoalkyl, worin Alkyl C₁-C₄-Alkyl bedeutet, Nitroguanidinoalkyl, worin Alkyl C₁-C₄-Alkyl bedeutet, C₃-C₇-Cycloalkyl, Phenylalkyl, worin Alkyl C₁-C₄-Alkyl bedeutet und der Phenylrest gegebenenfalls mit einem Halogen, einer Hydroxygruppe oder C₁-C₃-Alkyl substituiert ist, Heteroarylalkyl, worin Heteroaryl ein Imidazolyl, ein Indolyl, das nicht substituiert ist oder mit C₁-C₄-Alkyl, einer Hydroxygruppe oder C₁-C₄-Alkoxy substituiert ist, darstellt und Alkyl C₁-C₄-Alkyl bedeutet, Adamantyl, Adamantyl, das mit ein oder zwei Methylgruppen, Hydroxy, C₁-C₃-Alkoxy, einem Halogenatom substituiert ist, 1-Azaadamantyl, oder
- X Wasserstoff und X' und R zusammen mit dem Stickstoffatom, mit dem der Rest R verbunden ist, und mit dem Kohlenstoffatom, mit dem X verbunden ist, einen Ring, der nicht substituiert ist oder mit einer Hydroxygruppe substituiert ist, der Formel 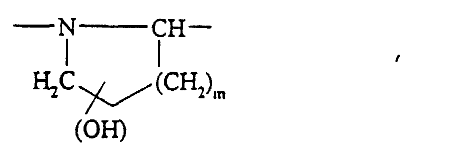 worin m 0,1 oder 2 bedeutet,
oder einen Ring der Formel 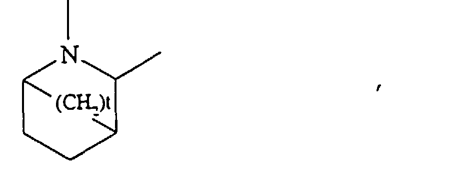 worin t 1 oder 2 bedeutet,
oder einen Ring der Formel 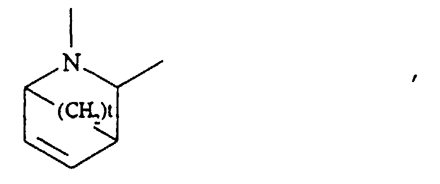 worin t 1 oder 2 bedeutet,
oder einen Indolinylring, Perhydroindolring, 4,5,6,7-Tetrahydrothieno[2,3-c]-6-pyridyl-Ring, oder
- X und X' unabhängig C₁-C₄-Alkyl, C₃-C₆-Cycloalkyl, Phenyl, oder
- X, X' und das Kohlenstoffatom, mit dem sie verbunden sind, eine Cycloalkylidengruppe mit maximal 12 Kohlenstoffatomen, die gegebenenfalls mit C₁-C₃-Alkyl substituiert ist, Adamantyliden, Adamantyliden, das mit ein oder zwei Methylgruppen, Hydroxy, C₁-C₃-Alkoxy, einem Halogenatom substituiert ist, 1-Azaadamantyliden, Chinuclidinyliden, 4-Piperidinyliden, das gegebenenfalls am N-Atom mit Benzyl substituiert ist, 2,2,6,6-Tetramethylpiperidinyliden, Tetrahydronaphthyliden, 4-Tetrahydropyranyliden oder 4-Tetrahydrothiopyranyliden, (4H)-2,3-Dihydro-4-benzopyranyliden, (4H)-2,3-Dihydro-4-benzothiopyranyliden, Bicyclo[2,2,1]-5-hepten-2-yliden, 8-Oxabicyclo[3,2,1]-6-octen-3-yliden, 8-Thiabicyclo[3,2,1]-3-octanyliden,
oder eines seiner möglichen Salze mit organischen oder anorganischen Säuren oder mit anorganischen oder organischen Basen,
dadurch gekennzeichnet, daß
ein funktionelles Derivat einer Pyrazolcarbonsäure der Formel (II) oder (II') 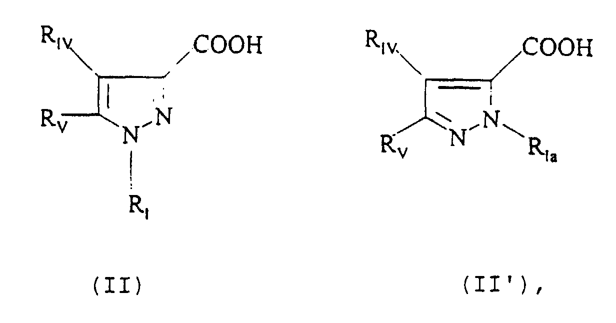 worin R_{I}, R_{IV}, R_{V} und R_{Ia} wie weiter oben definiert sind, mit einer Aminosäure, die gegebenenfalls mit bei der Peptidsynthese üblichen Schutzgruppen geschützt ist, der Formel 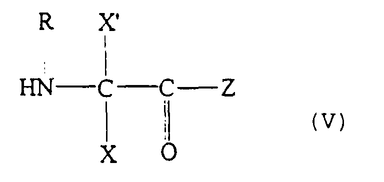 umgesetzt wird, in der R, X, X' und Z wie weiter oben definiert sind, und daß die so hergestellte Verbindung gegebenenfalls in eines ihrer Salze umgewandelt wird.

2. Verfahren zur Herstellung eines 3-Amidopyrazols der Formel (I) nach Anspruch 1, dadurch gekennzeichnet, daß eine Verbindung der Formel (II), in der R₁ Phenyl oder Naphthyl bedeutet, das mit R₁, R'₁ und R"₁, die wie in Anspruch 1 definiert sind, substituiert ist, und R_{IV} und R_{V} wie in Anspruch 1 definiert sind, mit einer Verbindung der Formel (V), in der R, X, X' und Z wie in Anspruch 1 definiert sind, umgesetzt wird und daß die erhaltene Verbindung gegebenenfalls in eines ihrer möglichen Salze mit anorganischen oder organischen Säuren oder mit organischen oder anorganischen Basen umgewandelt wird.

3. Verfahren zur Herstellung eines 3-Amidopyrazols der Formel (I) oder (I') nach Anspruch 1, dadurch gekennzeichnet, daß eine Verbindung der Formel (II) oder (II'), in der R₅ Phenyl oder Naphtyl bedeutet, das mit R₅, R'₅ und R"₅, die wie in Anspruch 1 definiert sind, substituiert ist, wobei es sich bei R₅, R'₅ und R"₅ vorzugsweise um Wasserstoff oder C₁-C₄-Alkoxy handelt, und R_{I} oder R_{Ia} und R_{IV} wie in Anspruch 1 definiert sind, mit einer Verbindung der Formel (V), in der R, X, X' und Z wie in Anspruch 1 definiert sind, umgesetzt wird, und daß die erhaltene Verbindung gegebenenfalls in eines ihrer möglichen Salze mit anorganischen oder organischen Säuren oder mit organischen oder anorganischen Basen umgewandelt wird.

4. Verfahren zur Herstellung eines 3-Amidopyrazols der Formel (I) oder (I') nach Anspruch 1, dadurch gekennzeichnet, daß eine Verbindung der Formel (V) verwendet wird, in der X, X' und das Kohlenstoffatom, mit dem sie verbunden sind, eine Adamantylidengruppe bilden.

5. Verfahren nach Anspruch 4 zur Herstellung eines 3-Amidopyrazols der Formel 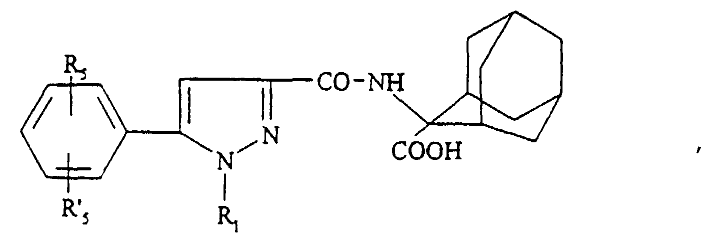 dadurch gekennzeichnet, daß eine Verbindung der Formel (II), in der R_{IV} Wasserstoff, R_{V} Phenyl, das mit R₅ und R'₅ substituiert ist, die 2-Methoxy bzw. 6-Methoxy darstellen, und R_{I} 1-Naphthyl, 4-Chlor-1-naphthyl, 5,6,7,8-Tetrahydro-1-naphthyl, 5-Isochinolyl, 8-Chinolyl, 7-Chlor-4,2,1,3-chinolyl-4-benzothiadiazolyl bedeutet, mit einer Verbindung der Formel (V) umgesetzt wird, in der R Wasserstoff bedeutet und X, X' und das Kohlenstoffatom, mit dem sie verbunden sind, eine Adamantylidengruppe bilden und Z eine Hydroxygruppe darstellt.

6. Verfahren zur Herstellung eines 3-Amidopyrazols der Formel (I) nach Anspruch 1, dadurch gekennzeichnet, daß eine Verbindung der Formel (II), in der R₁ 7-Chlor-4-chinolyl, R_{IV} Wasserstoff und R_{V} 2,6-Dimethoxyphenyl darstellt, mit einer Verbindung der Formel (V) umgesetzt wird, in der R Wasserstoff und Z Hydroxy bedeutet und X, X' und das Kohlenstoffatom, mit dem sie verbunden sind, eine Adamantylidengruppe bilden.

7. Verfahren zur Herstellung einer pharmazeutischen Zusammensetzung, dadurch gekennzeichnet, daß es darin besteht, eine nach dem Verfahren nach einem der Ansprüche 1 bis 6 hergestellte Verbindung oder eines ihrer möglichen pharmazutisch akzeptablen Salze mit mindestens einem pharmazeutischen Excipiens zu vermischen.

8. Verfahren zur Herstellung einer pharmazeutischen Zusammensetzung nach Anspruch 7, dadurch gekennzeichnet, daß die Zusammensetzung in eine Dosiereinheit umgewandelt wird.

9. Verfahren zur Herstellung einer pharmazeutischen Zusammensetzung nach Anspruch 8, dadurch gekennzeichnet, daß es darin besteht, 0,25 bis 250 mg Wirkstoff mit mindestens einem pharmazeutischen Excipiens zu vermischen.

10. Verfahren zur Herstellung des N,N-Diethyl-2-{[1-phenyl-5-(2-naphthyl)-3-pyrazol]-carbonylamino}-3-phenylpropanamids, dadurch gekennzeichnet, daß ein funktionelles Derivat einer Pyrazolcarbonsäure der Formel (II) 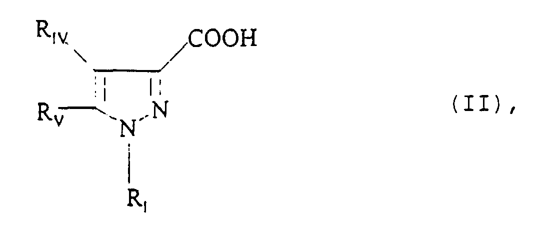 in der R₁ C₆H₅, R_{IV} H und R_{V} 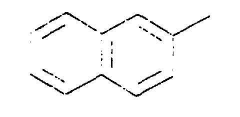 bedeutet,
mit einer Aminosäure, die gegebenenfalls mit bei der Peptidsynthese üblichen Schutzgruppen geschützt ist, der Formel 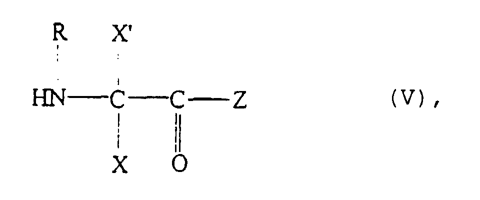 in der R H, X' H, X -CH₂-C₆H₅ und Z -N-(C₂H₅)₂ bedeutet, umgesetzt wird.

11. Verfahren zur Herstellung der 2-{[1-(1-Naphthyl)-5-(2,6-dimethoxyphenyl)-3-pyrazolyl]-carbonylamino}-bicyclo[2,2,1]-2-heptancarbonsäure, dadurch gekennzeichnet, daß ein funktionelles Derivat einer Pyrazolcarbonsäure der Formel (II) 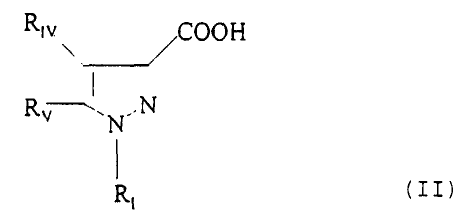 in der R₁ 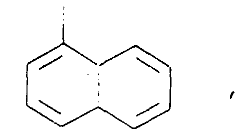 R_{IV} H und R_{V} 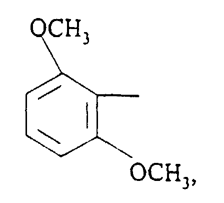 bedeutet,
mit einer Aminosäure, die gegebenenfalls mit bei der Peptidsynthese üblichen Schutzgruppen geschützt ist, der Formel 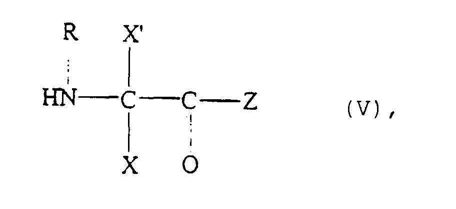 in der R Wasserstoff bedeutet und X, X' und das Kohlenstoffatom, mit dem sie verbunden sind, den Ring 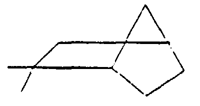 bilden und Z OH bedeutet, umgesetzt wird.

12. Verfahren zur Herstellung der 2-{[1-(1-Naphthyl)-5-(2,6-dimethoxyphenyl)-3-pyrazolyl]-carbonylamino}-bicyclo[3,3,1]-9-nonancarbonsäure, dadurch gekennzeichnet, daß ein funktionelles Derivat einer Pyrazolcarbonsäure der Formel (II) 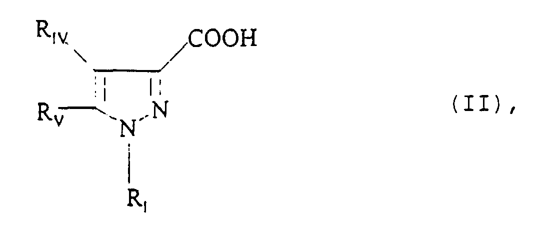 in der R₁ 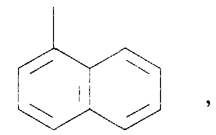 R_{IV} H und R₅ 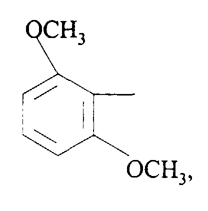 bedeutet,
mit einer Aminosäure, die gegebenenfalls mit den bei der Peptidsynthese üblichen Schutzgruppen geschützt ist, der Formel 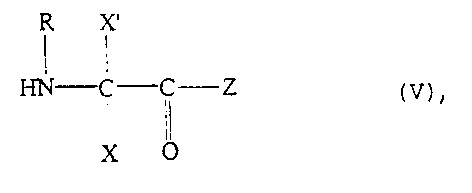 in der R H bedeutet und X', X und das Kohlenstoffatom, mitdem sie verbunden sind, den Ring 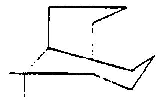 bilden, und Z OH bedeutet, umgesetzt wird.

13. Verfahren zur Herstellung der 2-{[1-(1-Naphthyl)-5-(2,6-dimethoxyphenyl)-3-pyrazolyl]-carbonylamino}-bicyclo[3,2,1]-2-octancarbonsäure, dadurch gekennzeichnet, daß ein funktionelles Derivat einer Pyrazolcarbonsäure der Formel (II) 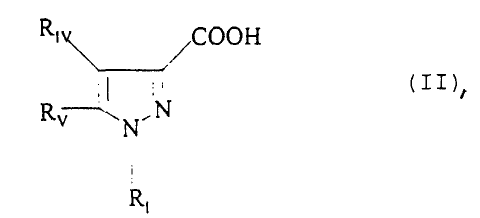 in der R₁ 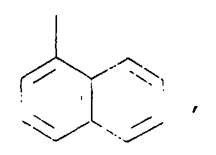 R_{IV} H und R₅ 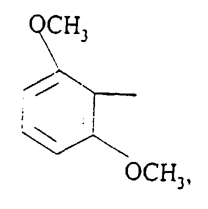 bedeutet,
mit einer Aminosäure, die gegebenenfalls mit bei der Peptidsynthese üblichen Schutzgruppen geschützt ist, der Formel 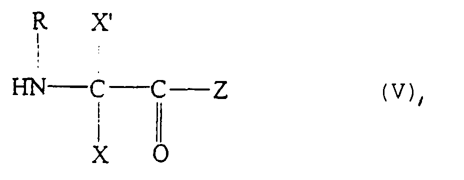 in der R H bedeutet und X', X und das Kohlenstoffatom, mit dem sie verbunden sind, den Ring 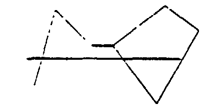 bilden, und Z OH bedeutet, umgesetzt wird.

14. Verfahren zur Herstellung des Natriumsalzes der 2(S)-{[5-(2-Naphthyl)-1-phenyl-3-pyrazolyl]-carbonylamino}-3-(acetamidomethylthio)-propansäure, dadurch gekennzeichnet, daß ein funktionelles Derivat einer Pyrazolcarbonsäure der Formel (II) 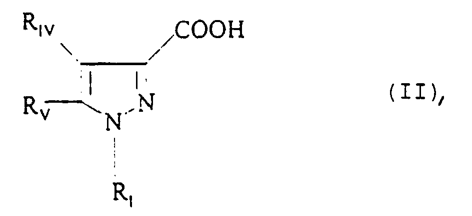 in der R₁ Phenyl, R_{IV} Wasserstoff und R_{V} Naphthyl in 2-Stellung bedeutet, mit einer Aminosäure, die gegebenenfalls mit bei der Peptidsynthese üblichen Schutzgruppen geschützt ist, der Formel 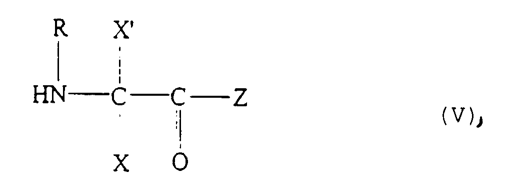 in der R Wasserstoff, X Wasserstoff bedeutet und X' ein Acetamidomethylthiomethyl ist und Z OH bedeutet, umgesetzt wird und die Verbindung in das Natriumsalz umgewandelt wird.

15. Verfahren zur Herstellung der 2-{[1-(2,3-Dihydro-8-phthalazinyl-1,4-dion)-5-(2,6-dimethoxyphenyl)-3-pyrazolyl]-carbonylamino}-2-adamantancarbonsäure, dadurch gekennzeichnet, daß ein funktionelles Derivat einer Pyrazolcarbonsäure der Formel (II) 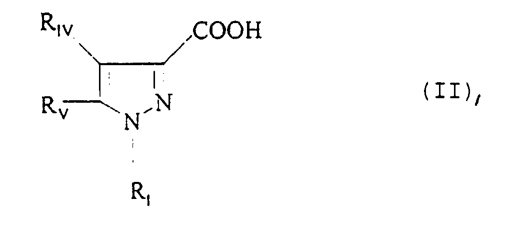 in der R₁ 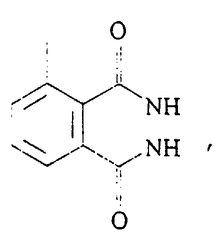 R_{IV} Wasserstoff und R_{V} 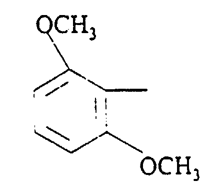 bedeutet, mit einer Aminosäure, die gegebenenfalls mit bei der Peptidsynthese üblichen Schutzgruppen geschützt ist, der Formel 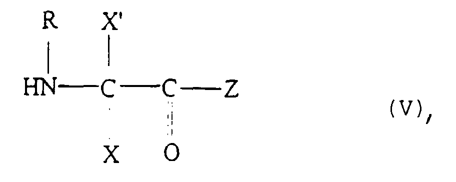 in der R Wasserstoff bedeutet und X, X' und das Kohlenstoffatom, mit dem sie verbunden sind, den Ring 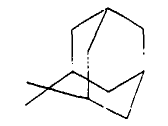 bilden und Z OH bedeutet, umgesetzt wird.

## Patentansprüche (Patentansprüche für folgende(n) Vertragsstaat(en): GR)

1. 3-Amidopyrazol der Formel (I) oder (I') 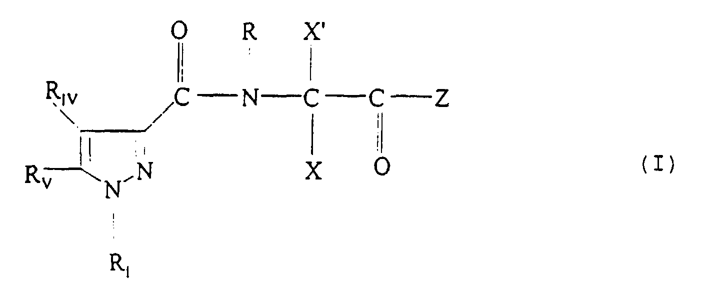 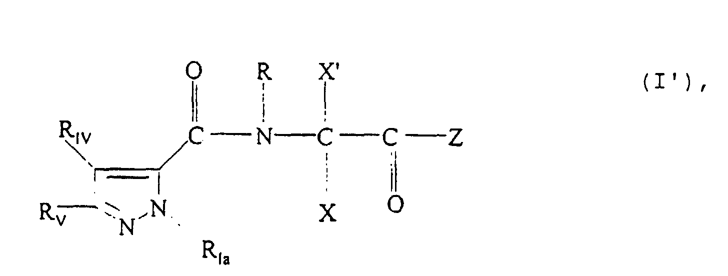 worin bedeuten:
- R_{I}
. eine Gruppe 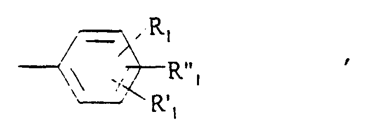 worin R₁, R'₁ und R"₁ unabhängig Wasserstoff, ein Halogenatom, Hydroxy, geradkettiges oder verzweigtes C₁-C₄-Alkyl, C₁-C₄-Alkoxy, Trifluormethyl, Trifluormethoxy, Nitro, Carboxy, Amino bedeuten,
. Carboxyalkyl oder Alkoxycarbonylalkyl, worin Alkyl C₁-C₄-Alkyl bedeutet,
. Cycloalkyl, worin Alkyl C₃-C₆-Alkyl bedeutet,
. Tetrahydronaphthyl,
. Pyridyl,
. Naphthyl, das mit R₁, R'₁ und R"₁, die wie weiter oben definiert sind, substituiert ist,
. Benzyl, das mit R₁, R'₁ und R"₁, die wie weiter oben definiert sind, substituiert ist,
. Cinnamyl, das gegebenenfalls am aromatischen Ring mit einem Halogen, Hydroxy, C₁-C₄-Alkoxy substituiert ist,
. Chinolyl oder Isochinolyl, das gegebenenfalls mit R₁, R'₁ und R"₁, die wie weiter oben definiert sind, substituiert ist,
. 2-Benzothiazolyl,
. Chinoxalinyldion,
. 1-Phthalazinyl,
. Benzothiadiazolyl,
. Methylen, das mit einer heterocyclischen Gruppe substituiert ist, die unter Pyridyl und Thienyl ausgewählt ist,
- R_{Ia} Benzyl, das mit R₁, R'₁ und R"₁, die wie weiter oben definiert sind, substituiert ist,
- R_{IV} Wasserstoff, ein Halogenatom, C₁-C₆-Alkyl,
- R_{V}
. eine Gruppe 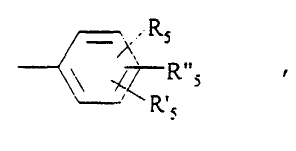 worin R₅, R'₅ und R"₅ unabhängig Wasserstoff, ein Halogenatom, geradkettiges oder verzweigtes C₁-C₄-Alkyl, Hydroxy, C₁-C₄-Alkoxy, Nitro, Trifluormethyl, Trifluormethoxy,Cyano, Amino, Carboxy, C₁-C₄-Carboxyalkyl, Phenyl bedeuten,
. Naphthyl, das nicht substituiert ist oder mit C₁-C₄-Alkyl substituiert ist,
. Pyridyl,
. Styryl, das nicht substituiert ist oder mit C₁-C₄-Alkyl substituiert ist,
- oder R_{IV} und R_{V} zusammen
. eine Gruppe 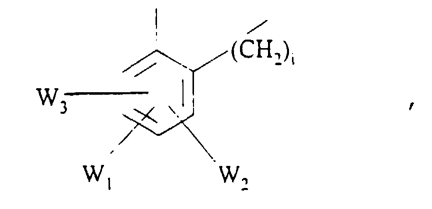 wobei der Phenylrest den Pyrazolring in 5-Stellung substituiert, die Gruppe -(CH₂)ᵢ-, in der i 1 bis 3 bedeutet, den Pyrazolring in 4-Stellung substituiert und die Reste W₁, W₂ und W₃ Substituenten am Benzolring sind und unabhängig Wasserstoff, Halogen oder Hydroxy bedeuten,
- R Wasserstoff, geradkettiges oder verzweigtes C₁-C₄-Alkyl,
- Z Hydroxy, C₁-C₆-Alkoxy, ein Sauerstoffatom, das mit einer Gruppe substituiert ist, die unter tert.-Butyl, Benzyl, Benzyl, das mit einem Halogen, C₁-C₆-Alkyl, Trifluormethyl, Trifluormethoxy, Carboxy substituiert ist, ausgewählt ist, Amino,
- X Wasserstoff und X' Wasserstoff, geradkettiges oder verzweigtes C₁-C₆-Alkyl, Phenyl, C₁-C₄-Aminoalkyl, C₁-C₄-Hydroxyalkyl, Carboxyalkyl, worin Alkyl C₁-C₄-Alkyl bedeutet, Guanidinoalkyl, worin Alkyl C₁-C₄-Alkyl bedeutet, Nitroguanidinoalkyl, worin Alkyl C₁-C₄-Alkyl bedeutet, C₃-C₇-Cycloalkyl, Phenylalkyl, worin Alkyl C₁-C₄-Alkyl bedeutet und der Phenylrest gegebenenfalls mit einem Halogen, einer Hydroxygruppe oder C₁-C₃-Alkyl substituiert ist, Heteroarylalkyl, worin Heteroaryl ein Imidazolyl, Indolyl das nicht substituiert ist oder mit C₁-C₄-Alkyl, einer Hydroxygruppe oder C₁-C₄-Alkoxy substituiert ist, darstellt und Alkyl C₁-C₄-Alkyl bedeutet, Adamantyl, Adamantyl, das mit ein oder zwei Methylgruppen, Hydroxy, C₁-C₃-Alkoxy, einem Halogenatom substituiert ist, 1-Azaadamantyl, oder
- X Wasserstoff und X' und R zusammen mit dem Stickstoffatom, mit dem der Rest R verbunden ist, und mit dem Kohlenstoffatom, mit dem X verbunden ist, einen Ring, der nicht substituiert ist oder mit einer Hydroxygruppe substituiert ist, der Formel 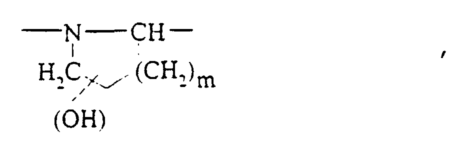 worin m 0,1 oder 2 bedeutet,
oder einen Ring der Formel 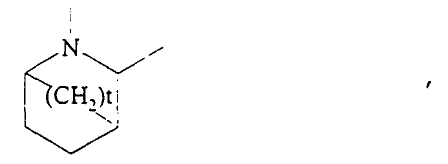 worin t 1 oder 2 bedeutet,
oder einen Ring der Formel 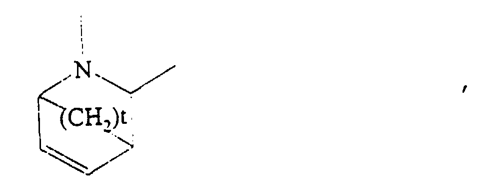 worin t 1 oder 2 bedeutet,
oder einen Indolinylring, Perhydroindolring, 4,5,6,7-Tetrahydrothieno[2,3-c]-6-pyridyl-Ring, oder
- X und X' unabhängig C₁-C₄-Alkyl, C₃-C₆-Cycloalkyl, Phenyl, oder
- X, X' und das Kohlenstoffatom, mit dem sie verbunden sind, eine Cycloalkylidengruppe mit maximal 12 Kohlenstoffatomen, die gegebenenfalls mit C₁-C₃-Alkyl substituiert ist, Adamantyliden, Adamantyliden, das mit ein oder zwei Methylgruppen, Hydroxy, C₁-C₃-Alkoxy, einem Halogenatom substituiert ist, 1-Azaadamantyliden, Chinuclidinyliden, 4-Piperidinyliden, das gegebenenfalls am N-Atom mit Benzyl substituiert ist, 2,2,6,6-Tetramethylpiperidinyliden, Tetrahydronaphthyliden, 4-Tetrahydropyranyliden oder 4-Tetrahydrothiopyranyliden, (4H)-2,3-Dihydro-4-benzopyranyliden, (4H)-2,3-Dihydro-4-benzothiopyranyliden, Bicyclo[2,2,1]-5-hepten-2-yliden, 8-Oxabicyclo[3,2,1]-6-octen-3-yliden, 8-Thiabicyclo[3,2,1]-3-octanyliden,
oder eines seiner möglichen Salze mit organischen oder anorganischen Säuren oder mit anorganischen oder organischen Basen.

2. 3-Amidopyrazol nach Anspruch 1 der Formel (I), in der R, X, X', Z, R_{IV} und R_{V} wie in Anspruch 1 definiert sind, dadurch gekennzeichnet, daß R₁ Phenyl oder Naphthyl bedeutet, das mit R₁, R'₁ und R"₁, die wie in Anspruch 1 definiert sind, substituiert ist, oder eines seiner möglichen Salze mit anorganischen oder organischen Säuren oder mit organischen oder anorganischen Basen.

3. 3-Amidopyrazol nach Anspruch 1 der Formel (I) oder (I'), in der R, X, X', Z, R_{I} oder R_{Ia} und R_{IV} wie in Anspruch 1 definiert sind, dadurch gekennzeichnet, daß R_{V} Phenyl oder Naphthyl bedeutet, das mit R₅, R'₅ und R"₅, die wie in Anspruch 1 definiert sind, substituiert ist, wobei R₅, R'₅ und R"₅ vorzugsweise Wasserstoff oder C₁-C₄-Alkoxy bedeuten, oder eines seiner möglichen Salze mit anorganischen oder organischen Säuren oder mit organischen oder anorganischen Basen.

4. 3-Amidopyrazol nach Anspruch 1 der Formel (I) oder (I'), in der X, X' und das Kohlenstoffatom, mit dem sie verbunden sind, eine Adamantylidengruppe bilden.

5. 3-Amidopyrazol nach Anspruch 4 der Formel 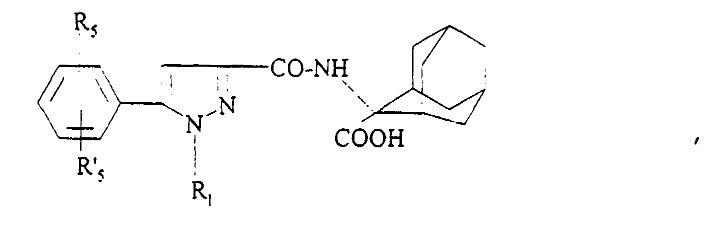 worin R_{I} 1-Naphthyl, 4-Chlor-1-naphthyl, 5,6,7,8-Tetrahydro-1-naphthyl, 5-Isochinolyl, 8-Chinolyl, 7-Chlor-4,2,1,3-chinolyl-4-benzothiadiazolyl bedeutet und R₅ und R'₅ 2-Methoxy bzw. 6-Methoxy darstellen.

6. 3-Amidopyrazol nach Anspruch 1 der Formel (I), worin R_{I} 7-Chlor-4-chinolyl, R_{IV} Wasserstoff, R_{V} 2,6-Dimethoxyphenyl, R Wasserstoff und Z Hydroxy bedeutet und X, X' und das Kohlenstoffatom, mit dem sie verbunden sind, eine Adamantylidengruppe bilden.

7. Verfahren zur Herstellung der Verbindungen der Formeln (I) und (I') nach Anspruch 1, dadurch gekennzeichnet, daß ein funktionelles Derivat einer Pyrazolcarbonsäure der Formel (II) oder (II') 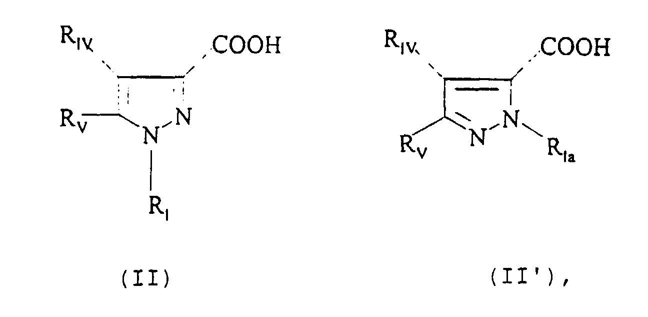 worin R_{I}, R_{IV}, R_{V} und R_{Ia} wie weiter oben definiert sind, mit einer Aminosäure, die gegebenenfalls mit bei der Peptidsynthese üblichen Schutzgruppen geschützt ist, der Formel 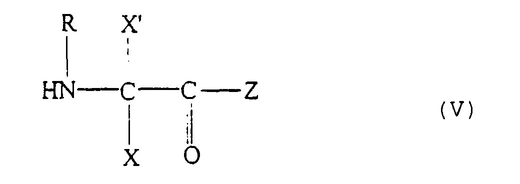 umgesetzt wird, in der R, X, X' und Z wie weiter oben definiert sind, und daß die so hergestellte Verbindung gegebenenfalls in eines ihrer Salze umgewandelt wird.

8. Verfahren zur Herstellung einer pharmazeutischen Zusammensetzung, dadurch gekennzeichnet, daß es darin besteht, als Wirkstoff eine Verbindung nach einem der Ansprüche 1 bis 6 oder eines ihrer möglichen pharmazeutisch akzeptablen Salze mit mindestens einem pharmazeutischen Excipiens zu vermischen.

9. Verfahren zur Herstellung einer pharmazeutischen Zusammensetzung nach Anspruch 8, dadurch gekennzeichnet, daß die Zusammensetzung in eine Dosiereinheit umgewandelt wird.

10. Verfahren zur Herstellung einer pharmazeutischen Zusammensetzung nach Anspruch 9, dadurch gekennzeichnet, daß es darin besteht, 0,25 bis 250 mg Wirkstoff mit mindestens einem pharmazeutischen Excipiens zu vermischen.

11. N,N-Diethyl-2-{[1-phenyl-5-(2-naphthyl)-3-pyrazol]-carbonylamino}-3-phenylpropanamid.

12. - 2-{[1-(1-Naphthyl)-5-(2,6-dimethoxyphenyl)-3-pyrazolyl]-carbonylamino}-bicyclo[2,2,1]-2-heptancarbonsäure;
- 2-{[1-(1-Naphthyl)-5-(2,6-dimethoxyphenyl)-3-pyrazolyl]-carbonylamino}-bicyclo[3,3,1]-9-nonancarbonsäure;
- 2-{[1-(1-Naphthyl)-5-(2,6-dimethoxyphenyl)-3-pyrazolyl]-carbonylamino}-bicyclo[3,2,1]-2-octancarbonsäure;
- Natriumsalz der 2(S)-{[5-(2-Naphthyl)-1-phenyl-3-pyrazolyl]-carbonylamino}-3-(acetamidomethylthio)-propansäure,
- 2-{[1-(2,3-Dihydro-8-phthalazinyl-1,4-dion)-5-(2,6-dimethoxyphenyl)-3-pyrazolyl]-carbonylamino}-2-adamantancarbonsäure.

## Claims (Claims for the following Contracting State(s): AT, BE, CH, LI, DE, DK, FR, GB, IT, LU, NL, SE)

1. 3-Amidopyrazole of formula (I) or (I'): 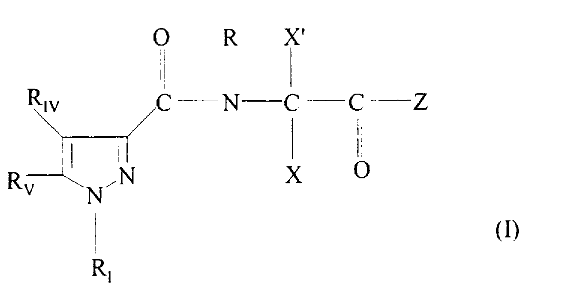 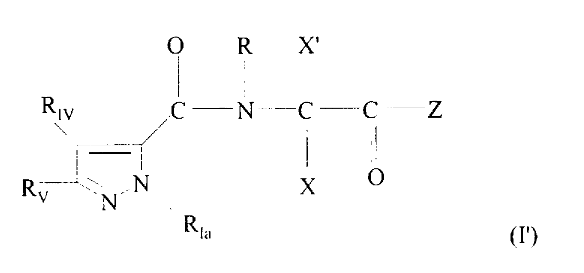 in which
- R_{I} represents
. a group 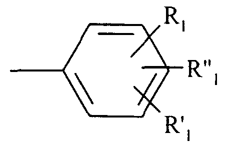 where R₁, R'₁ and R"₁ each independently represent a hydrogen atom, a halogen atom, a hydroxyl, a linear or branched C₁-C₄ alkyl group, a C₁-C₄ alkoxy group, a trifluoromethyl group, a trifluoromethoxy group, a nitro group, a carboxyl group, an amino group;
. a carboxyalkyl or alkoxycarbonylalkyl group in which the alkyls are C₁-C₄ groups;
. a cycloalkyl group in which the alkyl are C₃-C₆ groups;
. a tetrahydronaphthyl group;
. a pyridyl group;
. a naphthyl substituted with R₁, R'₁ and R"₁ as defined above;
. a benzyl group substituted with R₁, R'₁ and R"₁ as defined above;
. a cinnamyl group optionally substituted on the aromatic ring with a halogen, a hydroxyl or a C₁-C₄ alkoxy;
. a quinolyl or isoquinolyl group, optionally substituted with R₁, R'₁ and R"₁ as defined above;
. a 2-benzothiazolyl group;
. a quinoxalinyldione group;
. a 1-phthalazinyl group ;
. a benzothiadiazolyl group ;
. a methylene group substituted with a heterocyclic group selected from the pyridyl and thienyl groups ;
- R_{Ia} represents a benzyl group substituted with R₁, R'₁ and R"₁ as defined above ;
- R_{IV} represents a hydrogen atom, a halogen atom, a C₁-C₆ alkyl;
- R_{V} represents :
. a group 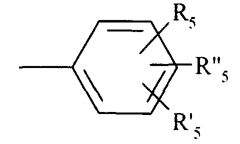
- where R₅, R'₅ and R"₅ each independently represent a hydrogen atom, a halogen atom, a linear or branched C₁-C₄ alkyl, a hydroxyl, a C₁-C₄ alkoxy, a nitro, a trifluoromethyl, a trifluoromethoxy, a cyano, an amino, a carboxyl, a C₁-C₄ carboxyalkyl, a phenyl;
. a naphthyl group unsubstituted or substituted with a C₁-C₄ alkyl;
. a pyridyl group;
. a styryl group unsubstituted or substituted with a C₁-C₄ alkyl;
- or alternatively R_{IV} and R_{V} considered together represent:
. a group 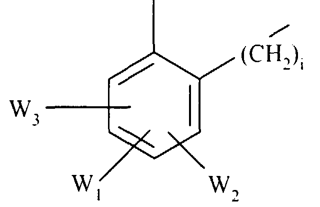 in which the phenyl group substitutes the pyrazole at position 5 and the group -(CH₂)ᵢ- in which i = 1 to 3 substitutes the pyrazole at position 4, W₁, W₂ and W₃ substitute the benzene cycle and independently represent hydrogen, a halogen or a hydroxyl group;
- R represents hydrogen, a linear or branched C₁-C₄ alkyl;
- Z represents a hydroxyl group, a C₁-C₆ alkoxy group ; an oxygen atom substituted with a group selected from the groups : tertio-butyl, benzyl, benzyl substituted with a halogen atom, C₁-C₆ alkyl, trifluoromethyl, trifluoromethoxy and carboxyl; an amino group ;
- either X represents hydrogen and X' represents hydrogen, a linear or branched C₁-C₆ alkyl; a phenyl ; a C₁-C₄ aminoalkyl; a C₁-C₄ hydroxyalkyl; a carboxyalkyl in which the alkyl group is a C₁-C₄ group; a guanidinoalkyl in which the alkyl group is a C₁-C₄ group; a nitroguanidinoalkyl in which the alkyl group is a C₁-C₄ group; a C₃-C₇ cycloalkyl; a phenylalkyl in which the alkyl is a C₁-C₄ group and in which the phenyl is optionally substituted with a halogen, a hydroxyl or with a C₁-C₃ alkyl; a heteroarylalkyl in which the heteroaryl represents an imidazolyl, an indolyl unsubstituted or substituted with a C₁-C₄ alkyl; with a hydroxyl or with a C₁-C₄ alkoxy and in which the alkyl is a C₁-C₄ alkyl; an adamantyl group; an adamantyl group substituted with one or two methyls, with a hydroxyl, a C₁-C₃ alkoxy; a halogen atom ; a 1-azaadamantyl group ;
- or X represents hydrogen, X' and R considered together form with the nitrogen atom to which R is bonded and with the carbon atom to which X is bonded a cycle, unsubstituted or substituted with a hydroxyl, of formula 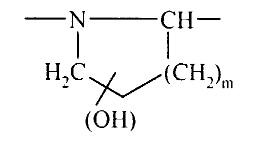 with m = 0, 1 or 2
or a cycle of formula : 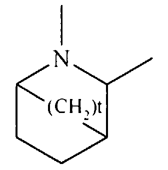 with t = 1 or 2
or a cycle of formula : 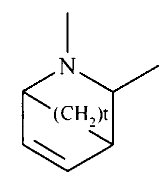 with t = 1 or 2
or an indolinyl ; perhydroindole, 4,5,6,7-tetrahydrothieno[2,3-c]pyrid-6-yl cycle;
- either X and X' each represent independently a C₁-C₄ alkyl; a C₃-C₆ cycloalkyl; a phenyl;
- or X, X' and the carbon atom to which they are bonded form a cycloalkylidene group of at most 12 carbon atoms, optionally substituted with a C₁-C₃ alkyl; an adamantylidene group; an adamantylidene group substituted with one or two methyl groups or with a hydroxyl, a C₁-C₃ alkoxy, a halogen atom ; a 1-azaadamantylidene group; a quinuclidinylidene group ; a 4-piperidinylidene group, optionally N-substituted with a benzyl group; a 2,2,6,6-tetramethylpiperidinylidene group; a tetrahydronaphthylidene group; a tetrahydropyran-4-ylidene or tetrahydrothiopyran-4-ylidene group; a 2,3-dihydro-(4H)-benzopyran-4-ylidene group; a 2,3-dihydro-(4H)-benzothiopyran-4-ylidene group ;
a bicyclo[2,2,1]hept-5-en-2-ylidene group; an 8-oxabicyclo[3,2,1]oct-6-en-3-ylidene; a 8-thiabicyclo[3,2,1]oct-3-anylidene; or one of its possible salts with organic or inorganic acids or with inorganic or organic bases.

2. 3-Amidopyrazole according to claim 1, of formula (I) in which R, X, X', Z, R_{IV} and R_{V} are as defined in claim 1, characterized in that R₁ represents a phenyl or naphthyl group substituted with R₁, R'₁ and R"₁ as defined in claim 1, or one of its possible salts with organic or inorganic acids or with organic or inorganic bases.

3. 3-Amidopyrazole according to claim 1, of formula (I) or (I') in which R, X, X', Z, R_{I}, or R_{Ia} and R_{IV} are as defined in claim 1, characterized in that R_{V} represents a phenyl or a naphthyl substituted with R₅, R'₅ and R"₅ as defined in claim 1, R₅, R'₅ and R"₅ being preferably hydrogen or a C₁-C₄ alkoxy; or one of its possible salts with inorganic or organic acids or with organic or inorganic bases.

4. 3-Amidopyrazole according to claim 1, of formula I or I', in which X, X' and the carbon atom to which they are bonded form an adamantylidene group.

5. 3-Amidopyrazole according to claim 4 of formula : 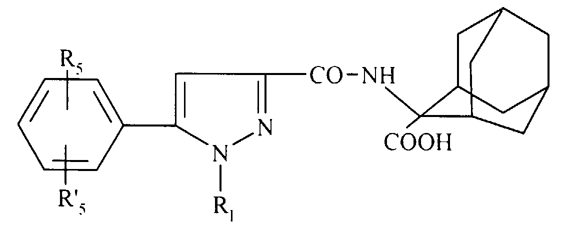 in which R_{I} is 1-naphthyl, 4-chloro-1-naphthyl, 5,6,7,8-tetrahydro-1-naphthyl, 5-isoquinolyl, 8-quinolyl, 7-chloro-4,2,1,3-quinolyl-4-benzothiadiazolyl, R₅ and R'₅ representing respectively 2- and 6-methoxy.

6. 3-Amidopyrazole according to claim 1, of formula I in which R_{I} is 7-chloro-4-quinolyl, R_{IV} is hydrogen, R_{V} is 2,6-dimethoxyphenyl, R is hydrogen, Z is hydroxyl and X, X' and the carbon atom to which they are bonded form an adamantylidene group.

7. Process for the preparation of compounds of formula (I) and (I') according to claim 1, characterized in that a functional derivative of a pyrazolecarboxylic acid of formula (II) or (II') : 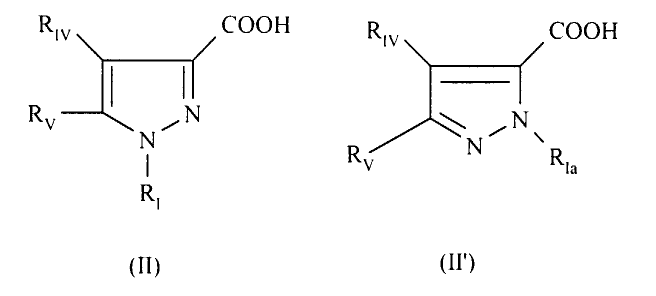 in which R_{I}, R_{IV}, R_{V} and R_{Ia} are as defined above, is treated with an amino acid, optionally protected by the protecting groups which are usual in peptide synthesis, of formula: 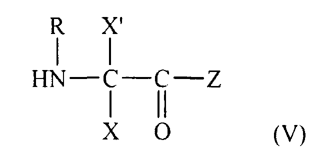 in which R, X, X' and Z are as defined above and the compound thus obtained is optionally converted into one of its salts.

8. Pharmaceutical composition containing as the active principle a compound according to any one of claims 1 to 6 or one of its possible pharmaceutically acceptable salts.

9. Pharmaceutical composition according to claim 8 in unit dosage form.

10. Pharmaceutical composition according to claim 9, characterized in that it contains from 0.25 to 250 mg of active principle in admixture with at least one pharmaceutical excipient.

11. N, N-diethyl-2-{[1-phenyl-5-(2-naphthyl)-3-pyrazol]carbonylamino}-3-phenyl-propanamide.

12. - 2-{[1-(1-naphthyl )-5-(2,6-d i methoxyphenyl )-3-pyrazolyl]carbonylamino}-bicyclo[2.2.1]-2-heptanecarboxylic acid ;
- 9-{[1-(1-naphthyl)-5-(2,6-dimethoxyphenyl)-3-pyrazolyl]-carbonylamino}-bicyclo[3.3. 1 ]-9-nonanecarboxylic acid ;
- 2-{[1-(1-naphtyl)-5-(2,6-dimethoxyphenyl)-3-pyrazolyl]-carbonylamino}-bicyclo[3.2.1]-2-octanecarboxylic acid ;
- 3-{[5-(2-naphthyl )-1-phenyl-3-pyrazolyl]carbonylamino}-2(S)(acetamidomethylthio)propanecarboxylic acid sodium salt ;
- 2-{[1-(2,3-dihydro-8-phtalazinyl-1,4-dione)-5-(2,6-dimethoxyphenyl)-3-pyrazolyl]carbonylamino}-2-adamantanecarboxylic acid.

## Claims (Claims for the following Contracting State(s): ES)

1. Process for the preparation of a 3-amidopyrazole of formula (I) or (I'): 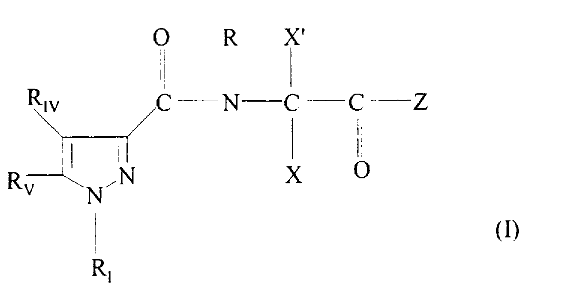 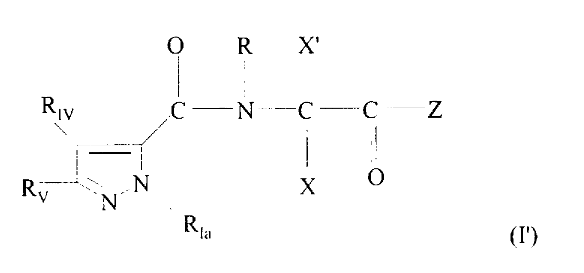 in which
- R_{I} represents
. a group 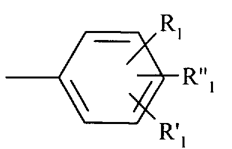 where R₁, R'₁ and R"₁ each independently represent a hydrogen atom, a halogen atom, a hydroxyl, a linear or branched C₁-C₄ alkyl group, a C₁-C₄ alkoxy group, a trifluoromethyl group, a trifluoromethoxy group, a nitro group, a carboxyl group, an amino group;
. a carboxyalkyl or alkoxycarbonylalkyl group in which the alkyls are C₁-C₄ groups;
. a cycloalkyl group in which the alkyl are C₃-C₆ groups;
. a tetrahydronaphthyl group;
. a pyridyl group;
. a naphthyl substituted with R₁, R'₁ and R"₁ as defined above;
. a benzyl group substituted with R₁, R'₁ and R"₁ as defined above;
. a cinnamyl group optionally substituted on the aromatic ring with a halogen, a hydroxyl or a C₁-C₄ alkoxy;
. a quinolyl or isoquinolyl group, optionally substituted with R₁, R'₁ and R"₁ as defined above;
. a 2-benzothiazolyl group;
. a quinoxalinyldione group;
. a 1-phthalazinyl group ;
. a benzothiadiazolyl group ;
. a methylene group substituted with a heterocyclic group selected from the pyridyl and thienyl groups ;
- R_{Ia} represents a benzyl group substituted with R₁, R'₁ and R"₁ as defined above ;
- R_{IV} represents a hydrogen atom, a halogen atom, a C₁-C₆ alkyl;
- R_{V} represents :
. a group 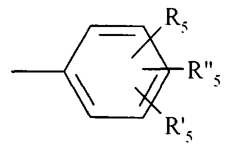
- where R₅, R'₅ and R"₅ each independently represent a hydrogen atom, a halogen atom, a linear or branched C₁-C₄ alkyl, a hydroxyl, a C₁-C₄ alkoxy, a nitro, a trifluoromethyl, a trifluoromethoxy, a cyano, an amino, a carboxyl, a C₁-C₄ carboxyalkyl, a phenyl;
. a naphthyl group unsubstituted or substituted with a C₁-C₄ alkyl;
. a pyridyl group;
. a styryl group unsubstituted or substituted with a C₁-C₄ alkyl;
- or alternatively R_{IV} and R_{V} considered together represent:
. a group 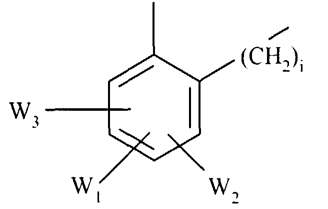 in which the phenyl group substitutes the pyrazole at position 5 and the group -(CH₂)ᵢ- in which i = 1 to 3 substitutes the pyrazole at position 4, W₁, W₂ and W₃ substitute the benzene cycle and independently represent hydrogen, a halogen or a hydroxyl group;
- R represents hydrogen, a linear or branched C₁-C₄ alkyl;
- Z represents a hydroxyl group, a C₁-C₆ alkoxy group ; an oxygen atom substituted with a group selected from the groups : tertio-butyl, benzyl, benzyl substituted with a halogen atom, C₁-C₆ alkyl, trifluoromethyl, trifluoromethoxy and carboxyl; an amino group ;
- either X represents hydrogen and X' represents hydrogen, a linear or branched C₁-C₆ alkyl; a phenyl ; a C₁-C₄ aminoalkyl; a C₁-C₄ hydroxyalkyl; a carboxyalkyl in which the alkyl group is a C₁-C₄ group; a guanidinoalkyl in which the alkyl group is a C₁-C₄ group; a nitroguanidinoalkyl in which the alkyl group is a C₁-C₄ group; a C₃-C₇ cycloalkyl; a phenylalkyl in which the alkyl is a C₁-C₄ group and in which the phenyl is optionally substituted with a halogen, a hydroxyl or with a C₁-C₃ alkyl; a heteroarylalkyl in which the heteroaryl represents an imidazolyl, an indolyl unsubstituted or substituted with a C₁-C₄ alkyl; with a hydroxyl or with a C₁-C₄ alkoxy and in which the alkyl is a C₁-C₄ alkyl; an adamantyl group; an adamantyl group substituted with one or two methyls, with a hydroxyl, a C₁-C₃ alkoxy; a halogen atom ; a 1-azaadamantyl group ;
- or X represents hydrogen, X' and R considered together form with the nitrogen atom to which R is bonded and with the carbon atom to which X is bonded a cycle, unsubstituted or substituted with a hydroxyl, of formula 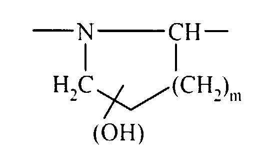 with m = 0, 1 or 2
or a cycle of formula : 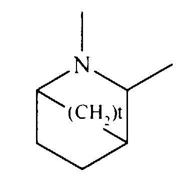 with t = 1 or 2
or a cycle of formula : 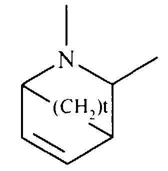 with t = 1 or 2
or an indolinyl ; perhydroindole, 4,5,6,7-tetrahydrothieno[2,3-c]pyrid-6-yl cycle;
- either X and X' each represent independently a C₁-C₄ alkyl; a C₃-C₆ cycloalkyl; a phenyl;
- or X, X' and the carbon atom to which they are bonded form a cycloalkylidene group of at most 12 carbon atoms, optionally substituted with a C₁-C₃ alkyl; an adamantylidene group; an adamantylidene group substituted with one or two methyl groups or with a hydroxyl, a C₁-C₃ alkoxy, a halogen atom ; a 1-azaadamantylidene group; a quinuclidinylidene group ; a 4-piperidinylidene group, optionally N-substituted with a benzyl group; a 2,2,6,6-tetramethylpiperidinylidene group; a tetrahydronaphthylidene group; a tetrahydropyran-4-ylidene or tetrahydrothiopyran-4-ylidene group; a 2,3-dihydro-(4H)-benzopyran-4-ylidene group; a 2,3-dihydro(4H)-benzothiopyran-4-ylidene group ;
a bicyclo[2,2,1]hept-5-en-2-ylidene group; an 8-oxabicyclo[3,2,1]oct-6-en-3-ylidene; a 8-thiabicyclo[3,2,1]oct-3-anylidene; or one of its possible salts with organic or inorganic acids or with inorganic or organic bases, characterized in that a functional derivative of a pyrazolecarboxylic acid of formula (II) or (II') : 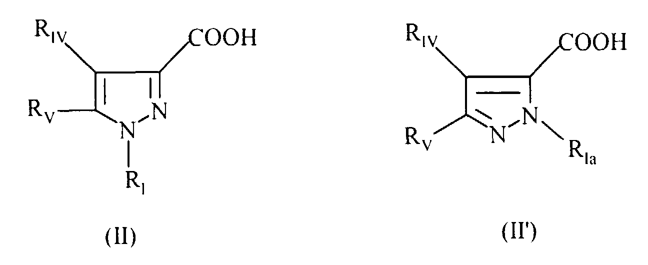 in which R_{I}, R_{IV}, R_{V} and R_{Ia} are as defined above, is treated with an amino acid, optionally protected by the protecting groups which are usual in peptide synthesis, of formula : 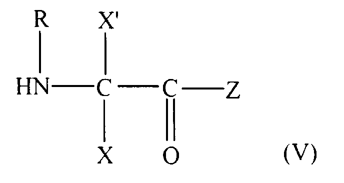 in which R, X, X' and Z are as defined above and the compound thus obtained is optionally converted into one of its salts.

2. Process for the preparation of a 3-amidopyrazole of formula (I) according to claim 1, characterized in that a compound of formula (II) in which R_{I} represents a phenyl or naphthyl group substituted with R₁, R'₁ and R"₁ as defined in claim 1, and R_{IV} and R_{V} are as defined in claim 1, is treated with a compound of formula (V) in which R, X, X' and Z are as defined in claim 1, and the compound thus obtained is optionally converted into one of its possible salts with organic or inorganic acids or with organic or inorganic bases.

3. Process for the preparation of a 3-amidopyrazole of formula (I) or (I') according to claim 1, characterized in that a compound of formula (II) or (II') in which R_{V} represents a phenyl or a naphthyl substituted with R₅, R'₅ and R"₅ as defined in claim 1, R₅, R'₅ and R"₅ being preferably hydrogen or a C₁-C₄ alkoxy, and R_{I}, or R_{Ia} and R_{IV} are as defined in claim 1, is treated with a compound of formula (V) in which R, X, X' and Z are as defined in claim 1, and the compound thus obtained is optionally converted into one of its possible salts with organic or inorganic acids or with organic or inorganic bases.

4. Process for the preparation of a 3-amidopyrazole of formula (I) or (I') according to claim 1, characterized in that a compound of formula (V) in which X, X' and the carbon atom to which they are bonded form an adamantylidene group, is used.

5. Process for the preparation according to claim 4 of a 3-amidopyrazole of formula : 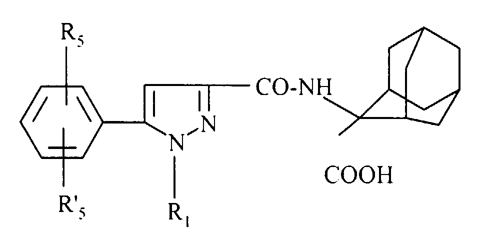 characterized in that a compound of formula (II) in which R_{IV} is a hydrogen, R_{V} is a phenyl substituted with R₅ and R'₅ representing respectively 2- and 6-methoxy, and R_{I} is 1-naphthyl, 4-chloro-1-naphthyl, 5,6,7,8-tetrahydro-1-naphthyl, 5-isoquinolyl, 8-quinolyl, 7-chloro-4,2,1,3-quinolyl-4-benzothiadiazolyl is treated with a compound of formula (V) in which R is hydrogen, X, X' and the carbon atom to which they are bonded form an adamantylidene group, and Z is a hydroxyl group.

6. Process for the preparation of a 3-amidopyrazole of formula (I) according to claim 1, characterized in that a compound of formula (II) in which R_{I} is 7-chloro-4-quinolyl, R_{IV} is hydrogen, and R_{V} is 2,6-dimethoxyphenyl is treated with a compound of formula (V) in which R is hydrogen, Z is hydroxyle, and X, X' and the carbon atom to which they are bonded form an adamantylidene group.

7. Process for the preparation of a pharmaceutical composition, characterized in that it consists in mixing, as active principle, a compound obtained by the process according to any one of claims 1 to 6 or one of its possible phamaceutically acceptable salts with at least one pharmaceutical excipient.

8. Process for the preparation of a pharmaceutical composition according to claim 7, characterized in that the composition is converted into a unit dosage form.

9. Process for the preparation of a pharmaceutical composition according to claim 8, characterized in that it consists in mixing 0.25 to 250 mg of active principle with at least one pharmaceutical excipient.

10. Process for the preparation of N,N-diethyl-2-{[1-phenyl-5-(2-naphthyl)-3-pyrazol]carbonylamino}-3-phenylpropanamide, characterized in that a functional derivative of a pyrazolecarboxylic acid of formula (II) : 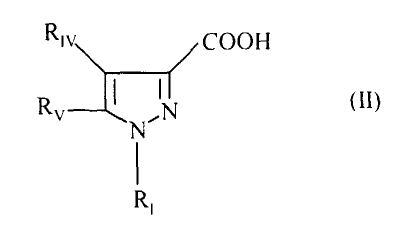 in which R_{I} is C₆H₅, R_{IV} is H and R_{V} is 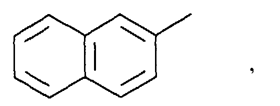 is treated with an amino acid, optionally protected by the protecting groups which are usual in peptide synthesis, of formula : 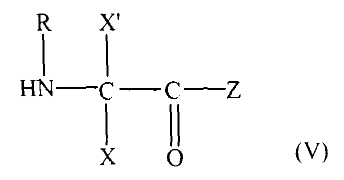 in which R is H, X' is H, X is -CH₂-C₆H₅ and Z is -N-(C₂H₅)₂.

11. Process for the preparation of 2-{[1-(1-naphthyl)-5-(2,6-dimethoxyphenyl)-3-pyrazolyl]carbonylamino}-bicyclo[2.2.1]-2-heptanecarboxylic acid, characterized in that a functional derivative of a pyrazolecarboxylic acid of formula (II) : 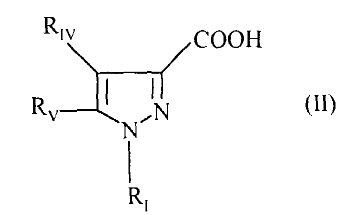 in which R_{I} is 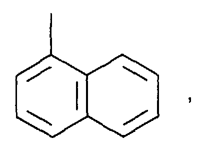 R_{IV} is H and R_{V} is 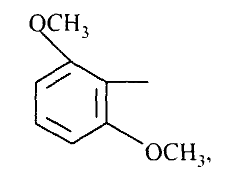 is treated with an amino acid, optionally protected by the protecting groups which are usual in peptide synthesis, of formula : 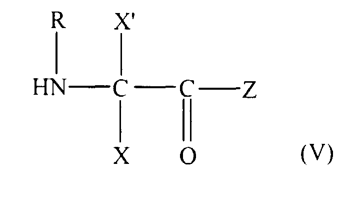 in which R is H, X, X' and the carbon atom to which they are bonded form the cycle 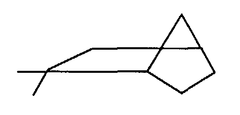 and Z is OH.

12. Process for the preparation of 9-{[1-(1-naphthyl)-5-(2,6-dimethoxyphenyl)-3-pyrazolyl]carbonylamino}-bicyclo[3.3.1]-9-nonanecarboxylic acid, characterized in that a functional derivative of a pyrazolecarboxylic acid of formula (II) : 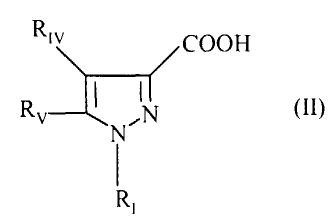 in which R_{I} is 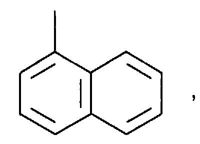 R_{IV} is H and R_{V} is 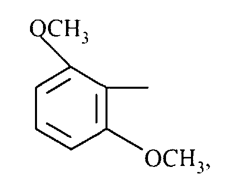 is treated with an amino acid, optionally protected by the protecting groups which are usual in peptide synthesis, of formula : 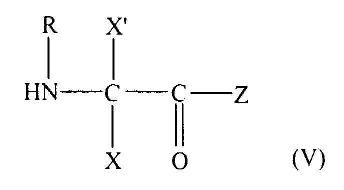 in which R is H, X, X' and the carbon atom to which they are bonded form the cycle 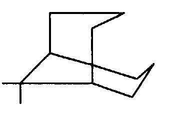 and Z is OH.

13. Process for the preparation of 2-{[1-(1-naphthyl)-5-(2,6-dimethoxyphenyl)-3-pyrazolyl]carbonylamino}-bicyclo[3.2.1]-2-octanecarboxylic acid, characterized in that a functional derivative of a pyrazolecarboxylic acid of formula (II) : 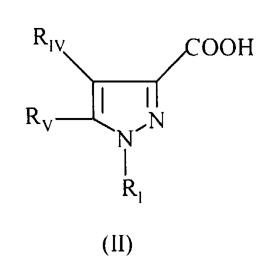 in which R_{I} is 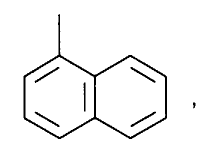 R_{IV} is H and R_{V} is 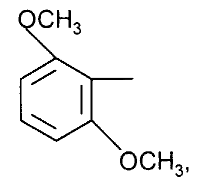 is treated with an amino acid, optionally protected by the protecting groups which are usual in peptide synthesis, of formula : 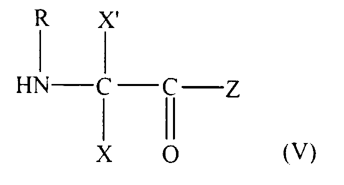 in which R is H, X, X' and the carbon atom to which they are bonded form the cycle 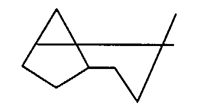 and Z is OH.

14. Process for the preparation of 3-{[5-(2-naphthyl)-1-phenyl-3-pyrazolyl]-carbonylamino}-2(S)-(acetamidomethylthio)propanecarboxylic acid sodium salt, characterized in that a functional derivative of a pyrazolecarboxylic acid of formula (II) : 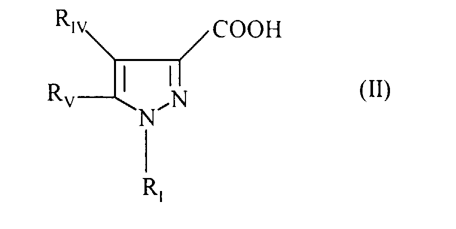 in which R_{I} is phenyl, R_{IV} is H and R_{V} is a naphthyl at position 2, is treated with an amino acid, optionally protected with the protecting groups which are usual in peptide synthesis, of formula : 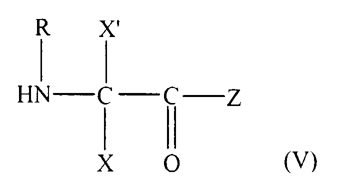 in which R is H, X is H, X' is an acetamidomethylthiomethyl and Z is OH, and the compound is converted into its sodium salt.

15. Process for the preparation of 2-{[1-(2,3-dihydro-8-phtalazinyl-1,4-dione)-5-(2,6-dimethoxyphenyl)-3-pyrazolyl]carbonylamino}-2-adamantanecarboxylic acid, characterized in that a functional derivative of a pyrazolecarboxylic acid of formula (II) : 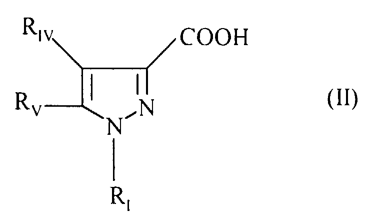 in which R_{I} is 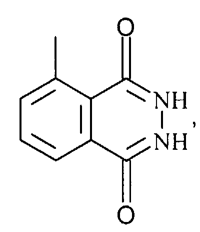 R_{IV} is H and R_{V} is 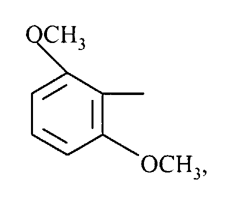 is treated with an amino acid, optionally protected with the protecting group which are usual in peptide synthesis, of formula : 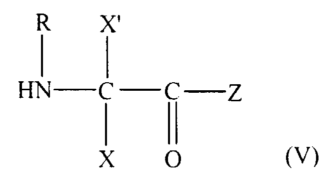 in which R is H, X, X' and the carbon atom to which they are bonded form the cycle 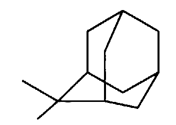 and Z is OH.

## Claims (Claims for the following Contracting State(s): GR)

1. 3-Amidopyrazole of formula (I) or (I'): 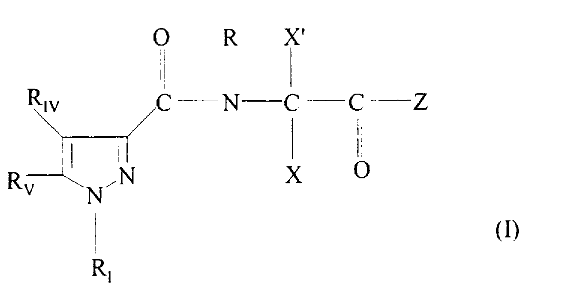 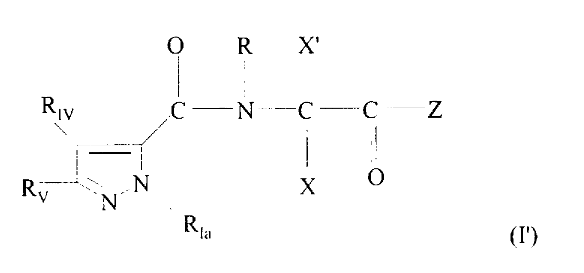 in which
- R_{I} represents
. a group 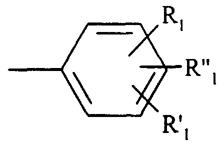 where R₁, R'₁ and R"₁ each independently represent a hydrogen atom, a halogen atom, a hydroxyl, a linear or branched C₁-C₄ alkyl group, a C₁-C₄ alkoxy group, a trifluoromethyl group, a trifluoromethoxy group, a nitro group, a carboxyl group, an amino group;
. a carboxyalkyl or alkoxycarbonylalkyl group in which the alkyls are C₁-C₄ groups;
. a cycloalkyl group in which the alkyl are C₃-C₆ groups;
. a tetrahydronaphthyl group;
. a pyridyl group;
. a naphthyl substituted with R₁, R'₁ and R"₁ as defined above;
. a benzyl group substituted with R₁, R'₁ and R"₁ as defined above;
. a cinnamyl group optionally substituted on the aromatic ring with a halogen, a hydroxyl or a C₁-C₄ alkoxy;
. a quinolyl or isoquinolyl group, optionally substituted with R₁, R'₁ and R"₁ as defined above;
. a 2-benzothiazolyl group;
. a quinoxalinyldione group;
. a 1-phthalazinyl group ;
. a benzothiadiazolyl group ;
. a methylene group substituted with a heterocyclic group selected from the pyridyl and thienyl groups ;
- R_{Ia} represents a benzyl group substituted with R₁, R'₁ and R"₁ as defined above ;
- R_{IV} represents a hydrogen atom, a halogen atom, a C₁-C₆ alkyl;
- R_{V} represents :
. a group 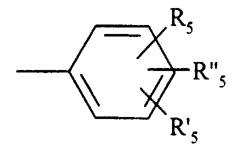
- where R₅, R'₅ and R"₅ each independently represent a hydrogen atom, a halogen atom, a linear or branched C₁-C₄ alkyl, a hydroxyl, a C₁-C₄ alkoxy, a nitro, a trifluoromethyl, a trifluoromethoxy, a cyano, an amino, a carboxyl, a C₁-C₄ carboxyalkyl, a phenyl;
. a naphthyl group unsubstituted or substituted with a C₁-C₄ alkyl;
. a pyridyl group;
- a styryl group unsubstituted or substituted with a C₁-C₄ alkyl;
- or alternatively R_{IV} and R_{V} considered together represent:
. a group 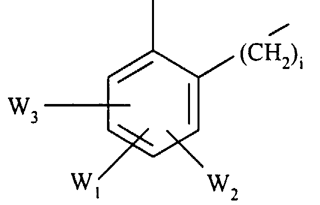 in which the phenyl group substitutes the pyrazole at position 5 and the group -(CH₂)ᵢ- in which i = 1 to 3 substitutes the pyrazole at position 4, W₁, W₂ and W₃ substitute the benzene cycle and independently represent hydrogen, a halogen or a hydroxyl group;
- R represents hydrogen, a linear or branched C₁-C₄ alkyl;
- Z represents a hydroxyl group, a C₁-C₆ alkoxy group ; an oxygen atom substituted with a group selected from the groups : tertio-butyl, benzyl, benzyl substituted with a halogen atom, C₁-C₆ alkyl, trifluoromethyl, trifluoromethoxy and carboxyl; an amino group ;
. either X represents hydrogen and X' represents hydrogen, a linear or branched C₁-C₆ alkyl; a phenyl ; a C₁-C₄ aminoalkyl; a C₁-C₄ hydroxyalkyl; a carboxyalkyl in which the alkyl group is a C₁-C₄ group; a guanidinoalkyl in which the alkyl group is a C₁-C₄ group; a nitroguanidinoalkyl in which the alkyl group is a C₁-C₄ group; a C₃-C₇ cycloalkyl; a phenylalkyl in which the alkyl is a C₁-C₄ group and in which the phenyl is optionally substituted with a halogen, a hydroxyl or with a C₁-C₃ alkyl; a heteroarylalkyl in which the heteroaryl represents an imidazolyl, an indolyl unsubstituted or substituted with a C₁-C₄ alkyl; with a hydroxyl or with a C₁-C₄ alkoxy and in which the alkyl is a C₁-C₄ alkyl; an adamantyl group; an adamantyl group substituted with one or two methyls, with a hydroxyl, a C₁-C₃ alkoxy; a halogen atom ; a 1-azaadamantyl group ;
- or X represents hydrogen, X' and R considered together form with the nitrogen atom to which R is bonded and with the carbon atom to which X is bonded a cycle, unsubstituted or substituted with a hydroxyl, of formula 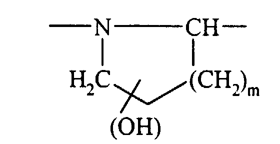 with m = 0, 1 or 2
or a cycle of formula : 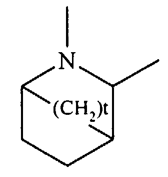 with t = 1 or 2
or a cycle of formula : 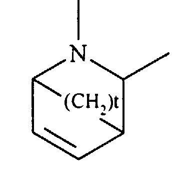 with t = 1 or 2
or an indolinyl ; perhydroindole, 4,5,6,7-tetrahydrothieno[2,3-c]pyrid-6-yl cycle;
- either X and X' each represent independently a C₁-C₄ alkyl; a C₃-C₆ cycloalkyl; a phenyl;
- or X, X' and the carbon atom to which they are bonded form a cycloalkylidene group of at most 12 carbon atoms, optionally substituted with a C₁-C₃ alkyl; an adamantylidene group; an adamantylidene group substituted with one or two methyl groups or with a hydroxyl, a C₁-C₃ alkoxy, a halogen atom ; a 1-azaadamantylidene group; a quinuclidinylidene group ; a 4-piperidinylidene group, optionally N-substituted with a benzyl group; a 2,2,6,6-tetramethylpiperidinylidene group; a tetrahydronaphthylidene group; a tetrahydropyran-4-ylidene or tetrahydrothiopyran-4-ylidene group; a 2,3-dihydro-(4H)-benzopyran-4-ylidene group; a 2,3-dihydro-(4H)-benzothiopyran-4-ylidene group ;
a bicyclo[2,2,1]hept-5-en-2-ylidene group; an 8-oxabicyclo[3,2,1]oct-6-en-3-ylidene; a 8-thiabicyclo[3,2,1]oct-3-anylidene; or one of its possible salts with organic or inorganic acids or with inorganic or organic bases.

2. 3-Amidopyrazole according to claim 1, of formula (I) in which R, X, X', Z, R_{IV} and R_{V} are as defined in claim 1, characterized in that R_{I} represents a phenyl or naphthyl group substituted with R₁, R'₁ and R"₁ as defined in claim 1, or one of its possible salts with organic or inorganic acids or with organic or inorganic bases.

3. 3-Amidopyrazole according to claim 1, of formula (I) or (I') in which R, X, X', Z, R_{I}, or R_{Ia} and R_{IV} are as defined in claim 1, characterized in that R_{V} represents a phenyl or a naphthyl substituted with R₅, R'₅ and R"₅ as defined in claim 1, R₅, R'₅ and R"₅ being preferably hydrogen or a C₁-C₄ alkoxy; or one of its possible salts with inorganic or organic acids or with organic or inorganic bases.

4. 3-Amidopyrazole according to claim 1, of formula I or I', in which X, X' and the carbon atom to which they are bonded form an adamantylidene group.

5. 3-Amidopyrazole according to claim 4 of formula : 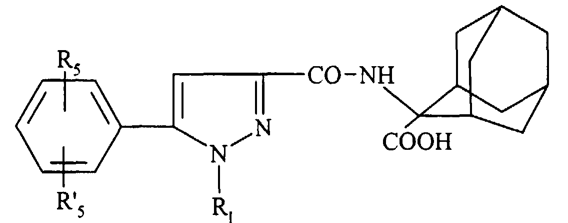 in which R_{I} is 1-naphthyl, 4-chloro-1-naphthyl, 5,6,7,8-tetrahydro-1-naphthyl, 5-isoquinolyl, 8-quinolyl, 7-chloro-4,2,1,3-quinolyl-4-benzothiadiazolyl, R₅ and R'₅ representing respectively 2- and 6-methoxy.

6. 3-Amidopyrazole according to claim 1, of formula I in which R_{I} is 7-chloro-4-quinolyl, R_{IV} is hydrogen, R_{V} is 2,6-dimethoxyphenyl, R is hydrogen, Z is hydroxyl and X, X' and the carbon atom to which they are bonded form an adamantylidene group.

7. Process for the preparation of compounds of formula (I) and (I') according to claim 1, characterized in that a functional derivative of a pyrazolecarboxylic acid of formula (II) or (II'): 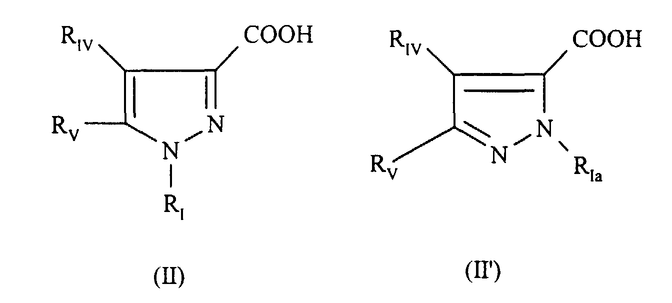 in which R_{I}, R_{IV}, R_{V} and R_{Ia} are as defined above, is treated with an aminoacid, optionally protected by the protecting groups which are usual in peptide synthesis, of formula: 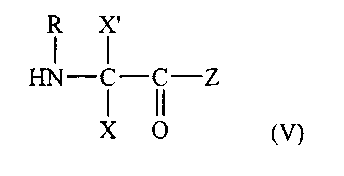 in which R, X, X' and Z are as defined above and the compound thus obtained is optionally converted into one of its salts.

8. Process for the preparation of a pharmaceutical composition, characterized in that it consists in mixing as the active principle, a compound obtained according to any one of claims 1 to 6 or one of its possible pharmaceutically acceptable salts which at least pharmaceutical excipient.

9. Process for the preparation of a pharmaceutical composition according to claim 8, characterized in that the composition is converted into a unit dosage form.

10. Process for the preparation of a pharmaceutical composition according to claim 9, characterized in that it consists in mixing 0.25 to 250 mg of active principle with at least one pharmaceutical excipient.

11. N,N-diethyl-3-{[1-phenyl-5-(2-naphthyl)-3-pyrazol]carbonylamino}-3-phenylpropanamide.

12. - 2-{[1-(1-naphthyl)-5-(2,6-dimethoxyphenyl)-3-pyrazolyl]carbonylamino}-bicyclo[2.2.1]-2-heptanecarboxylic acid ;
- 9-{[1-(1-naphthyl )-5-(2,6-dimethoxyphenyl )-3-pyrazolyl]carbonylamino}bicyclo[3.3.1]-9-nonanecarboxylic acid ;
- 2-{[1-(1-naphtyl)-5-(2,6-dimethoxyphenyl)-3-pyrazolyl]carbonylamino}bicyclo[3.2.1]-2-octanecarboxylic acid ;
- 3-{[5-(2-naphthyl)-1-phenyl-3-pyrazolyl]carbonylamino}-2(S)-(acetamidomethylthio)propanecarboxylic acid sodium salt ;
- 2-{[1-(2,3-dihydro-8-phtalazinyl-1,4-dione)-5-(2,6-dimethoxyphenyl)-3-pyrazolyl]carbonylamino}-2-adamantanecarboxylic acid.
